# EUROPEAN PATENT APPLICATION

(11) **EP 2 298 774 A1**
(43) Date of publication of application: **23.03.2011**
(21) Application number: 09758283.7
(22) Date of filing: 01.06.2009
(51) Int. Cl.: C07D 487/04, C07D 491/048, C07D 493/04, C09K 11/06, H01L 51/50, C07F 15/00

(54) **HALOGEN COMPOUND, POLYCYCLIC COMPOUND AND ORGANIC ELECTROLUMINESCENT ELEMENT USING THE POLYCYCLIC COMPOUND**

(30) Priority: 05.06.2008 JP 2008148515; 17.10.2008 US 253627; 16.04.2009 JP 2009100320
(71) Applicant: Idemitsu Kosan Co., Ltd., Chiyoda-ku Tokyo 100-8321 (JP)
(72) Inventor: KATO, Tomoki, Sodegaura-shi Chiba 299-0293 (JP); NUMATA, Masaki, Sodegaura-shi Chiba 299-0293 (JP); YOSHIDA, Kei, Sodegaura-shi Chiba 299-0293 (JP); NISHIMURA, Kazuki, Sodegaura-shi Chiba 299-0293 (JP); IWAKUMA, Toshihiro, Sodegaura-shi Chiba 299-0293 (JP); HOSOKAWA, Chishio, Sodegaura-shi Chiba 299-0293 (JP)
(74) Representative: Vossius & Partner
(86) International application number: PCT/JP2009/059981
(87) International publication number: WO 2009/148016

(57) **Abstract**

Provided are a polycyclic compound of a specific structure having a π-conjugated heteroacene skeleton crosslinked with a carbon atoms, a nitrogen atom, or an oxygen atom, and a halogen compound which can be used for the synthesis of the polycyclic compound. Also provided is an organic electroluminescence device having one or more organic thin film layers including a light emitting layer between a cathode and an anode in which at least one layer of the organic thin film layers contains the polycyclic compound. The organic electroluminescence device shows high luminous efficiency and has a long lifetime.

## Description

### Technical Field

The present invention relates to a halogen compound, a polycyclic compound, and an organic electroluminescence device using the same, in particular, to an organic electroluminescence device, which shows high luminous efficiency and has a long lifetime, and to a polycyclic compound for realizing the same, and a halogen compound serving as an intermediate for the polycyclic compound.

### Background Art

An organic electroluminescence device (hereinafter, "electroluminescence" may be abbreviated as "EL") is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported, many studies have been conducted on organic EL devices using organic materials as the constituent materials. The devices of the laminate type use tris (8-quinolinolato) aluminum for a light emitting layer and a triphenyldiamine derivative for a hole transporting layer. Advantages of the laminate structure are that the efficiency of hole injection into the light emitting layer can be increased, that the efficiency of forming exciton which are formed by blocking and recombining electrons injected from the cathode can be increased, and that exciton formed within the light emitting layer canbe enclosed. As described above, for the structure of the organic EL device, a two-layered structure having a hole transporting (injecting) layer and an electron transporting light emitting layer and a three-layered structure having a hole transporting (injecting) layer, a light emitting layer, and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris (8-quinolinolato) aluminum complexes, coumarine derivatives, tetraphenylbutadiene derivatives, distyrylarylene derivatives, and oxadiazole derivatives are known. It is reported that light in the visible region ranging from blue light to red light can be obtained by using these light emitting materials, and development of a device exhibiting color images is expected.
In addition, it has been recently proposed that a phosphorescent material as well as a fluorescent material be utilized in the light emitting layer of an organic EL device. High luminous efficiency is achieved by utilizing the singlet and triplet states of an excited state of an organic phosphorescent material in the light emitting layer of an organic EL devices. Upon recombination of an electron and a hole in an organic EL device, singlet excitons and triplet excitons may be produced at a ratio of 1:3 owing to a difference in spin multiplicity between the singlet and triplet excitons, so the use of a phosphorescent material may achieve luminous efficiency three to four times as high as that of a device using fluorescence alone.

Patent Literatures 1 to 7 are exemplary inventions each describing such materials for an organic EL device.
Patent Literature 1 describes a compound using a structure obtained by crosslinking a terphenylene skeleton with, for example, a carbon atom, nitrogen atom, or oxygen atom as a mother skeleton. The document, which mainly discloses data indicative of the potential of the compound to serve as a hole transporting material, describes that the compound is used as a host material for a phosphorescent material in a light emitting layer. However, the description is limited to a red phosphorescent device, and the luminous efficiency of the device is not high enough for the device to be put into practical use.
Patent Literature 2 describes an indolocarbazole compound having a substituent on a nitrogen atom or on an aromatic ring. The document recommends that the compound be used as a hole transporting material, and describes that a thermally and morphologically stable, thin hole transporting layer can be prepared from the compound. However, the document does not describe data indicative of the usefulness of the compound as a host material or electron transporting material to be used together with a phosphorescent material.
Patent Literature 3 describes indolocarbazole compounds each having a substituent on a nitrogen atom or on an aromatic ring. The document discloses data on a green light emitting device using any one of those compounds as a host material for a phosphorescent material in its light emitting layer. However, a high voltage must be applied to the device to drive the device, and the device shows low luminous efficiency, so the device cannot be sufficiently put into practical use.
Patent Literature 4 describes indolocarbazole compounds each having a substituent. The document describes that each of the compounds functions as a host material for a phosphorescent material in a light emitting layer. However, each of those compounds is characterized in that the compound has a dimer or trimer structure through a linking group, and each of the compounds tends to have a large molecular weight. The document discloses data on a green phosphorescent device using any one of those compounds, but all the compounds used each have a large molecular weight of 800 or more. The efficiency with which a material having a large molecular weight is deposited in a vacuum is poor, and the material may decompose owing to heating for a long time period, so the material may be insufficient in terms of practical use.
Patent Literatures 5 and 6 describe indenofluorene compounds each having a substituent on an aromatic ring, and describe that each of the compounds functions as a fluorescent material in a light emitting layer. However, none of the documents describes data indicative of the usefulness of each of the compounds as a host material or electron transporting material to be used together with a phosphorescent material.
Patent Literature 7 describes compounds each using a structure obtained by crosslinking a terphenylene skeleton with a sulfur atom, boron atom, or phosphorus atom as a mother skeleton. The document describes that each of those compounds has excellent oxidation resistance, and allows the formation of an organic semiconductor active layer by an application method. However, the document does not describe data indicative of the usefulness of eachof the compounds as a host material or electron transporting material to be used together with a fluorescent material or phosphorescent material.

### Citation List

### Patent Literature

[PTL 1] WO 2006/122630 A1
[PTL 2] EP 0908787 A
[PTL 3] WO 2007/063796 A1
[PTL 4] WO 2007/063754 A1
[PTL 5] US 2002/0132134 A1
[PTL 6] US 2003/0044646 A1
[PTL 7] JP 2008-81494 A

### Summary of Invention

### Technical Problem

The present invention has been made with a view to solving the above-mentioned problems, and an object of the present invention is to provide an organic EL device which shows high luminous efficiency and has a long lifetime, and a polycyclic compound for realizing the same, and a halogen compound serving as an intermediate for the polycyclic compound.

### Solution to Problem

The inventors of the present invention have made extensive studies to achieve the object. As a result, the inventors have found that the use of a polycyclic compound represented by the following formula (1) or (2) as a material for an organic EL device achieves the object. Thus, the inventors have completed the present invention.

That is, the present invention provides a polycyclic compound, which is represented by the following formula (1) or (2).

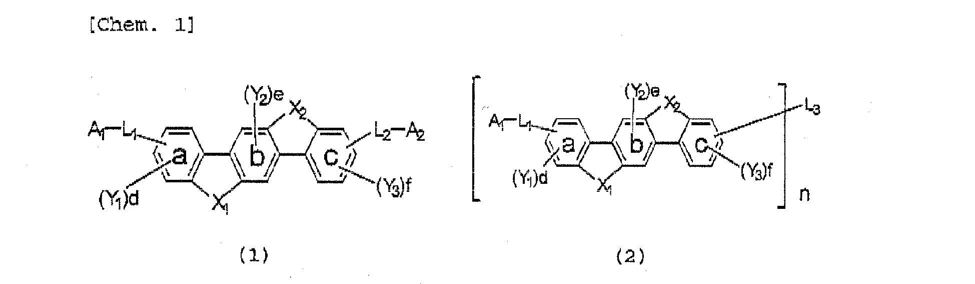

[In the formulae (1) and (2), X₁ and X₂ each independently represent oxygen (O), N-R₁, or CR₂R₃, provided that a case where both X₁ and X₂ represent CR₂R₃ is excluded, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms.
In the formula (2), n represents 2, 3, or 4, and the compound represented by the formula (2) includes a dimer using L₃ as a linking group for n=2, a trimer using L₃ as a linking group for n=3, or a tetramer using L₃ as a linking group for n=4.
In the formulae (1) and (2), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formula (1), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formula (2), when n represents 2, L₃ represents a single bond, an alkylene group having 1 to 20 carbon atoms, a substituted unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond.
In the formulae (1) and (2), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formula (1), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which, has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formulae (1) and (2), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2.
It should be noted that, when X₁ and X₂ each represent oxygen (O) or CR₂R₂, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.
In the formulae (1) and (2), A₁, A₂, L₁, L₂, and L₃ are each free of any carbonyl group.]

The present invention also provides a halogen compound, which is represented by the following formula (19) which can be used for the polycyclic compound.

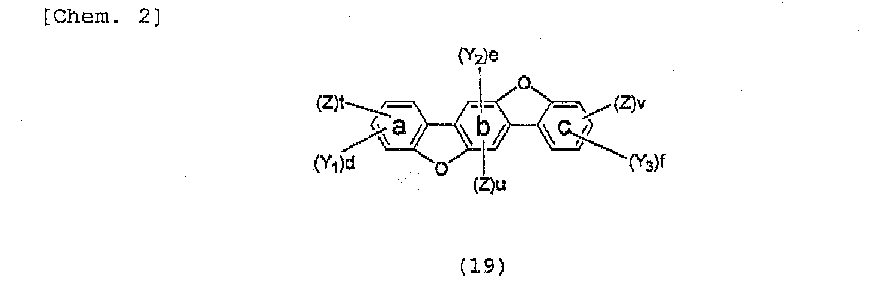

[In the formula (19), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent a number of 0, 1, 2, or 3, and e represents a number of 0, 1, or 2,
Z represents a halogen atom that is a fluorine atom, a chlorine atom, a bromine atom, or an iodine atom, and
t, u, and v each represent 0 or 1, provided that t+u+v≥1.]

The present invention also provides an organic EL device, including one or more organic thin film layers including a light emitting layer between a cathode and an anode, in which at least one layer of the organic thin film layers contains the polycyclic compound.
Further, the material for an organic EL device is effective also as a material for an organic electron device such as an organic solar cell, organic semiconductor laser, a sensor using organic matter, or an organic TFT.

### Advantageous Effects of Invention

According to the present invention, there can be provided an organic EL device which shows high luminous efficiency and has a long lifetime, and a polycyclic compound for realizing the same.

### Description of Embodiments

A material for an organic EL device of the present invention is represented by the following formula (1) or (2).

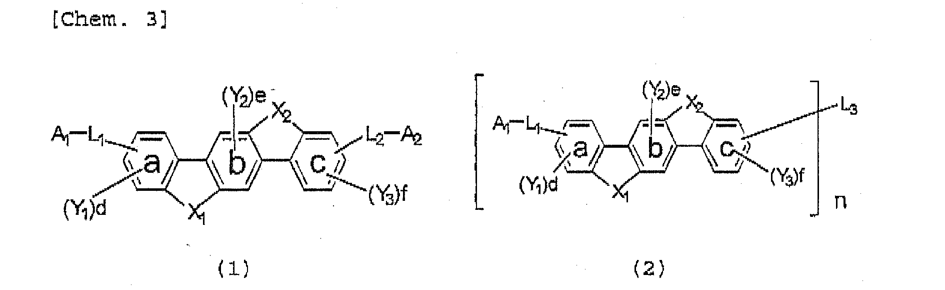

[In the formulae (1) and (2), X₁ and X₂ each independently represent oxygen (O), N-R₁, or CR₂R₃, provided that a case where both X₁ and X₂ represent CR₂R₃ is excluded, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms.
In the formula (2), n represents 2, 3, or 4, and the compound represented by the formula (2) includes a dimer using L₃ as a linking group for n=2, a trimer using L₃ as a linking group for n=3, or a tetramer using L₃ as a linking group for n=4.
In the formulae (1) and (2), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formula, (1), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formula (2), when n represents 2, L₃ represents a single bond, an alkylene group having 1 to 20 carbon atoms, a substituted unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond.
In the formulae (1) and (2), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formula (1), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formulae (1) and (2), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0, 1, or 2.
It should be noted that, when X₁ and X₂ each represent oxygen (O) or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.

In the case where A₁ and/or A₂ in the formulae (1) and (2) each represent/represents a pyrimidyl group or a dibenzofuranyl group, electron transporting property is improved, and a reduction in voltage at which a device using the polycyclic compound is driven can be expected.
In the case where A₁ and/or A₂ in the formulae (1) and (2) each represent/represents a carbazolyl group, hole transporting property is improved, and a reduction in voltage at which the device using the polycyclic compound is driven can be expected.
In the case where A₁ and/or A₂ in the formulae (1) and (2) each represent/represents pyrimidyl group, a dibenzofuranyl group, or a carbazolyl group, when any such substituent further has at least one aryl group (preferably a phenyl group, a biphenyl group, a terphenyl group, or a quaterphenyl group, and particularly preferably a phenyl group, a meta-biphenyl group, or a meta- terphenyl group) as a substituent, molecular stability is improved, and the lengthening of the lifetime of the device using the polycyclic compound can be expected. Although the pyrimidyl group, dibenzofuranyl group, or carbazolyl group is not particularly limited, specific examples of the groups include the following examples.

(Pyrimidyl group)

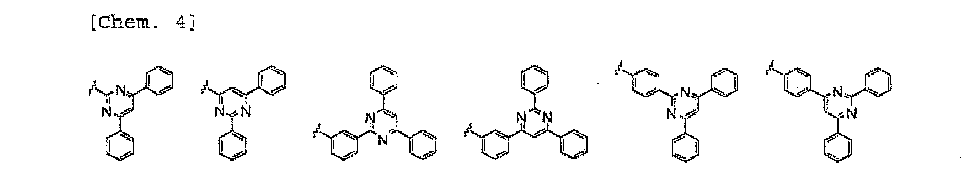

(Dibenzofuranyl group) 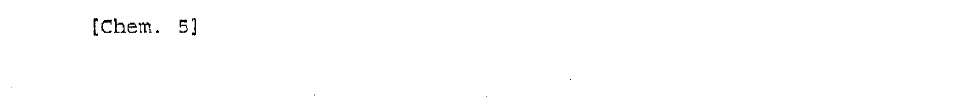 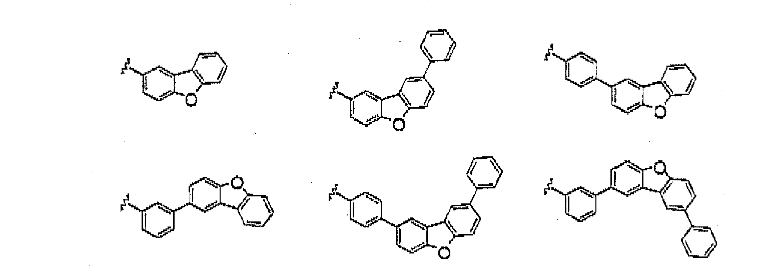 Of those, the following substituents are particularly preferred.

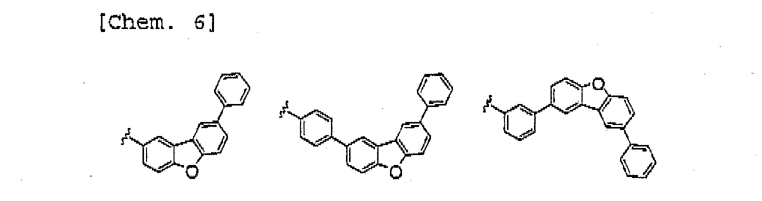

(Carbazolyl group) 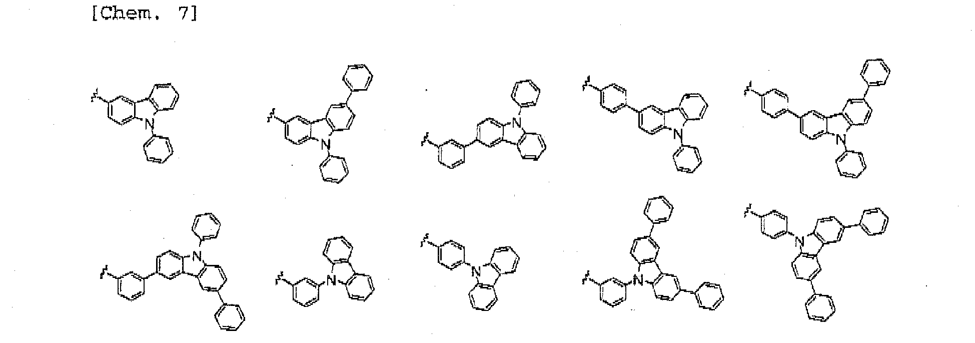 Of those, the following substituents are particularly preferred.

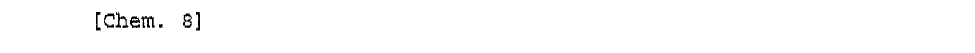

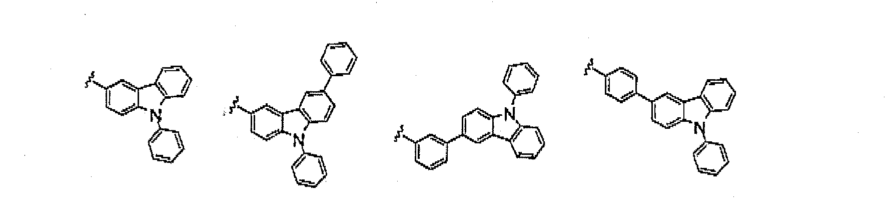

In the case where A₁ and/or A₂ in the formulae (1) and (2) each represent/represents a substituted or unsubstituted aromatic hydrocarbon group having a ring formed of 6 to 24 carbon atoms, a feature of the n-conjugated heteroacene skeleton as a mother skeleton, i.e., carrier transporting property is maintained, a Tg is increased, and a proper deposition characteristic is obtained. Further, in the case where A₁ and/or A₂ each represent/represents an aromatic hydrocarbon group having a structure in which two or more benzene rings are bonded to each other at meta positions, the expansion of the conjugated system can be prevented, and triplet energy can be widened. It should be noted that the term "triplet energy" as used in the present invention refers to a difference in energy between the lowest excited triplet state and the ground state.
Although the aromatic hydrocarbon group having a structure in which two or more benzene rings are bonded to each other at meta positions is not particularly limited, specific examples of the group include the following examples. 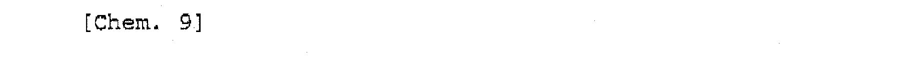 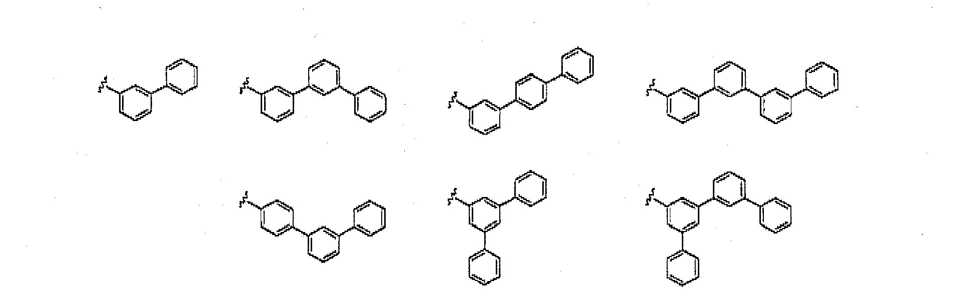 Of those, the following structures are particularly preferred.

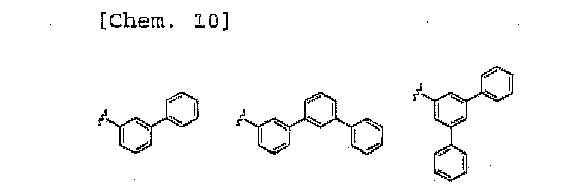

The compounds represented by the formulae (1) and (2) are each preferably a polycyclic compound represented by any one of the following formulae (1a), (1b), (2a), and (2b).
As described above, it is preferred that the bonding sites of substituents such as L₁-A₁- and L₂-A₂- be meta positions because the expansion of the conjugated system can be prevented, and triplet energy can be widened. 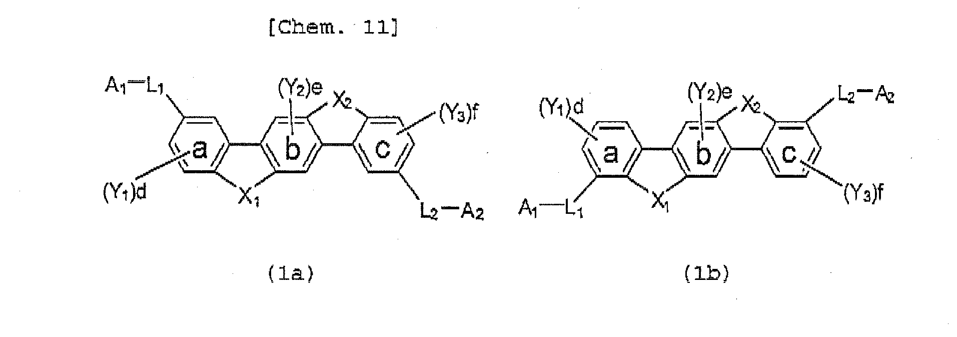 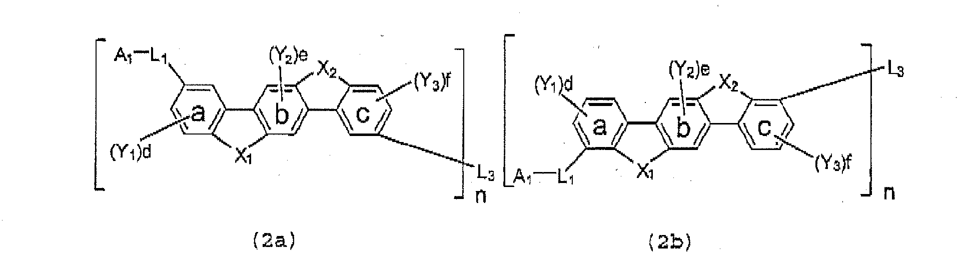 [In the formulae (1a), (1b), (2a), and (2b), X₁, X₂, L₁, L₂, L₃, A₁, A₂, Y₁, Y₂, Y₃, n, d, f, and e each have the same meaning as that described above.

In the formulae (1) and (2), when a plurality of Y₁'s, a plurality of Y₂'s, or a plurality of Y₃'s substitutes the benzene ring a, b, or c, respectively, the substituted benzene ring is represented as described below. 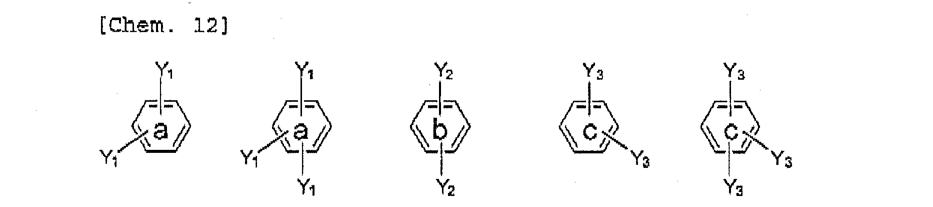 In the formulae (1) and (2), A₁, A₂, L₁, L₂, and L₃ are each free of any carbonyl group.]

The polycyclic compound represented by the formula (1) is preferably a polycyclic compound represented by any one of the following formulae (3) to (6), (11), and (13), and the polycyclic compound represented by the formula (2) is preferably a polycyclic compound represented by any one of the following formulae (7) to (10), (12), and (14).

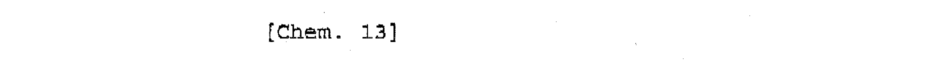

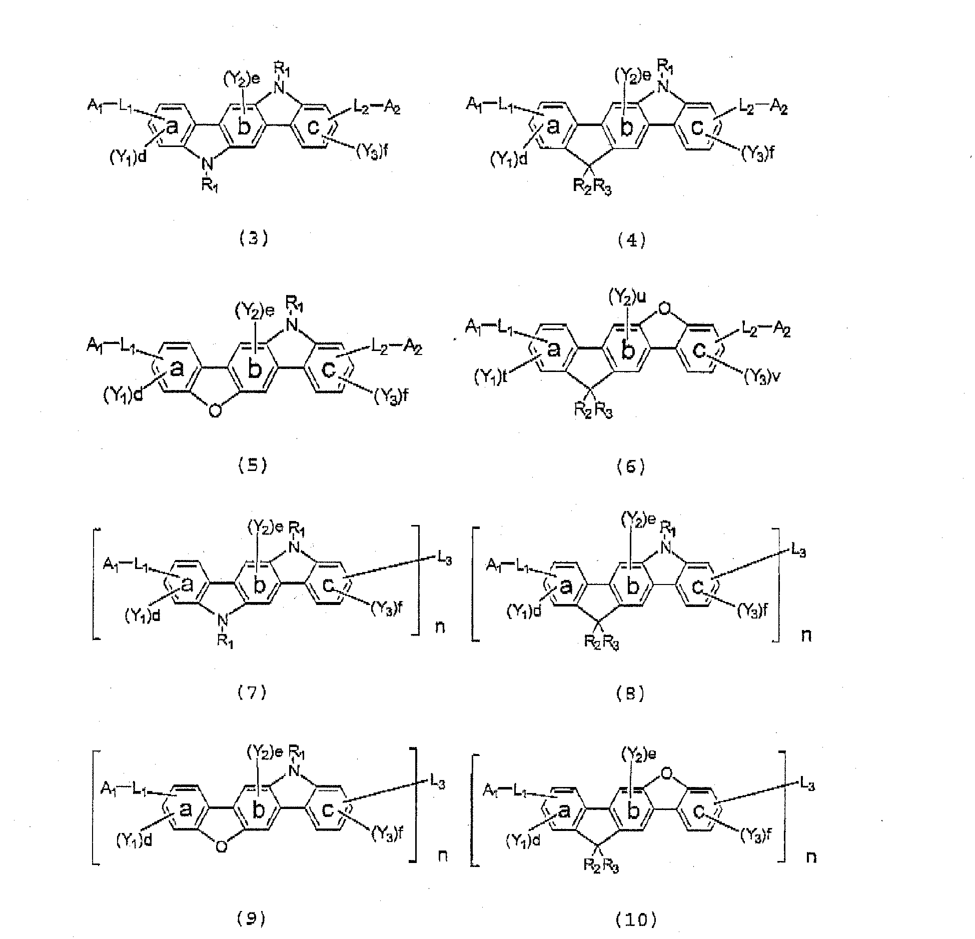

[In the formulae (3) to (10), R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, in the formula (3) and (7), at least one R₁ represents a substituted or unsubstituted, monovalent fused aromatic heterocyclic group having a ring formed of 8 to 24 atoms.
In the formulae (7) to (10), n represents 2, 3, or 4, and the compound represented by any one of the formulae (7) to (10) includes a dimer using L₃ as a linking group for n=2, a trimer using L₃ as a linking group for n=3, or a tetramer using L₃ as a linking group for n=4.
In the formulae (3) to (10), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formulae (3) to (6), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formulae (7) to (10), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond.
In the formulae (3) to (10), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formulae (3) to (6), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formulae (3) to (10), Y₂, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0, 1, or 2, provided that, when both L₁ and L₂ represent single bonds and both A₁ and A₂ represent hydrogen atoms, the benzene ring b has one or two Y₂'s and a case where Y₂ represents a methyl group or an unsubstituted phenyl group is excluded.
In the formulae (3) to (10), A₁, A₂, L₁, L₂, and L₃ are each free of any carbonyl group.] 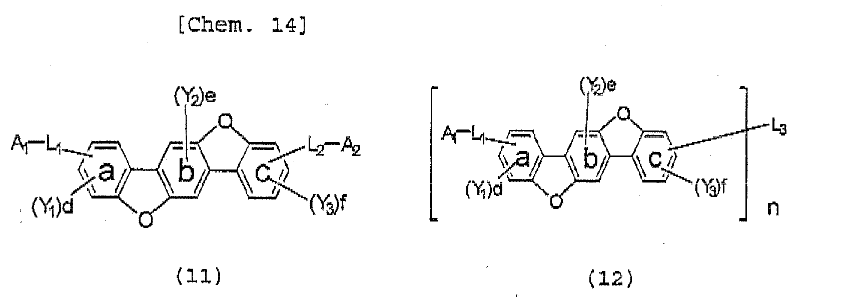 Further, the formulae (11) and (12) are preferably the following formulae (13) and (14).

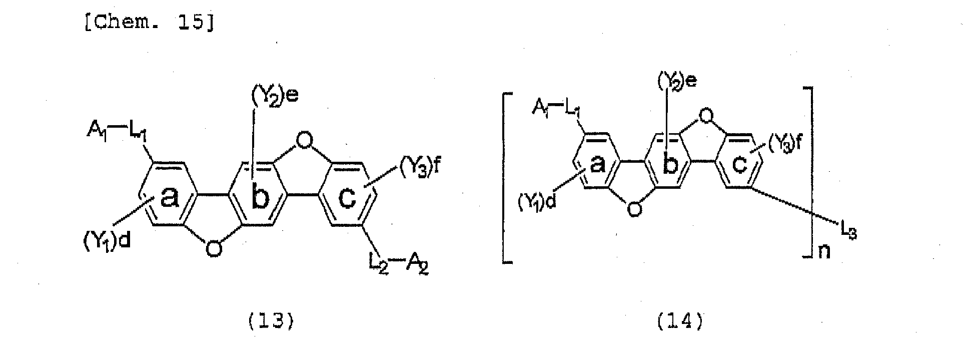

[In the formulae (12) and (14), n represents 2, 3, or 4, and the compound represented by one of the formulae (12) and (14) includes a dimer using L₃ as a linking group for n=2, a trimer using L₃ as a linking group for n=3, or a tetramer using L₃ as a linking group for n=4.
In the formulae (11) to (14), L₁ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formulae (11) and (13), L₂ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formulae (12) and (14), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond.
In the formulae (11) to (14), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formulae (11) and (13), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon, bond, provided that, when L₂ represents an alkylene group having 1 to 20 carbon atoms, a case where A₂ represents a hydrogen atom is excluded. In the formulae (11) to (14), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0, 1, or 2, provided that, when both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.
In the formulae (11) to (14), A₁, A₂, L₁, L₂, and L₃ are each free of any carbonyl group.]

In addition, the present invention also provides a polycyclic compound represented by the following formula (15).

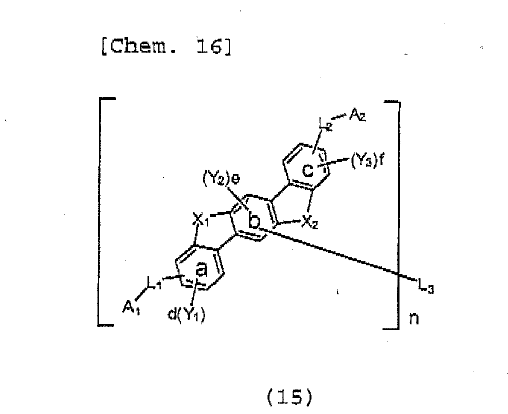

[In the formula (15), X₁ and X₂ each independently represent oxygen (O), N-R₁, or CR₂R₃, provided that a case where both X₁ and X₂ represent CR₂R₃ is excluded, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms.
In the formula (15), n represents 2, 3, or 4, and the compound represented by the formula (15) includes a dimer using L₃ as a linking group for n=2, a trimer using L₃ as a linking group for n=3, or a tetramer using L₃ as a linking group for n=4.
In the formula (15), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formula (15), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formula (15), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond.
In the formula (15), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded. In the formula (15), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded. In the formula (15), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0 or 1.
In the formula (15), A₁, A₂, L₁, L₂, and L₃ are each free of any carbonyl group.]

Further, the polycyclic compound represented by the formula (15) is preferably a polycyclic compound represented by the following formula (16).

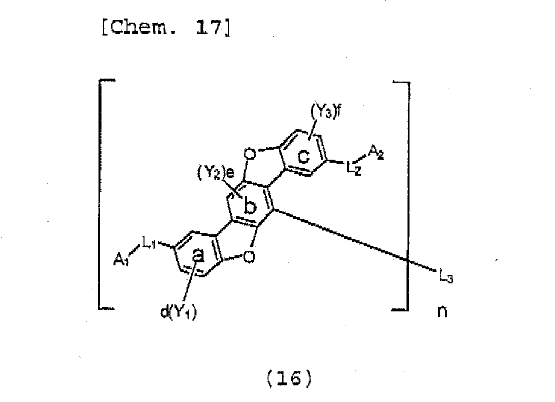

[In the formula (16) n represents 2, 3, or 4, and the compound represented by the formula (16) includes a dimer using L₃ as a linking group for n=2, a trimer using L₃ as a linking group for n=3, or a tetramer using L₃ as a linking group for n=4.
In the formula (16), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formula (16), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formula (16), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond.
In the formula (16), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formula (16), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formula (16), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0 or 1.
In the formula (16), A₁, A₂, L₁, L₂, and L₃ are each free of any carbonyl group.]

In addition, the present invention also provides a polycyclic compound represented by the following formula (17).

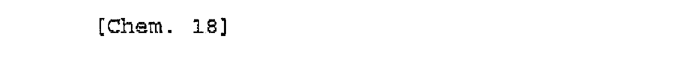

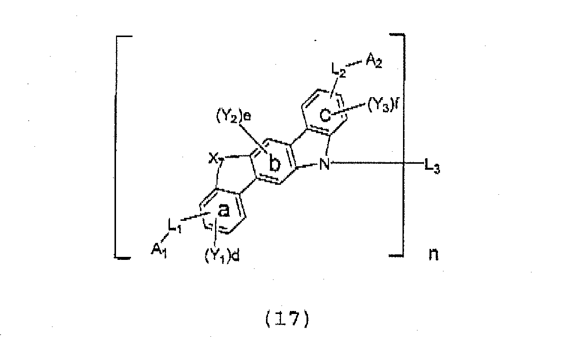

[In the formula (17), X₇ represents oxygen (O) or CR₂R₃.
R₂ and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms.
In the formula (17), n represents 2, 3, or 4, and the compound represented by the formula (17) includes a dimer using L₃ as a linking group for n=2, a trimer using L₃ as a linking group for n=3, or a tetramer using L₃ as a linking group for n=4.
In the formula (17), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formula (17), L₂ represents a single bond, as alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond.
In the formula (17), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbonatoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring b through a carbon-carbon bond, when n represents 3, L₃ represents a trivalent saturated hydrocarbon group having 1 to to carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond.
In the formula (17), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formula (17), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formula (17), Y₁, Y₂, and Y₃ each represent an alkyl group having 1 to 20 carbon atoms, a substituted or unsubstituted, cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0, 1, or 2.
In the formula (17), A₁, A₂, L₁, L₂, and L₃ are each free of any carbonyl group.]

In the formulae (11) to (17), A₁ preferably represents a silyl group or a substituted silyl group having 3 to 20 carbon atoms, or an aromatic heterocyclic group which has a ring formed of 3 to 24 atoms and which is linked with L₁ through a carbon-carbon bond.
Alternatively, in the formulae (11) to (17), A₁ preferably represents an aromatic heterocyclic group selected from pyridazine, pyrimidine, pyrazine, 1,3,5-triazine, carbazole, dibenzofuran, dibenzothiophene, phenoxazine, phenothiazine, and dihydroacridine, the aromatic heterocyclic group being linked with L₁ through a carbon-carbon bond.

In addition, the present invention also provides a halogen compound represented by the following formula (18) as an intermediate suitable for use in the synthesis of the polycyclic compound of the present invention.

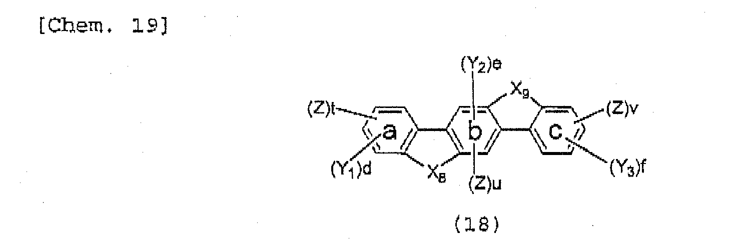

[In the formula (18), X₈ and X₉ each independently represent oxygen (0) or N-R₁.
R₁ represents an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms.
Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.
Z represents a halogen atom that is a fluorine atom, a chlorine atom, a bromine atom, or an iodine atom.
t, u, and v each represent 0 or 1, provided that t+u+v≥1.]

The halogen compound represented by the formula (18) is preferably represented by the following formula (19).

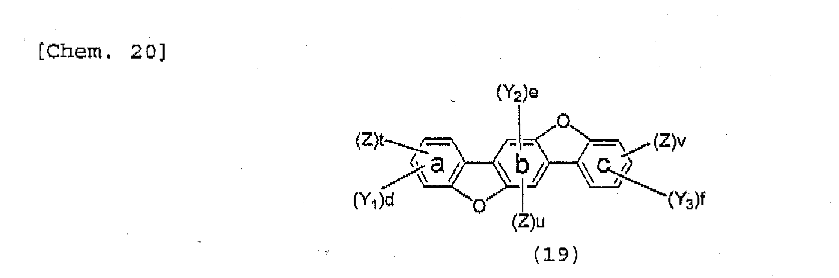

[In the formula (19), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted, aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.
Z represents a halogen atom that is a fluorine atom, a chlorine atom, a bromine atom, or an iodine atom.
t, u, and v each represent 0 or 1, provided that t+u+v≥1.]

The halogen compound represented by the formula (19) can be obtained by subjecting an aromatic halogen compound represented by the following formula (28) to an intramolecular cyclization reaction.

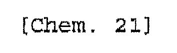

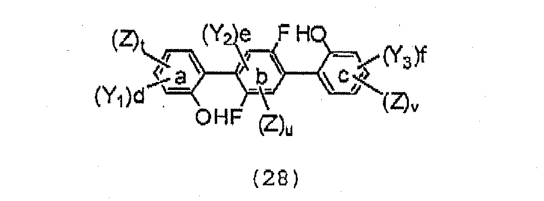

[In the formula (28), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, d and f each represent the number of 0, 1, 2, or 3, and e represents the number of 0, 1, or 2.
Z represents a halogen atom that is a fluorine atom, a chlorine atom, a bromine atom, or an iodine atom.
t, u, and v each represent 0 or 1, provided that t+u+v≥1.]

The reaction of the aromatic halogen compound represented by the above-mentioned formula (28) is typically performed in the presence of a basic catalyst. Examples of the basic catalyst include sodium amide, triethylamine, tributylamine, trioctylamine, pyridine, N,N-dimethylaniline, 1, 5-diazabicyclo [4,3,0] nonene-5 (DEN), 1,8-diazabicyclo [5,4,0] undecene-7 (DBU), sodium hydroxide, potassium hydroxide, sodium hydride, sodium phosphate, potassium phosphate, sodium carbonate, potassium carbonate, sodium methoxide, and potassium t-butoxide.
The basic catalyst is used for the aromatic halogen compound represented by the formula (28) as a reaction raw material in such an amount that a molar ratio of the basic catalyst to the aromatic halogen compound is about 2 to 10 and preferably 2 to 5.
A solvent can be used as required at the time of the reaction. Specific examples of the solvent include toluene, dimethylformamide (DMF), N,N-dimethylacetamide (DMAc), dimethyl sulfoxide (DMSO), N-methylpyrrolidone (NMP), tetrahydrofuran, and dimethoxyethane. One kind of them may be used alone, or two or more kinds of them may be used in combination.
The reaction of the aromatic halogen compound represented by the above-mentioned formula (28) is performed at a temperature of about 0 to 250°C and preferably 150 to 200°C in ordinary cases. When the reaction temperature is 150°C or more, a reaction rate does not reduce but becomes moderate, and hence a reaction time is shortened. In addition, when the reaction temperature is 200°C or less, the coloring of a product is suppressed. A reaction time is typically about 1 minute to 24 hours, and is desirably 1 to 10 hours.
In addition, such a halogen compound that both X₈ and X₉ in the above-mentioned formula (18) represent N-R₁ can be obtained by subjecting an aromatic halogen compound represented by the following formula (29) to an intramolecular cyclization reaction in the same manner as in the foregoing. 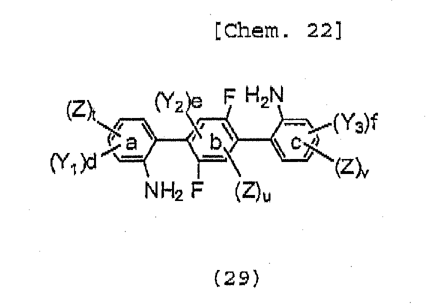 It should be noted that Y₁, Y₂, Y₃, Z, d, e, f, t, u, and v in the formula (29) are identical to Y₁, Y₂, Y₃, Z, d, e, f, t, u, and in the formula (28), respectively.

In the formulae (1) to (19) and (28) and (29), specific examples of each group are described below.
Examples of the substituted or unsubstituted aromatic hydrocarbon group having a ring formed of 6 to 24 carbon atoms represented by each of Y₁ to Y₃, R₁ to R₃, L₁ to L₃, and A₁ and A₂ include residues having corresponding valencies of substituted or unsubstituted benzene, naphthalene, biphenyl, terphenyl, fluorene, phenanthrene, triphenylene, perylene, chrysene, fluoranthene, benzofluorene, bnezotriphenylene, benzochrysene, and anthracene. Preferred are benzene, naphthalene, biphenyl, terphenyl, fluorine, and phenanthrene.
Examples of the substituted or unsubstituted aromatic heterocyclic group having a ring formed of 3 to 24 atoms represented by each of Y₁ to Y₃, R₁ to R₃, L₁ to L₃, and A₁ and A₂ include residues having corresponding valencies of pyridine, pyridazine, pyrimidine, pyrazine, 1,3,5-triazine, carbazole, dibenzofuran, dibenzothiophene, phenoxazine, phenothiazine, and dihydroacridine. Preferred are pyridine, pyridazine, pyrimidine, pyrazine, carbazole, dibenzofuran, dibenzothiophene, phenoxazine, and dihydroacridine. In addition, examples of at least one substituted or unsubstituted, monovalent fused aromatic heterocyclic group having a ring formed of 8 to 24 atoms represented by R₁ include aromatic heterocyclic groups each having a fused structure in examples of the aromatic heterocyclic groups.
Examples of the alkyl group, alkylene group, and trivalent or tetravalent saturated hydrocarbon group, each of which has 1 to 20 carbon atoms represented by each of Y, Y₁ to Y₃, L₁ to L₃, and R₁ to R₃ include a methyl group, an ethyl group, a propyl group, an isopropyl group, an n-butyl group, an s-butyl group, a t-butyl group, an isobutyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a neopentyl group, a 1-methylpentyl group, a 2-methylpentyl group, a 1-pentylhexyl group, a 1-butylpentyl group, a 1-heptyloctyl group, and a 3-methylpentyl group, and groups obtained by allowing those groups to have two to four valencies. Preferred are a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a neopentyl group, a 1-methylpentyl group, a 1-pentylhexyl group, a 1-butylpentyl group, and a 1-heptyloctyl group.
Examples of the substituted or unsubstituted cycloalkyl group, cycloalkylene group, and trivalent or tetravalent cyclic saturated hydrocarbon group, each of which has a ring formed of 3 to 20 carbon atoms, represented by each of Y₁ to Y₃, L₁ to L₃, R₁ two R₃, and A, and A₂ include a cyclopropyl group, a cyclobutyl group, a cyclopentyl group, and a cyclohexyl group, and groups obtained by allowing those group to have two to four valencies. Preferred are a cyclobutyl group, a cyclopentyl group, and a cyclohexyl group.
Examples of the alkoxy group having 1 to 20 carbon atoms and represented by each of Y₁ to Y₃ include a methoxy group, an ethoxy group, a methoxy group, an i-propoxy group, an n-propoxy group, ann-butoxygroup, an s-butaxy group, and at-butoxygroup. Preferred are a methoxy group, an ethoxy group, a methoxy group, an i-propoxy group, and an n-propoxy group.
Examples of the substituted silyl group having 1 to 20 carbon atoms represented by each of Y₁ to Y₃, L₁ to L₃, R₁ to R₃, and A₁ and A₂ include a trimethylsilyl group, a triethylsilyl group, a tributylsilyl group, a trioctylsilyl group, a triisobutylsilyl group, a dimethylethylsilyl group, a dimethylisopropylsilyl group, a dimethylpropylsilyl group, a dimethylbutylsilyl group, a dimethyl tertiary butylsilyl group, a diethylisopropylsilyl group, a phenyldimethylsilyl group, a diphenylmethylsilyl group, a diphenyl tertiary butyl group, and a triphenylsilyl group, and groups obtained by allowing those groups to have two or three valencies. Preferred are a trimethylsilyl group, a triethylsilyl group, and a tributylsilyl group.
Examples of the aralkyl group having 7 to 24 carbon atoms represented by each of Y₁ to Y₃, and R₁ to R₃ include a benzyl group, a phenethyl group, and a phenylpropyl group.

Examples of the substituent that may substitute each of the group in the formulae (1) to (19) and (28) and (29) include alkyl groups each having 1 to 10 carbon atoms (such as a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, a iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, and a 1,2,3-trinitropropyl group), cycloalkyl groups each having a ring formed of 3 to 40 carbon atoms (such as a cyclopropyl group, a cyclobutyl group, a cyclopentyl group, acyclohexylgroup, a 4-methylcyclohexyl group, a 1-adamantyl group, a 2-adamantyl group, a 1-norbornyl group, and a 2-norbornyl groups), alkoxy groups each having 1 to 6 carbon atoms (such as an ethoxy group, a methoxy group, an i-propoxy group, an n-propoxy group, an s-butoxy group, a t-butoxy group, a pentoxy group, and a hexyloxy group), cycloalkoxy groups each having a ring formed of 3 to 10 carbon atoms (such as a cyclopentoxy group and a cyclohexyloxy group), aromatic hydrocarbon groups each having a ring formed of 6 to 40 carbon atoms, aromatic heterocyclic groups each having a ring formed of 3 to 40 atoms, amino groups each substituted with an aromatic hydrocarbon group having a ring formed of 6 to 40 carbon atoms, ester groups each having an aromatic hydrocarbon group having a ring formed of 6 to 40 carbon atoms, an ester group, cyano group, and nitro group, each of which has an alkyl group having 1 to 6 carbon atoms, and halogen atom.
Of those, an alkyl group having 1 to 6 carbon atoms, a phenyl group, a pyridyl group, a carbazolyl group, and a dibenzofuranyl group are preferred and the number of substituents is preferably 1 or 2.

In the polycyclic compound represented by the formula (2), (7) to (10), (12), (14), or (15) to (17), n preferably represents 2.
In the formula (1), (3) to (6), (11), or (13), the total number of the substituents represented by Y₁, Y₂, and Y₂ is preferably 3 or less, and the total number of the substituents represented by Y₁, Y₂, and Y₃ in the structure of []ₙ in the formula (2), (7) to (10), (12), (14), or (15) to (17) is preferably 3 or less.

It is preferred that, in the formula (1), (2), or (15), X₁ and X₂ each represent N-R₁, and N-R₁ represented by X₁ and N-R₁ represented by X₂ be different from each other. As described above, in the case where any one of the formulae (1), (2), and (15) is of an symmetric structure, crystallization is suppressed, the stability of a thin film is improved, and the lifetime of the device is lengthened as compared with the case where the formula is of a symmetric structure.
The compounds represented by the formula (1) and (2), and further the formula (15) are each preferably such that both X₁ and X₂ represent N-R₁ like the formula (3) or (7). Crosslinking with N improves the hole transporting property of the compound, and hence can promote a reduction, in voltage at which the device is driven. Those compounds can each be particularly suitably used as a host material or a hole transporting material.
In addition, it is preferred that, in the formula (3) or (7), at least one of R₁'s represent a dibenzofuran residue or a carbazole residue, and it is more preferred that all of R₁'s each represent a dibenzofuran residue or a carbazole residue. Similarly, it is preferred that, in the formula (4), (5), (8), (9), or (15), R₁ represents a dibenzofuran residue or a carbazole residue.
Bonding an electron transportable, fused aromatic heterocyclic ring such as dibenzofuran or carbazole improves stability against a hole (oxidation resistance) as compared with a nitrogen-containing heterocyclic ring that is not a fused ring such as pyrimidine, thereby leading to the lengthening of the lifetime. In addition, bonding an electron transportable, fused aromatic heterocyclic ring having a large energy gap such as dibenzofuran or carbazole to N prevents a reduction in efficiency when the device is used as a phosphorescent device, and improves the stability against a hole (oxidation resistance).

In addition, when the benzene rings a and c are bonded at para positions with respect to the benzene ring b like any one of the formulae (1) to (17), the electron transporting property can be improved, and a reduction in voltage at which the device is driven can be achieved.
It is preferred that, in the formula (1) or (2), at least one of X₁ and X₂ preferably represents an oxygen atom like any one of the formulae (5), (6), (9), and (10), and both the substituents more preferably represent oxygen atoms like any one of the formulae (11) to (14) and (16). An oxygen atom has a high electronegativity, and can improve the electron transporting property. As a result, a reduction in voltage at which the device is driven can be achieved. Accordingly, any such compound can be particularly suitably used as a host material or an electron transporting material. In addition, whenboth the substituents represent oxygen atoms, the triplet energy can be widened, and hence the luminous efficiency can be improved.
In addition, when A₁, A₂, L₁, L₂, and L₃ in any one of the formulae (1) to (17) are each free of any carbonyl group, that is, a terminal structure is free of any carbonyl group, the shortening of the life time is suppressed.

Specific examples of the material for an organic EL device represented by the formulae (1) to (17) of the present invention are shown below. However, the present invention is not limited to these exemplary compounds.

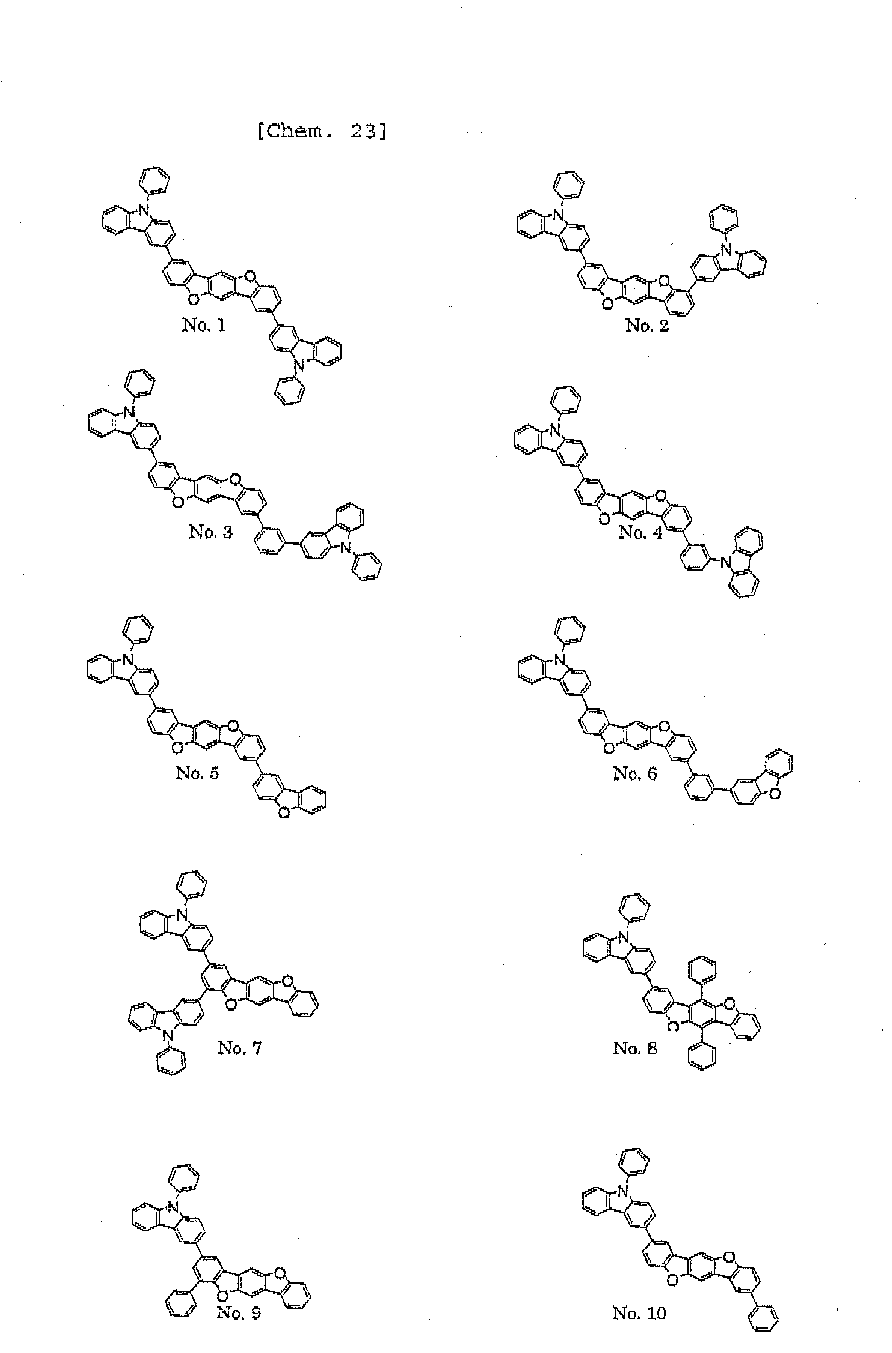

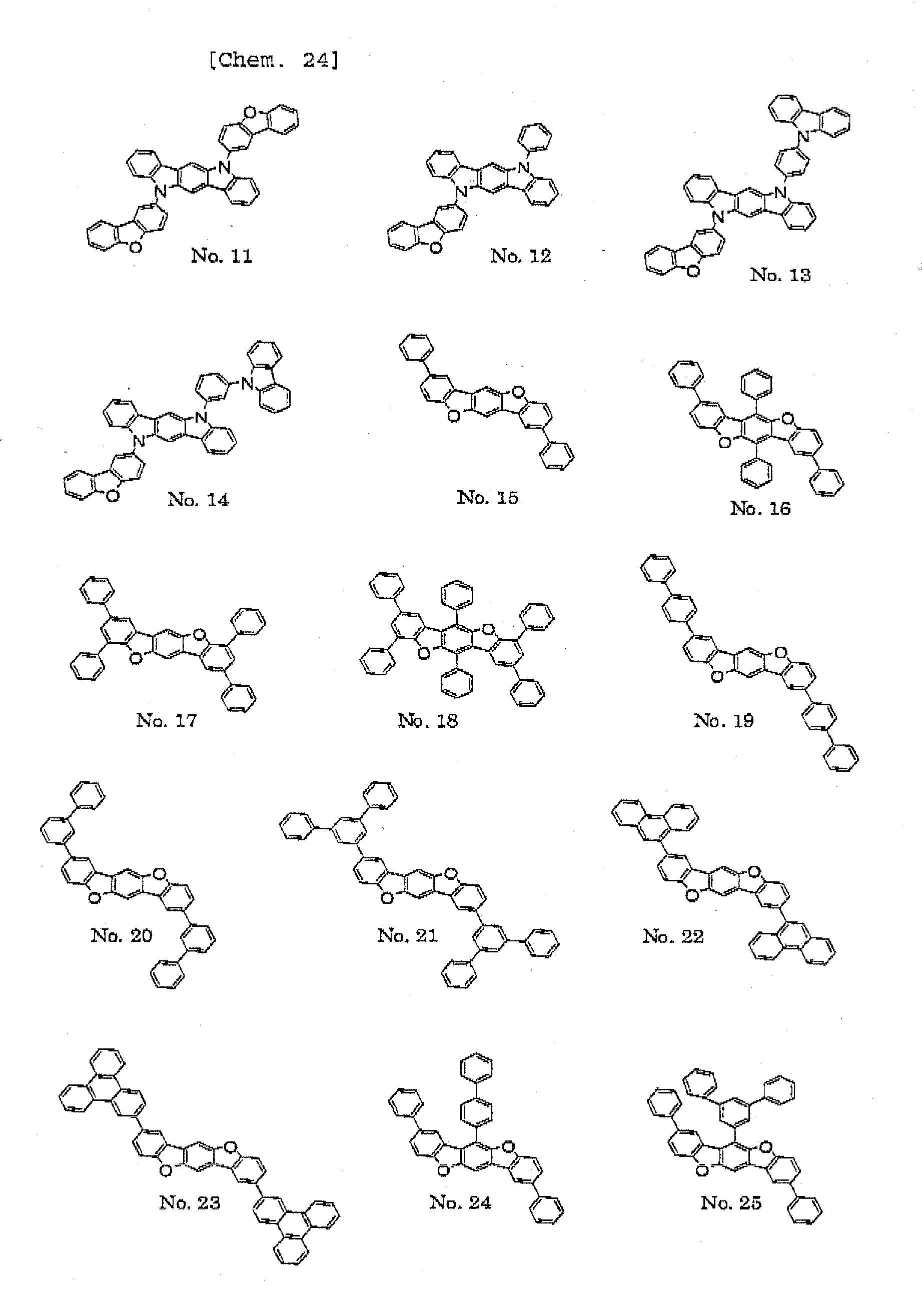

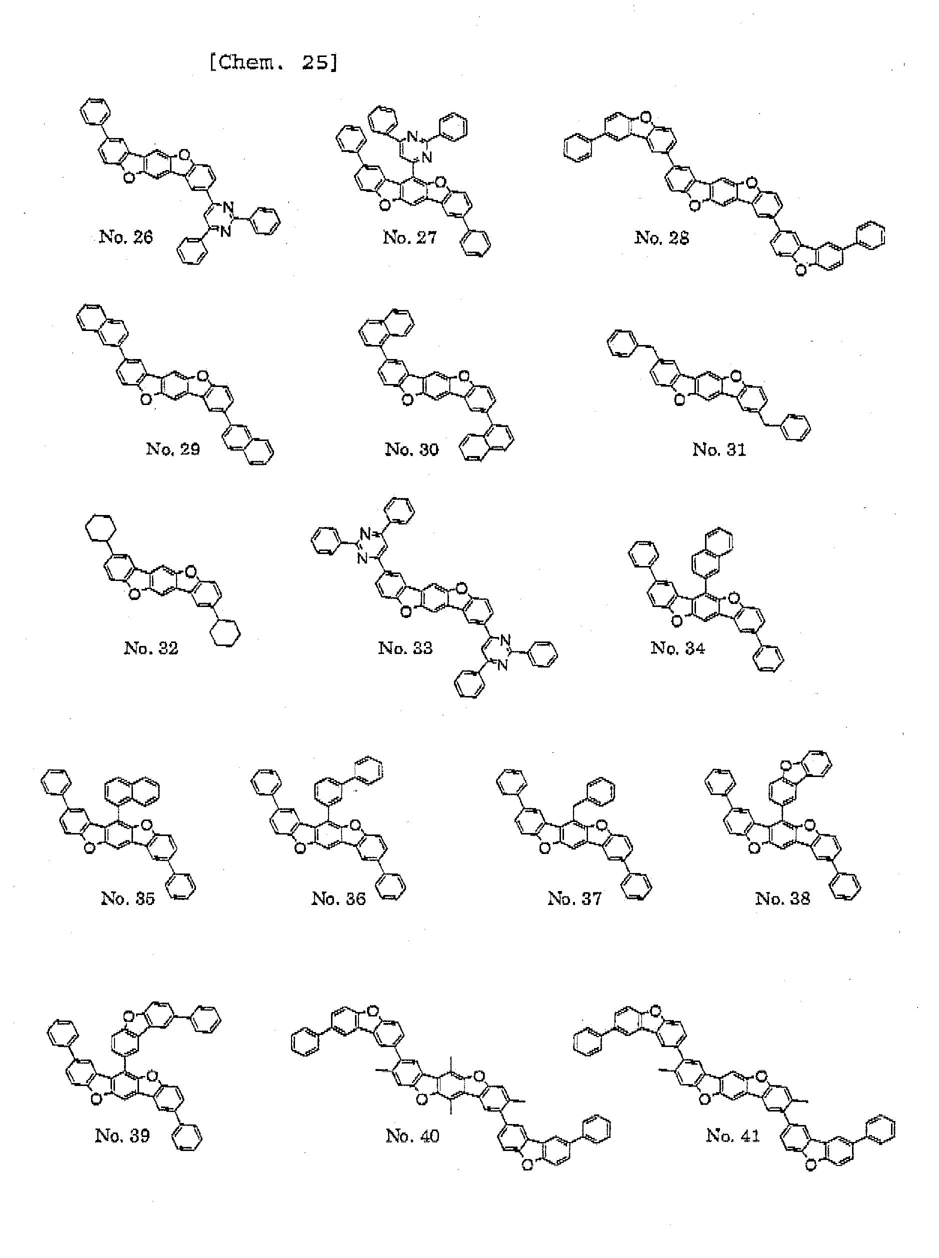

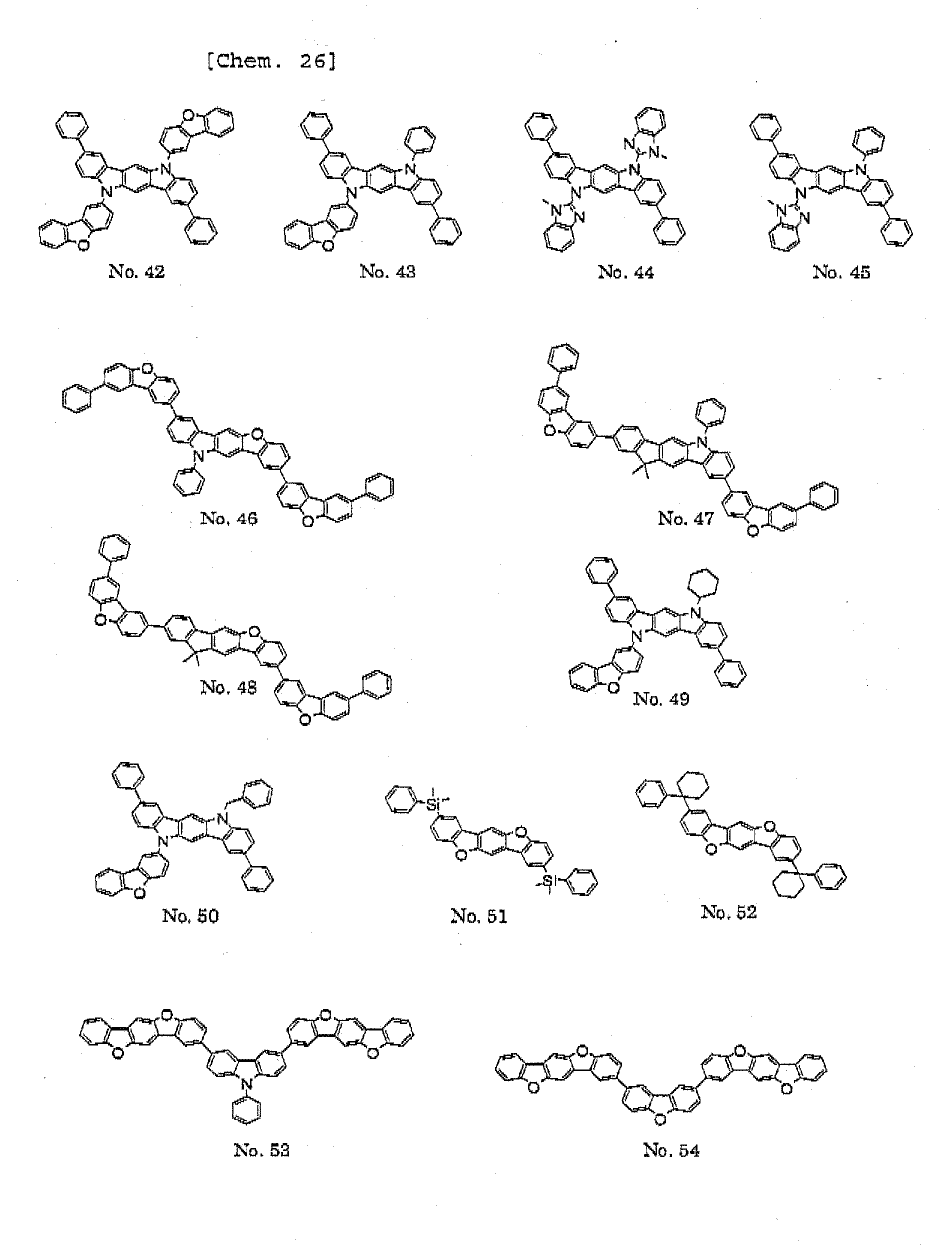

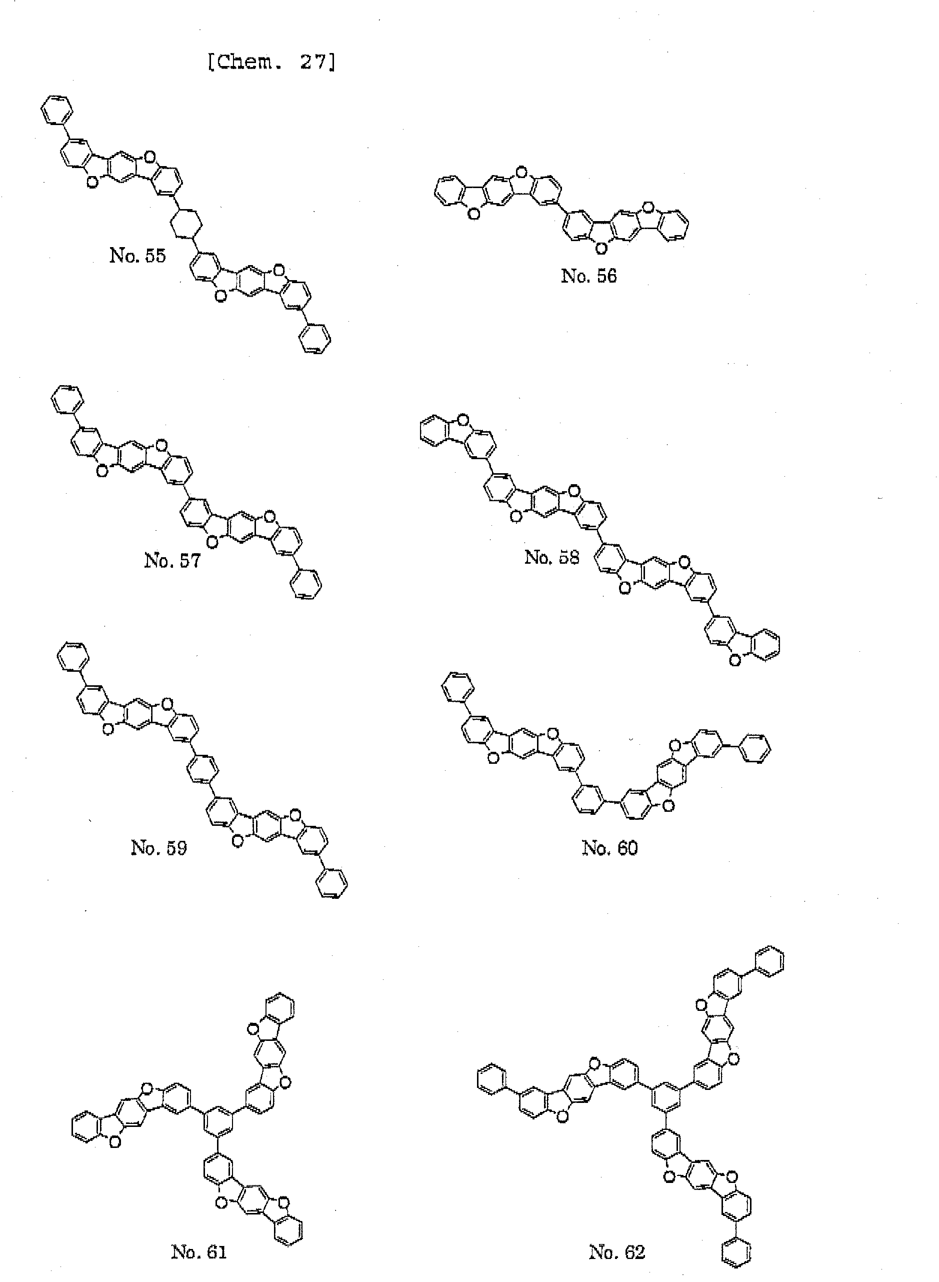

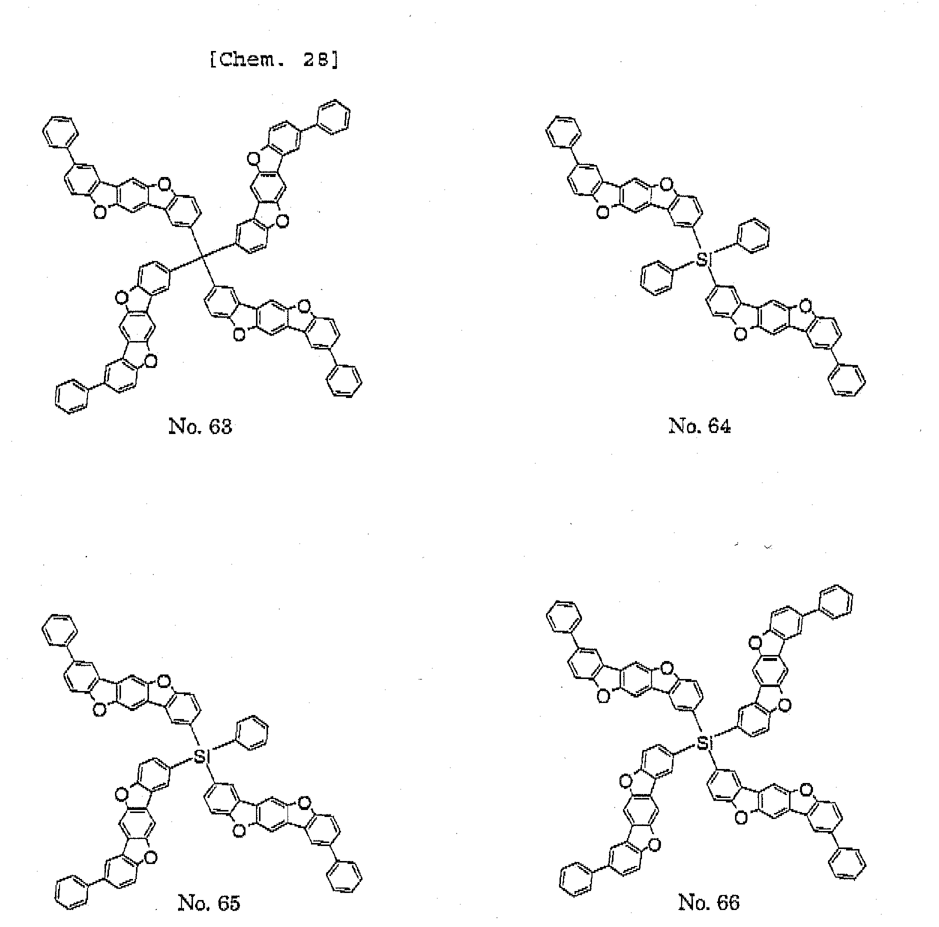

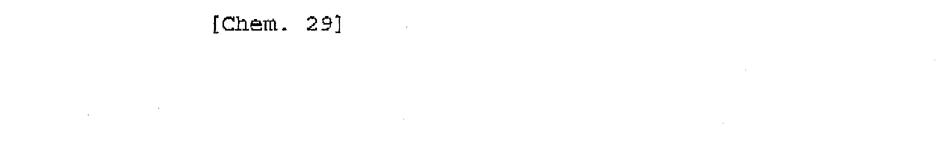

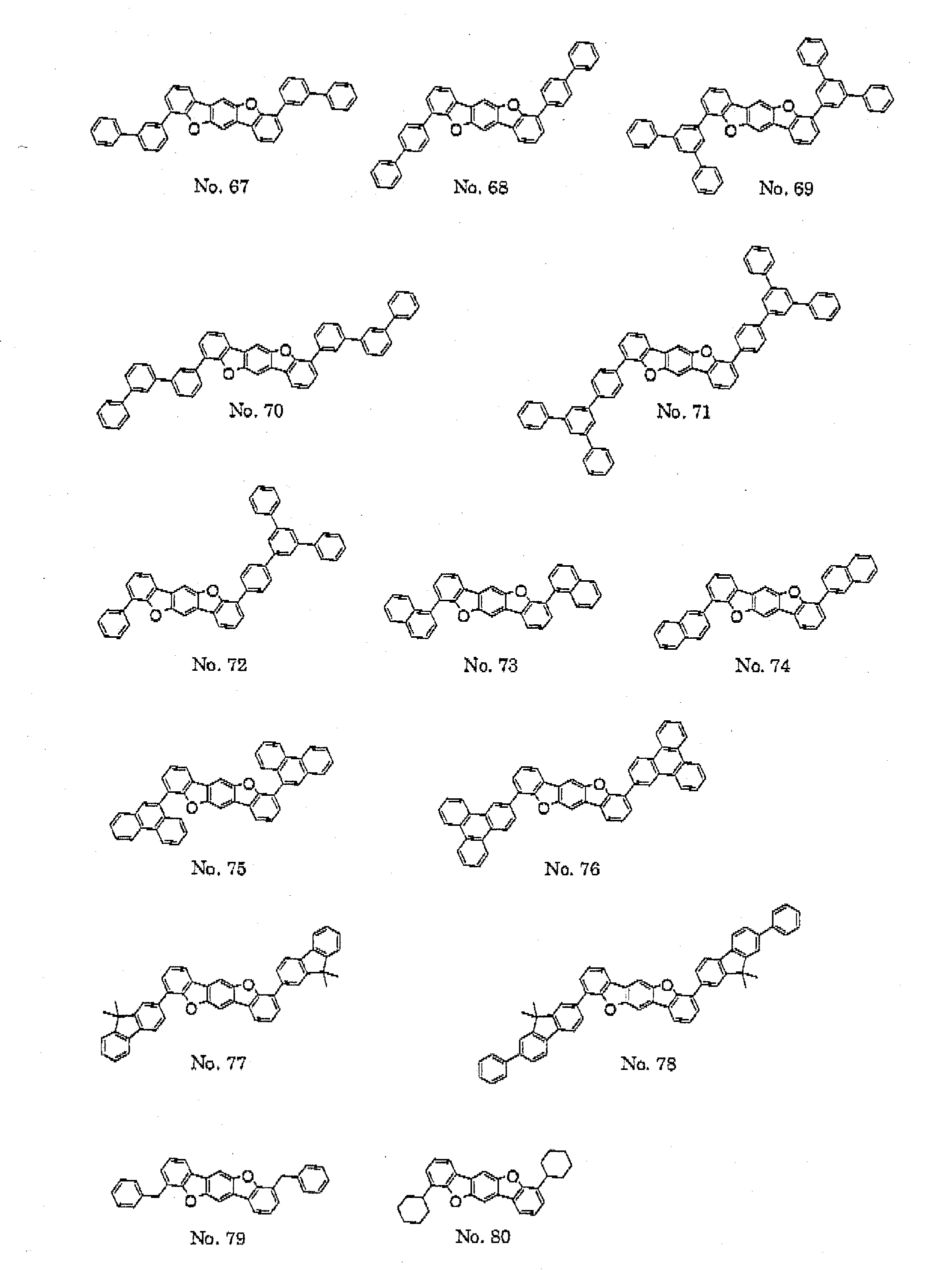

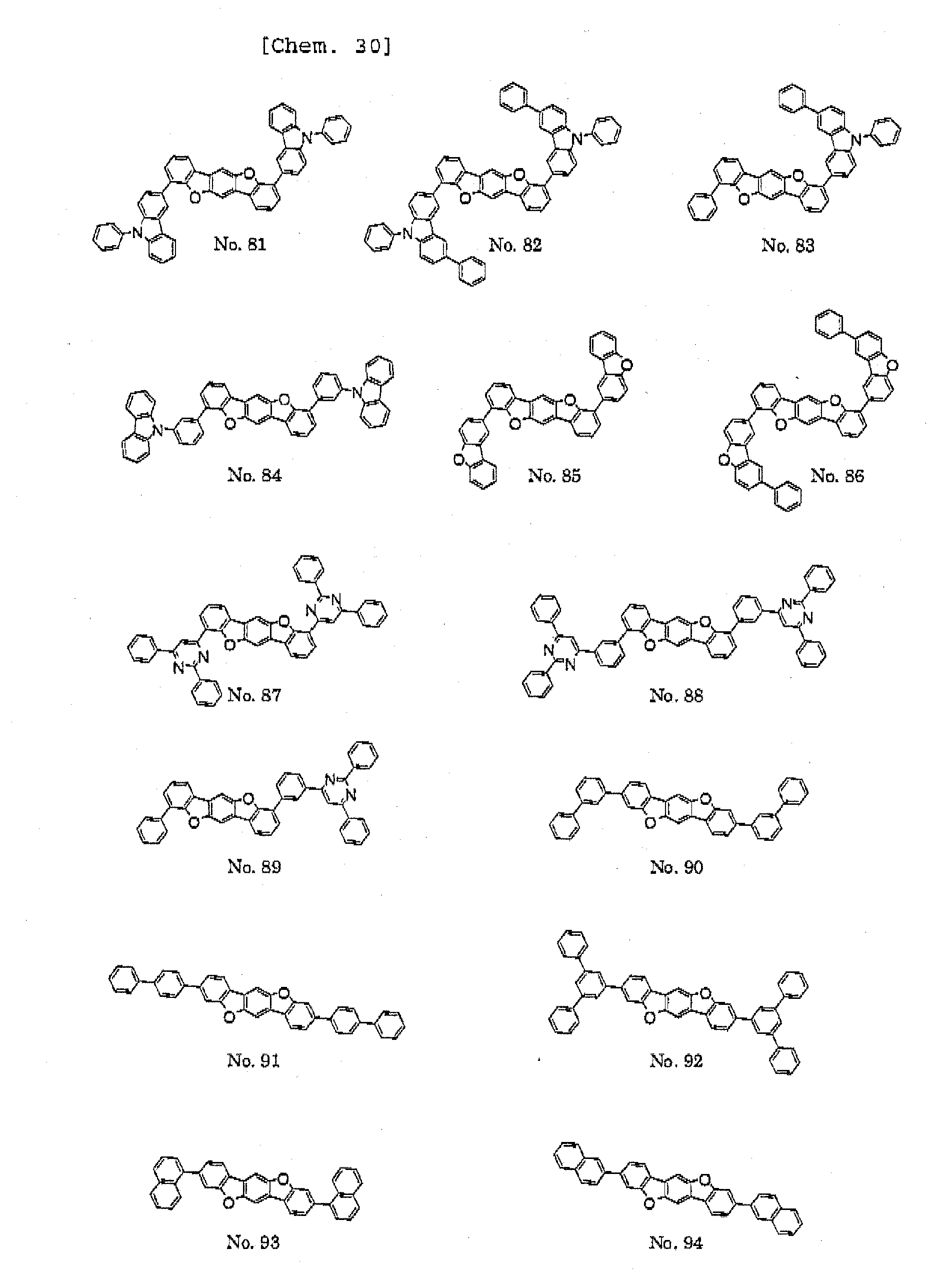

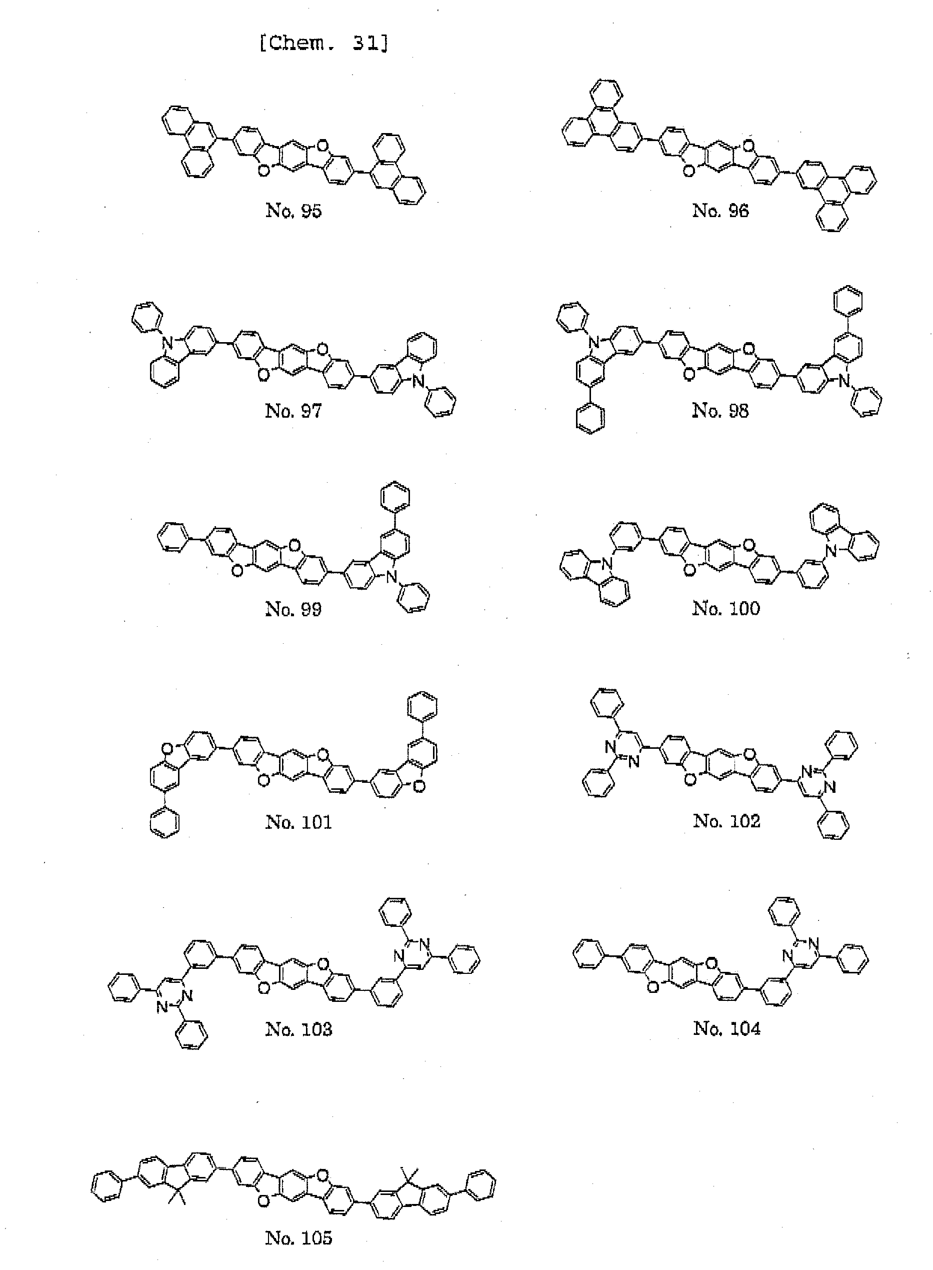

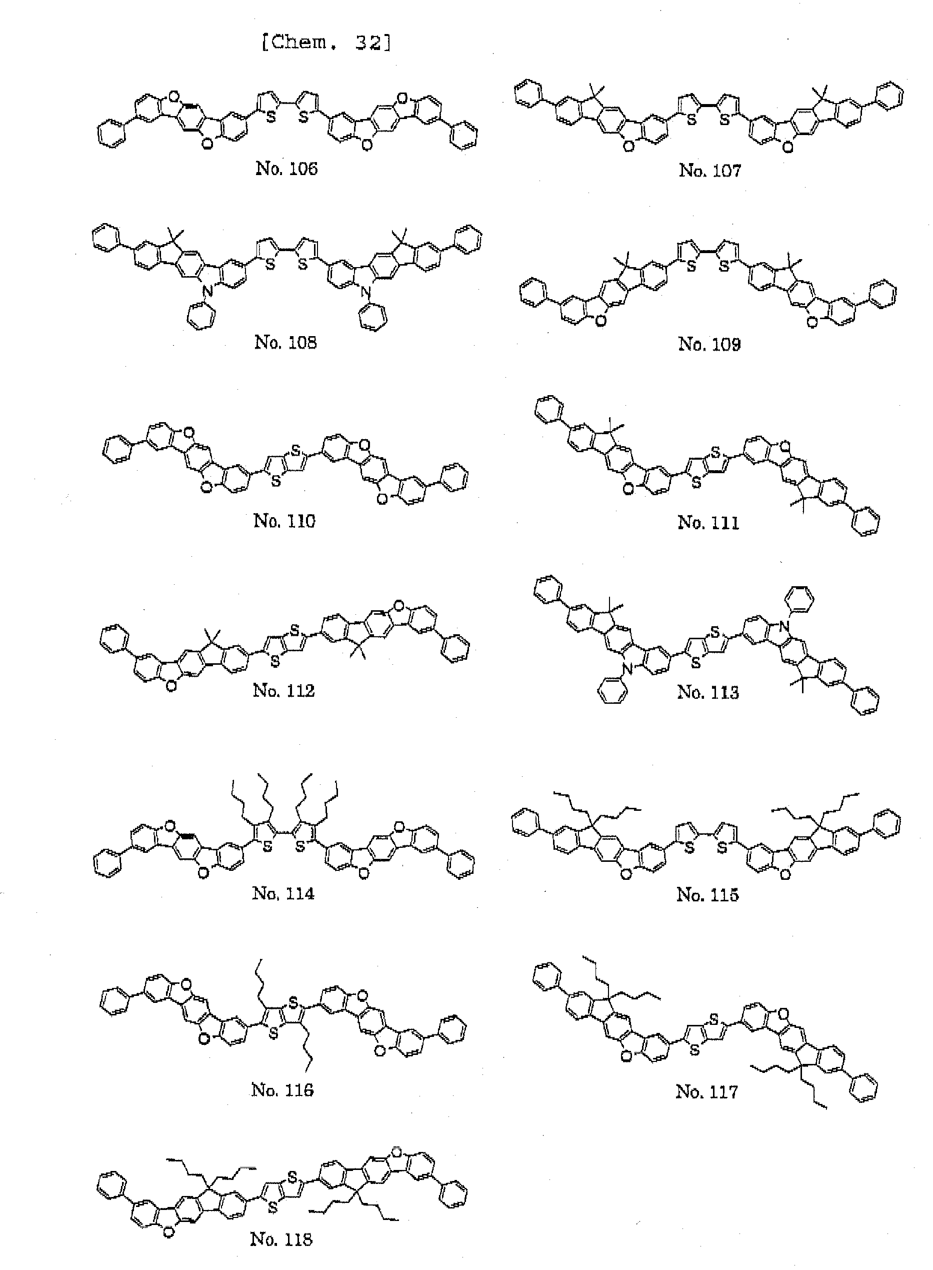

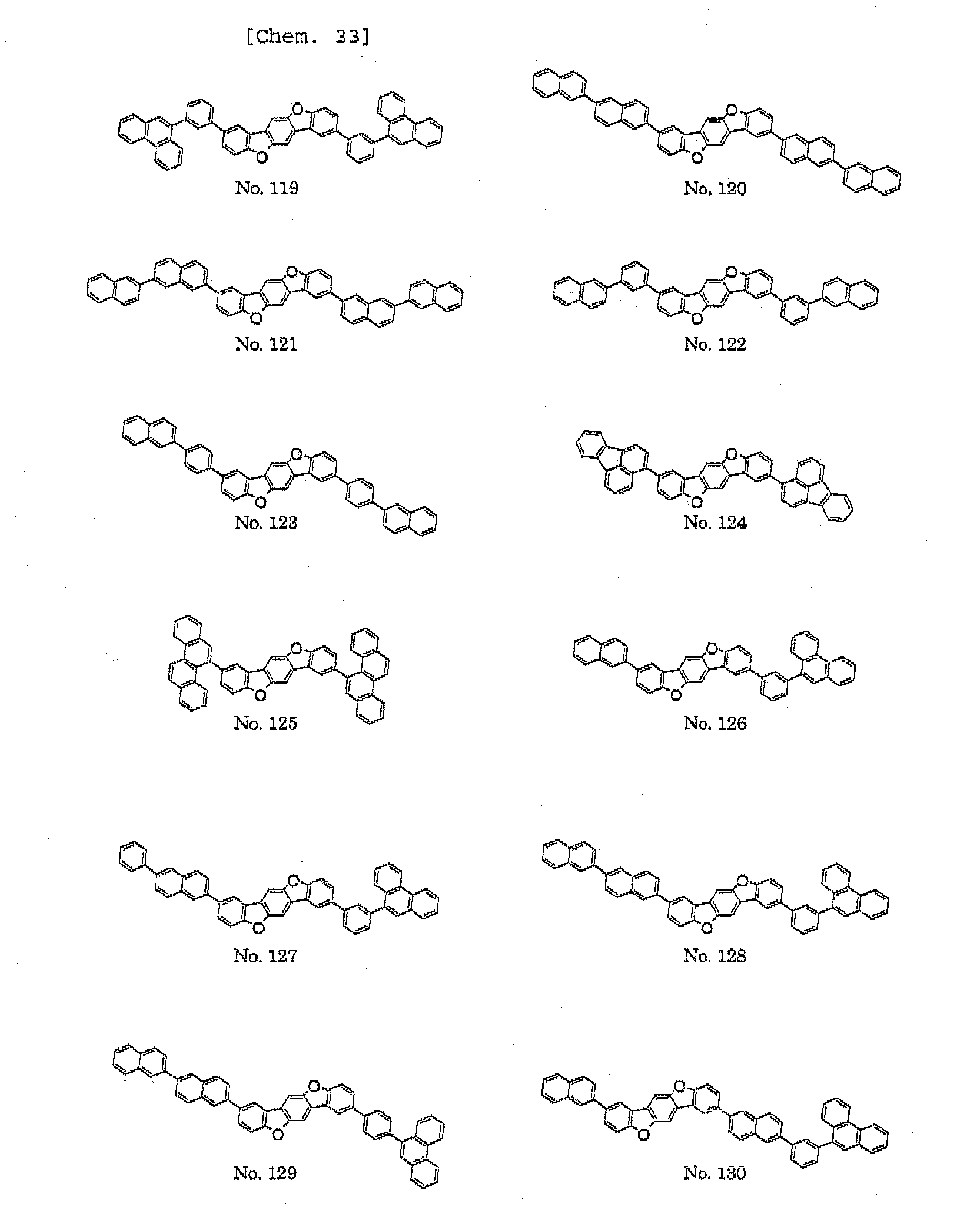

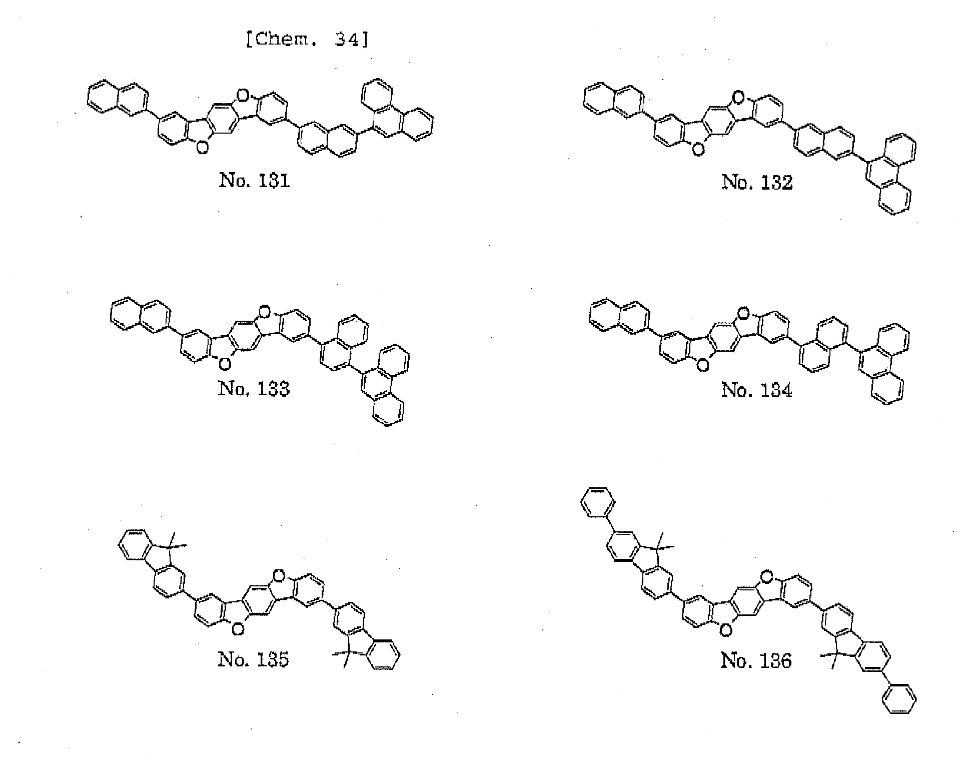

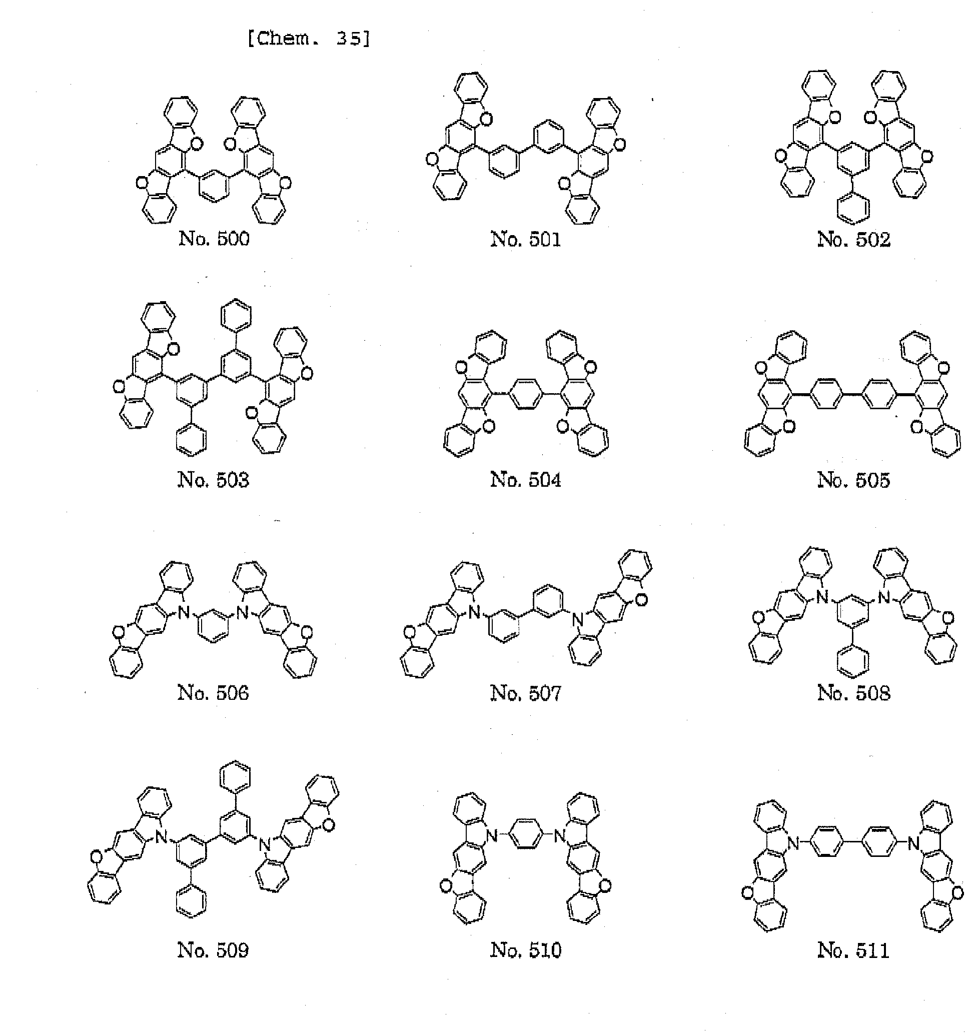

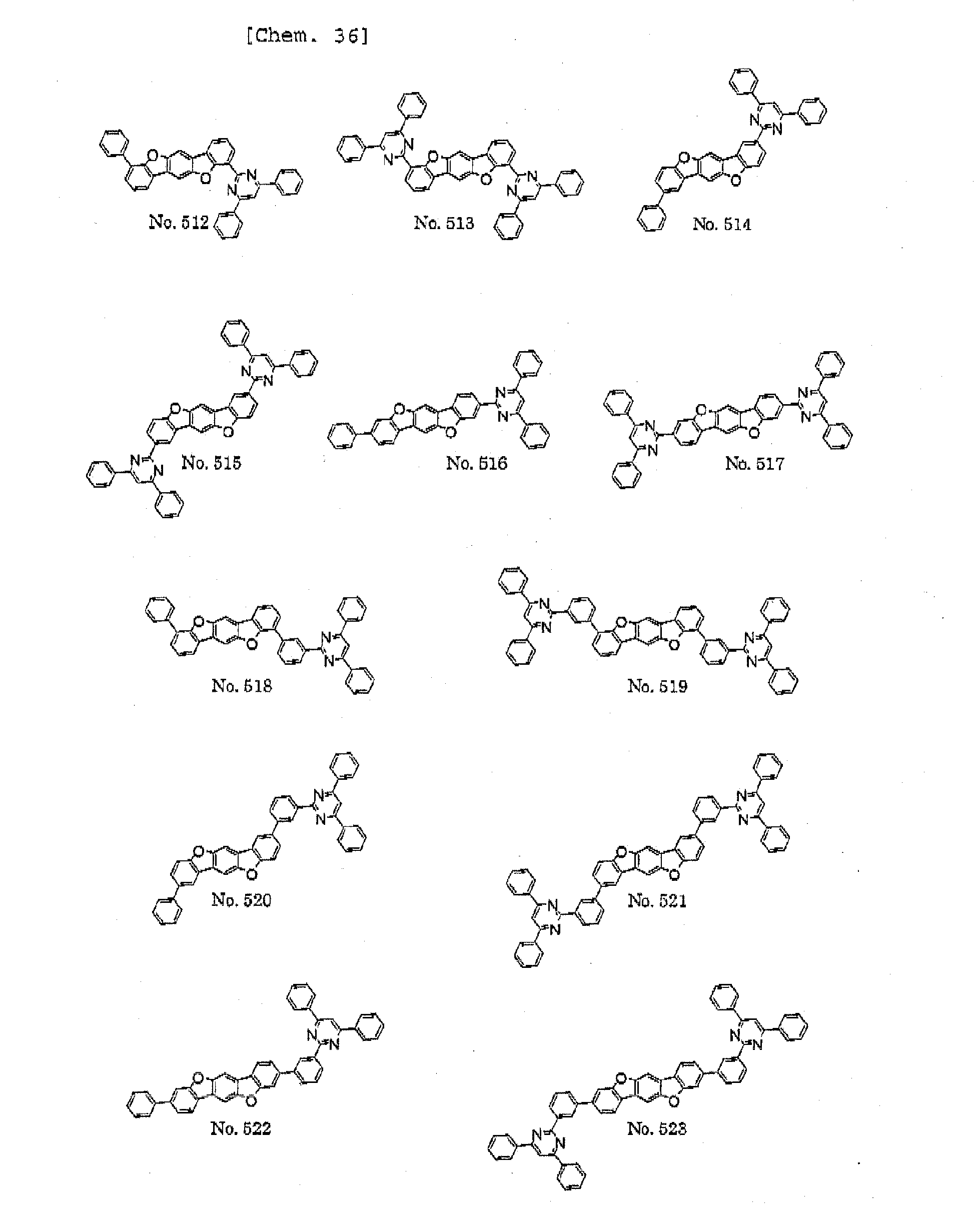

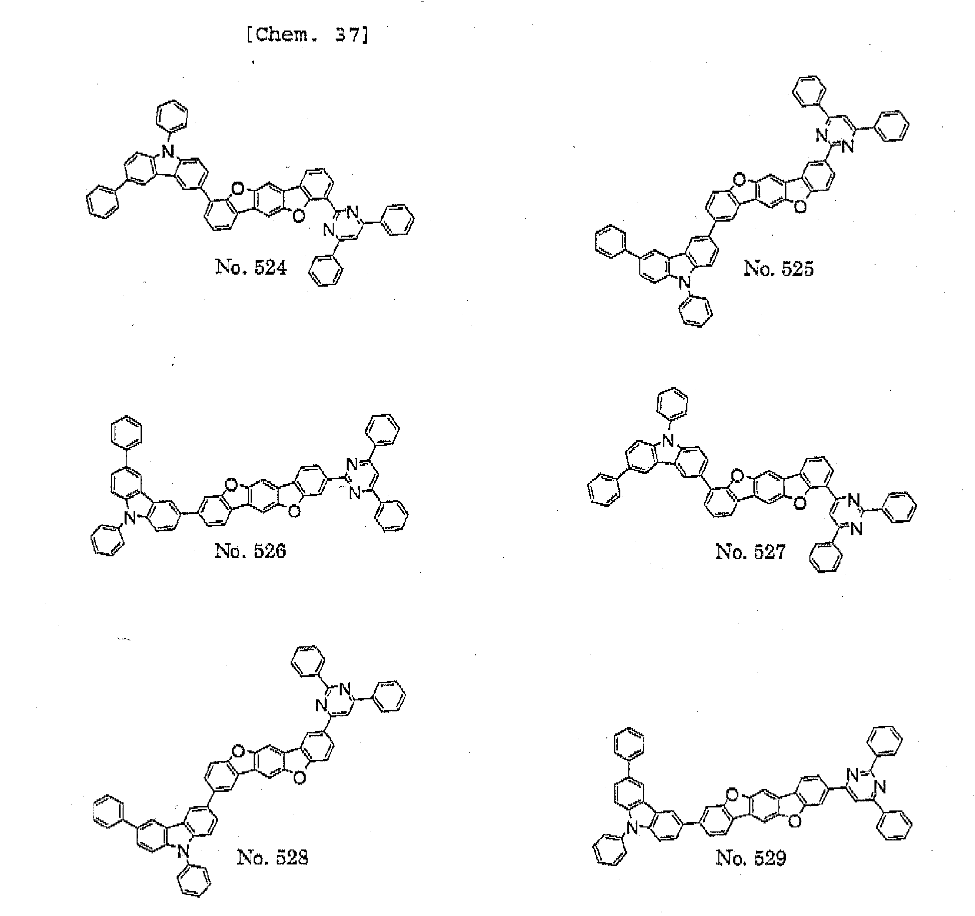

Specific examples of the halogen compound represented by the formula (18) of the present invention are shown below. However, the present invention is not limited to these exemplary compounds.

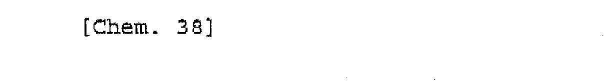

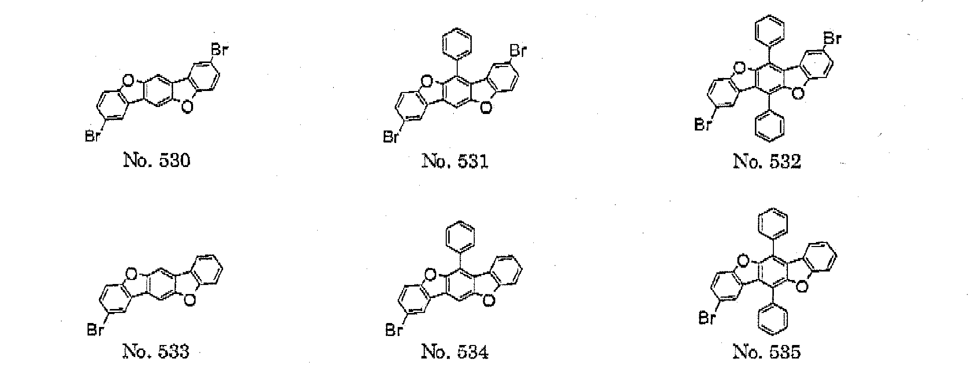

Next, an organic EL device of the present invention is described.
The organic EL device of the present invention has one or more organic thin film layers including a light emitting layer between a cathode and an anode, and at least one layer of the organic thin film layers contains a polycyclic compound having a n-conjugated heteroacene skeleton crosslinked with a carbon atom, a nitrogen atom, an oxygen atom, or a sulfur atom. Specific examples of the n-conjugated heteroacene skeleton are shown below.

Indenofluorene (crosslinked with a carbon atom) 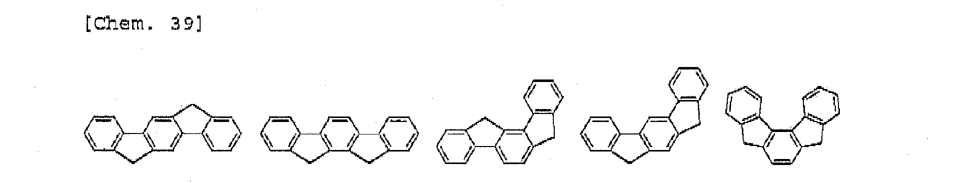 Indolocarbazole (crosslinked with a nitrogen atom) 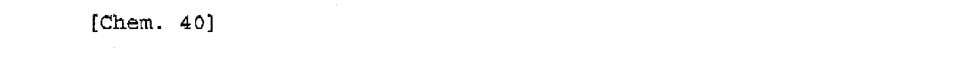 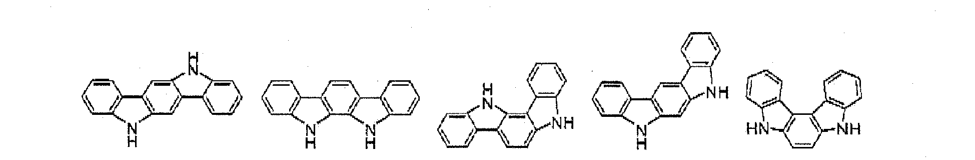 Benzofuranodibenzofuran (crosslinked with an oxygen atom) 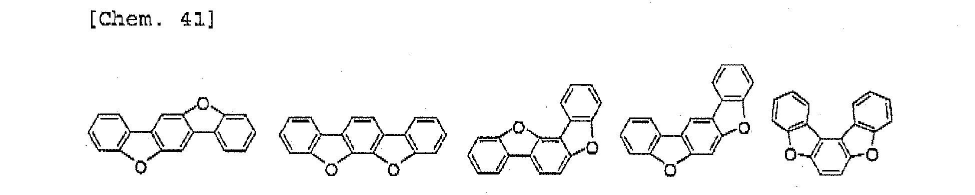 Benzothiophenodibenzothiophene (crosslinked with a sulfur atom) 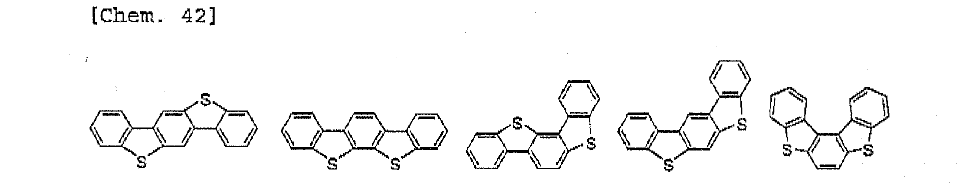 In addition to the foregoing, a n-conjugated heteroacene skeleton crosslinked with a combination of two or more of a carbon atom, a nitrogen atom, an oxygen atom, and a sulfur atom is also permitted. Specific examples of the n-conjugated heteroacene skeleton are shown below. The electron and hole transporting properties can be adjusted by combining two or more of those examples. In particular, a combination of an oxygen atom and a nitrogen atom can achieve compatibility between the electron and hole transporting properties, and reduce the voltage at which the device is driven.

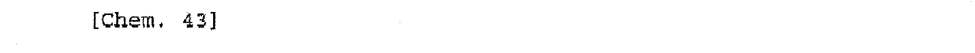

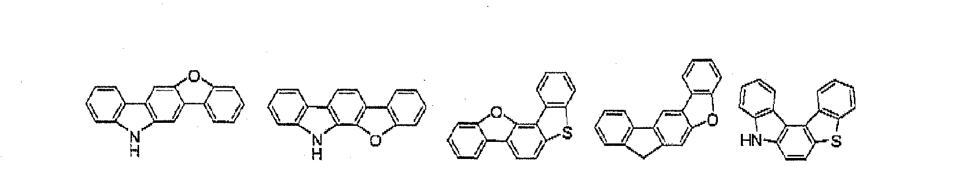

In addition, the above-mentioned polycyclic compound of the present invention is preferablyused as the polycyclic compound having a n-conjugated heteroacene skeleton in the organic EL device of the present invention.
The organic EL device may have an electron transporting layer between the light emitting layer and the cathode, and the electron transporting layer may contain the polycyclic compound. Further, both the light emitting layer and the electron transporting layer each preferably contain the polycyclic compound.
Alternatively, the organic EL device may have a hole transporting layer between the light emitting layer and the anode, and the hole transporting layer may contain the polycyclic compound.
Further, the polycyclic compound of the present invention is preferably incorporated into at least the light emitting layer. When the compound is used in the light emitting layer, the lifetime of the organic EL device can be lengthened. When the compound is used in the electron transporting layer or the electron injecting layer, the voltage at which the device is driven can be reduced. The compound is preferably incorporated into each of two or more layers including the light emitting layer and the electron transporting layer or the electron injecting layer at the same time because both the reduced voltage and the lengthened lifetime can be achieved.
In particular, in addition to the electron transporting layer or the electron injecting layer, the light emitting layer preferably contains the polycyclic compound of the present invention as a host material and preferably contains the material for an organic EL device represented by any one of the following formulae (20) to (27) as a host material.

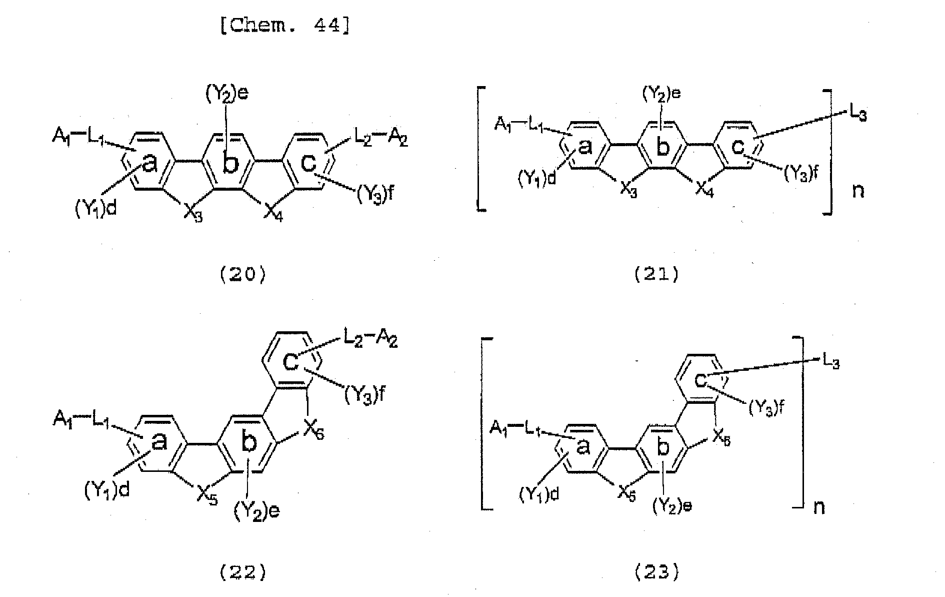

[In the formulae (20) to (23), X₃, X₄, X₅, and X₆ each independently represent oxygen (O), sulfur (S), N-R₁, or CR₂R₃, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₃ and X₄ or both X₅ and X₆ represent N-R₁, at least one R₁ represents a substituted or unsubstituted, monovalent fused aromatic heterocyclic group having a ring formed of 8 to 24 atoms.
In the formulae (21) and (23), n represents 2, 3, or 4, and the compound represented by one of the formulae (21) and (23) includes a dimer using L₃ as a linking group for n=2, a trimer using L₃ as a linking group for n=3, or a tetramer using L₃ as a linking group for n=4.
In the formulae (20) to (23), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond.
In the formulae (20) and (22), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond, provided that, when both X₃ and X₄ or both X₅ and X₆ represent CR₂R₃ and both L₁ and L₂ represent substituted or unsubstituted, divalent aromatic hydrocarbon groups each having a ring formed of 6 to 24 carbon atoms, a case where L₁ and L₂ are simultaneously linked at para positions with respect to a benzene ring b is excluded.
In the formulae (21) and (23), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond, provided that, when both X₃ and X₄ or both X₅ and X₆ represent CR₂R₃ and both L₁ and L₃ represent substituted or unsubstituted, divalent, trivalent, or tetravalent aromatic hydrocarbon groups each having a ring formed of 6 to 24 carbon atoms, a case where L₁ and L₃ are simultaneously linked at para positions with respect to the benzene ring b is excluded.
In the formulae (20) to (23), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formulae (20) and (22), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formulae (20) to (23), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0, 1, or 2, provided that, when both X₃ and X₄ or both X₅ and X₆ represent oxygen (0), sulfur (S), or CR₂R₃, both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.
In the formulae (20) to (23), A₁, A₂, L₁, L₂, and L₃ are each free of any carbonyl group.]

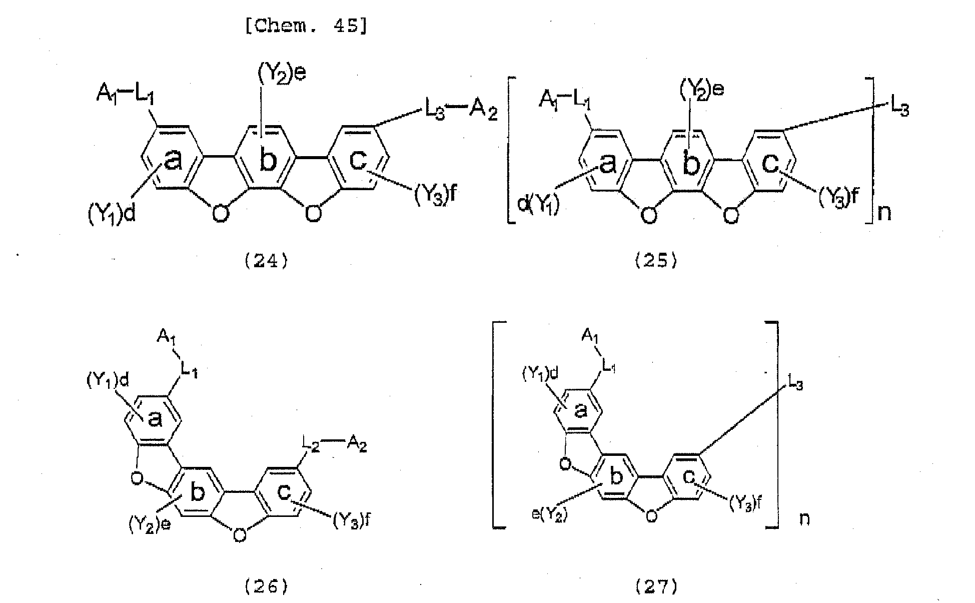

[In the formulae (25) and (27), n represents 2, 3, or 4, and the compound represented by one of the formulae (25) and (27) includes a dimer using L₃ as a linking group for n=2, a trimer using L₃ as a linking group for n=3, or a tetramer using L₃ as a linking group for n=4. In the formulae (24) to (27), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond. In the formulae (24) and (26), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond. In the formulae (25) and (27), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond. In the formulae (24) to (27), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded.
In the formulae (24) and (26), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded.
In the formulae (24) to (27), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent the number of 0, 1, 2, or 3, and e represents the number of 0, 1, or 2, provided that, when both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded.
In the formulae (24) to (27), A₁, A₂, L₁, L₂, and L₃ are each free of any carboxyl groups.]

In the formulae (20) to (27), examples of the respective groups represented by Y₁ to Y₃, R₁ to R₃, L₁ to L₃, and A₁ and A₂, and examples of the substituents of the groups include the same examples given in the formulae (1) to (19),
Specific examples of the material for an organic EL device represented by the formulae (20) to (27) of the present invention are shown below. However, the present invention is not limited to these exemplary compounds.

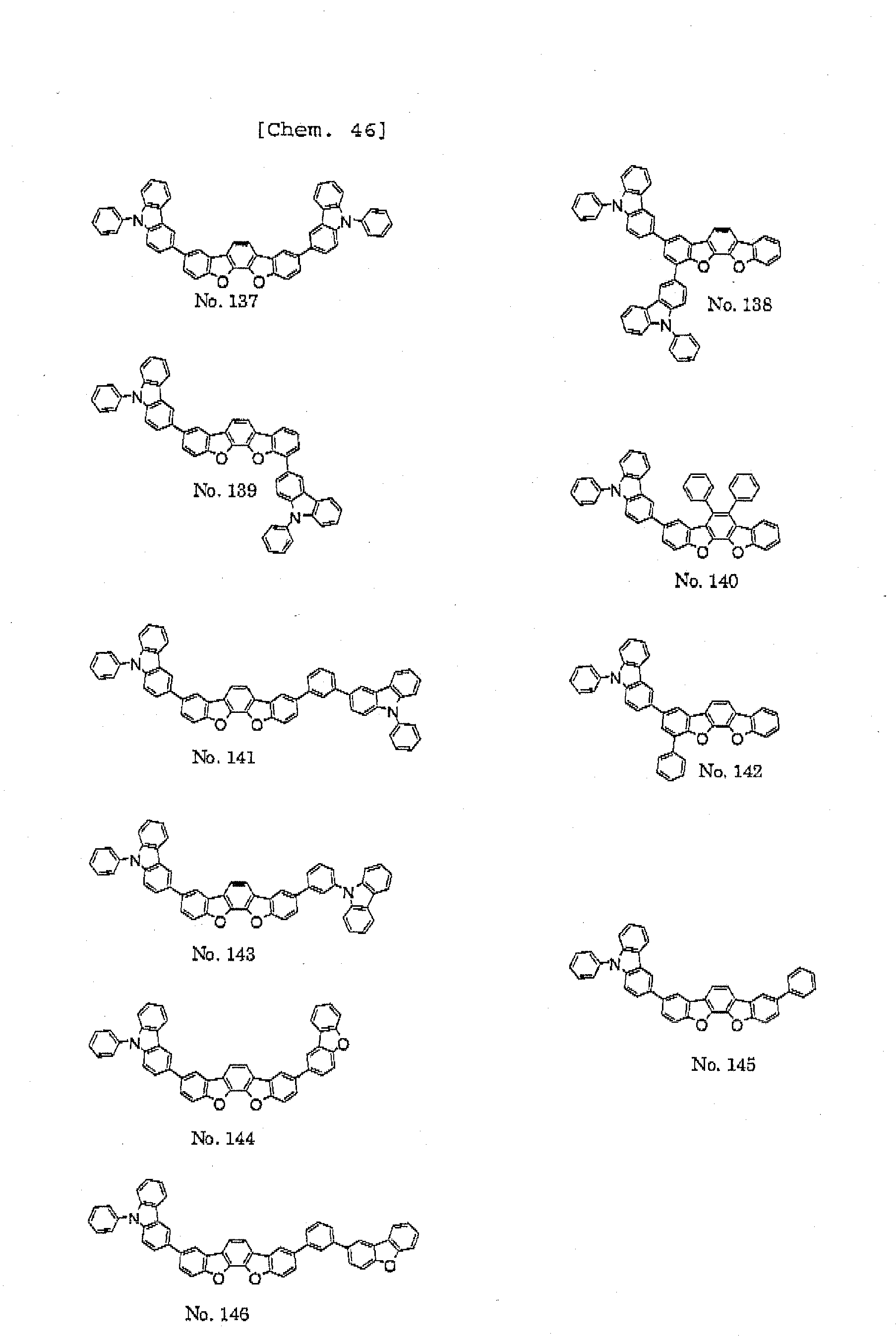

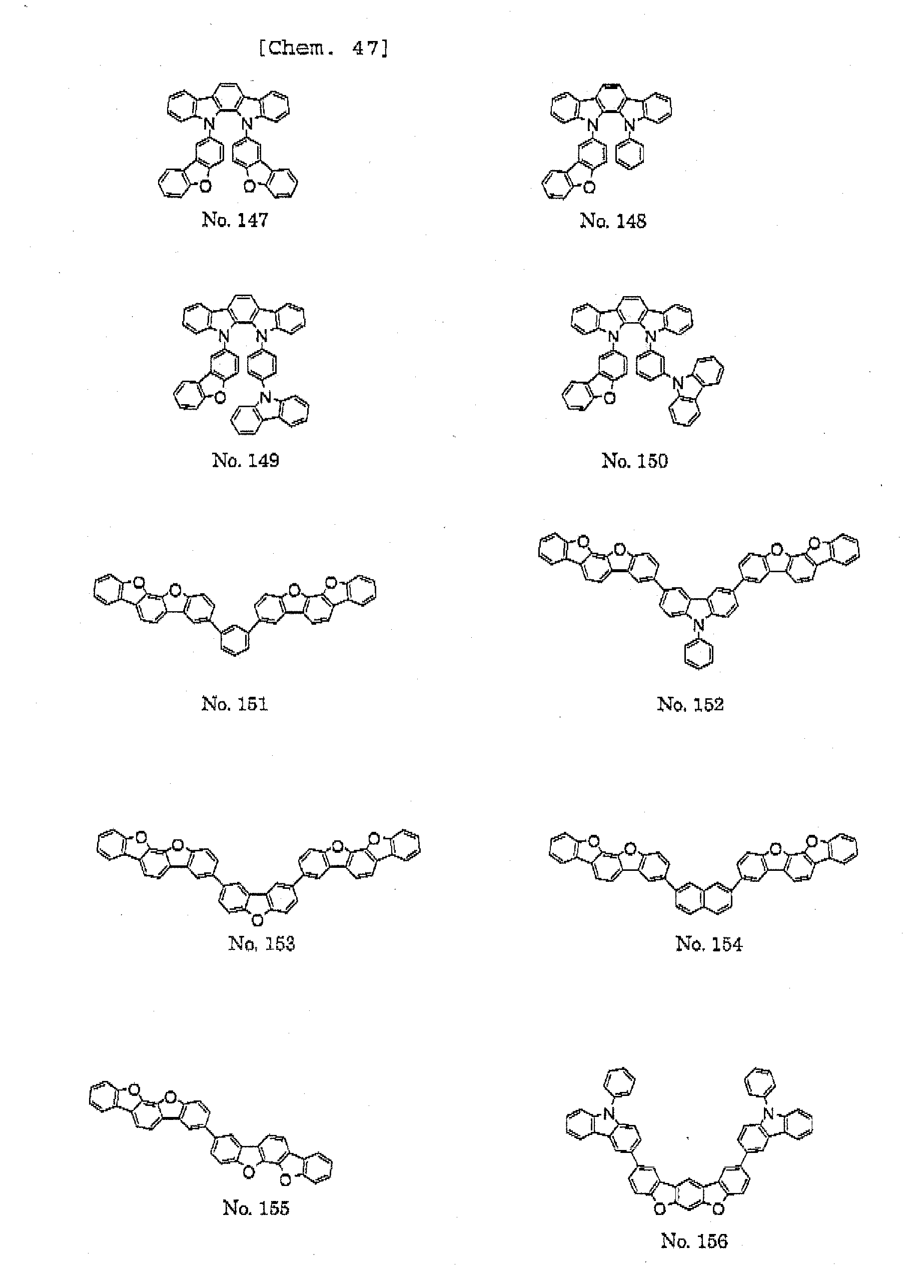

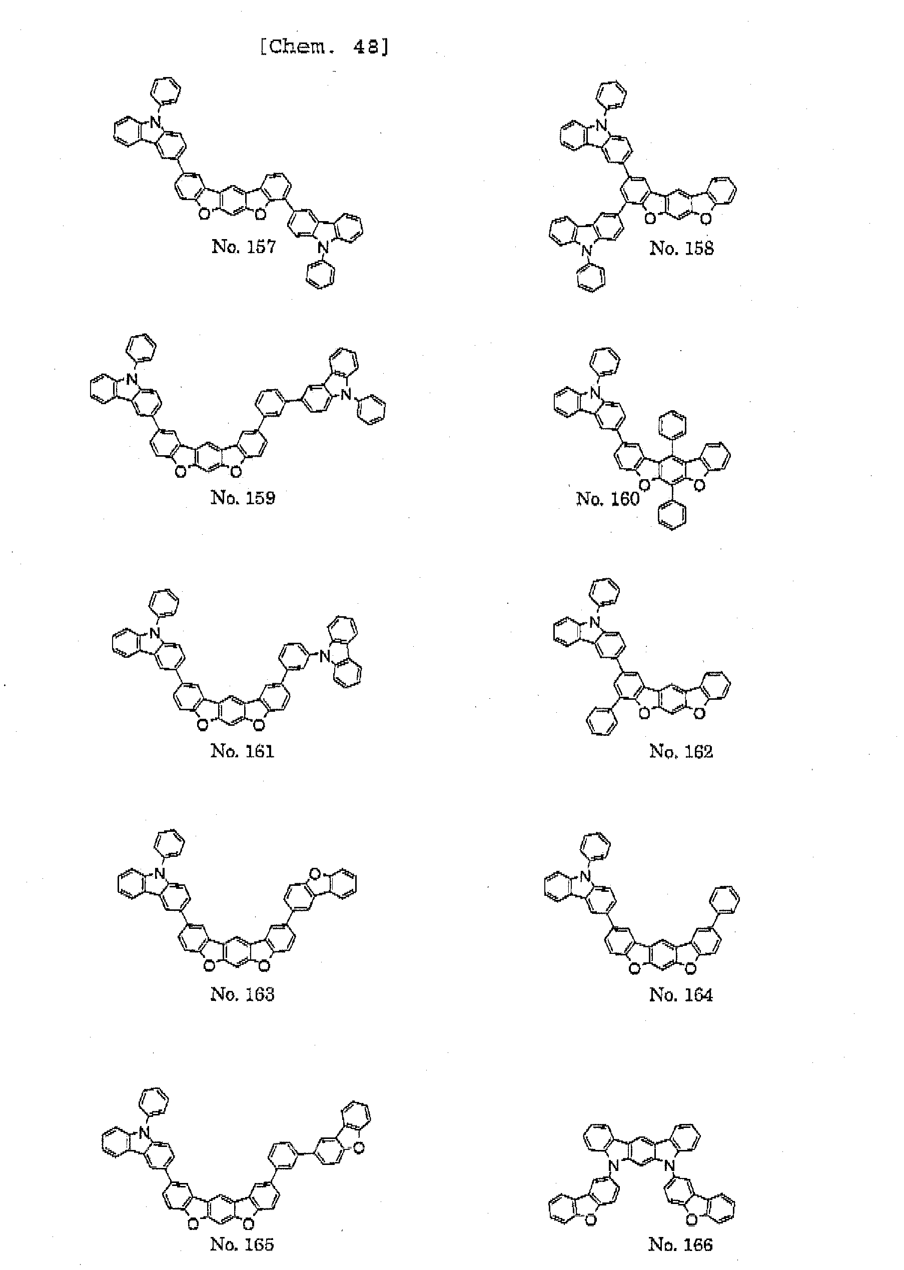

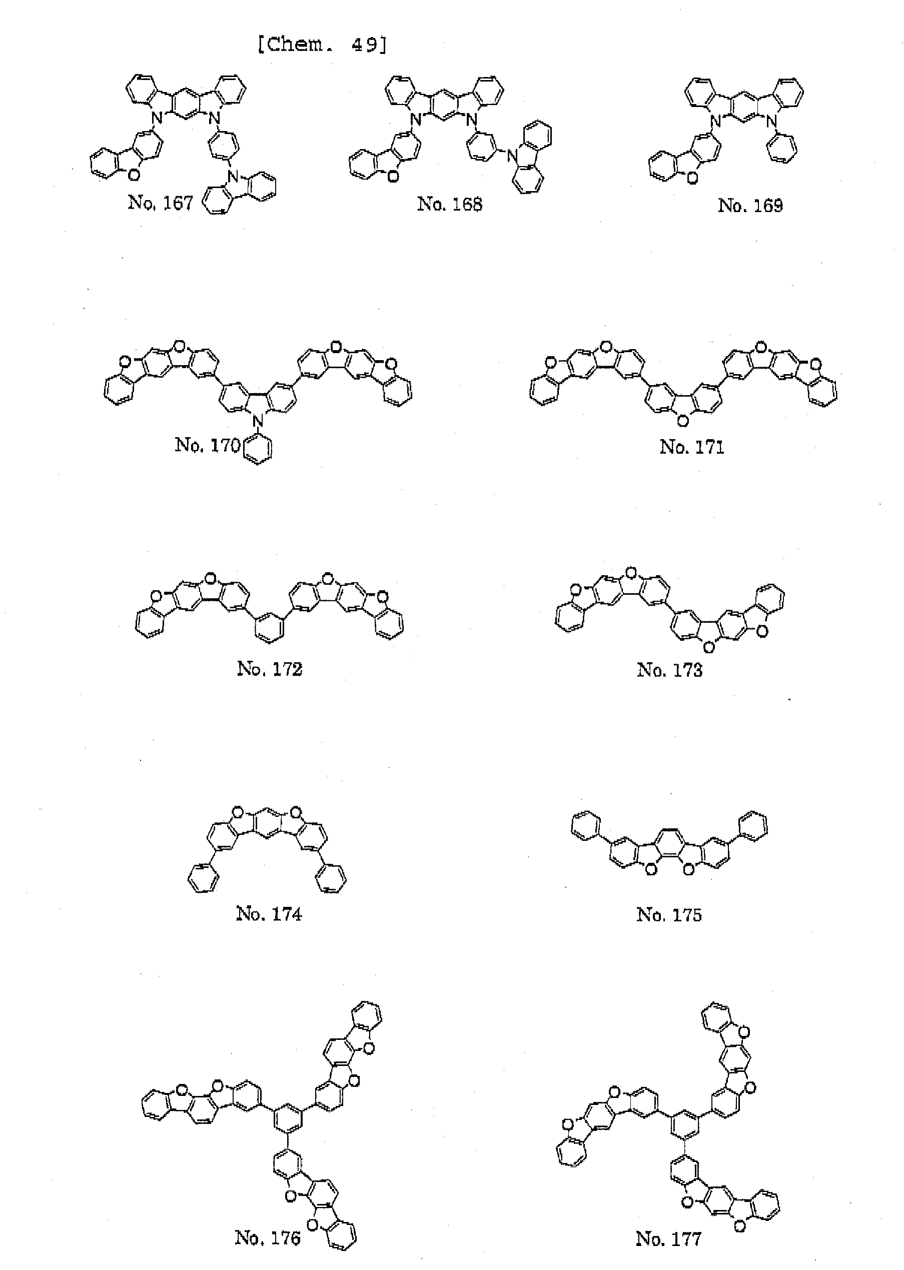

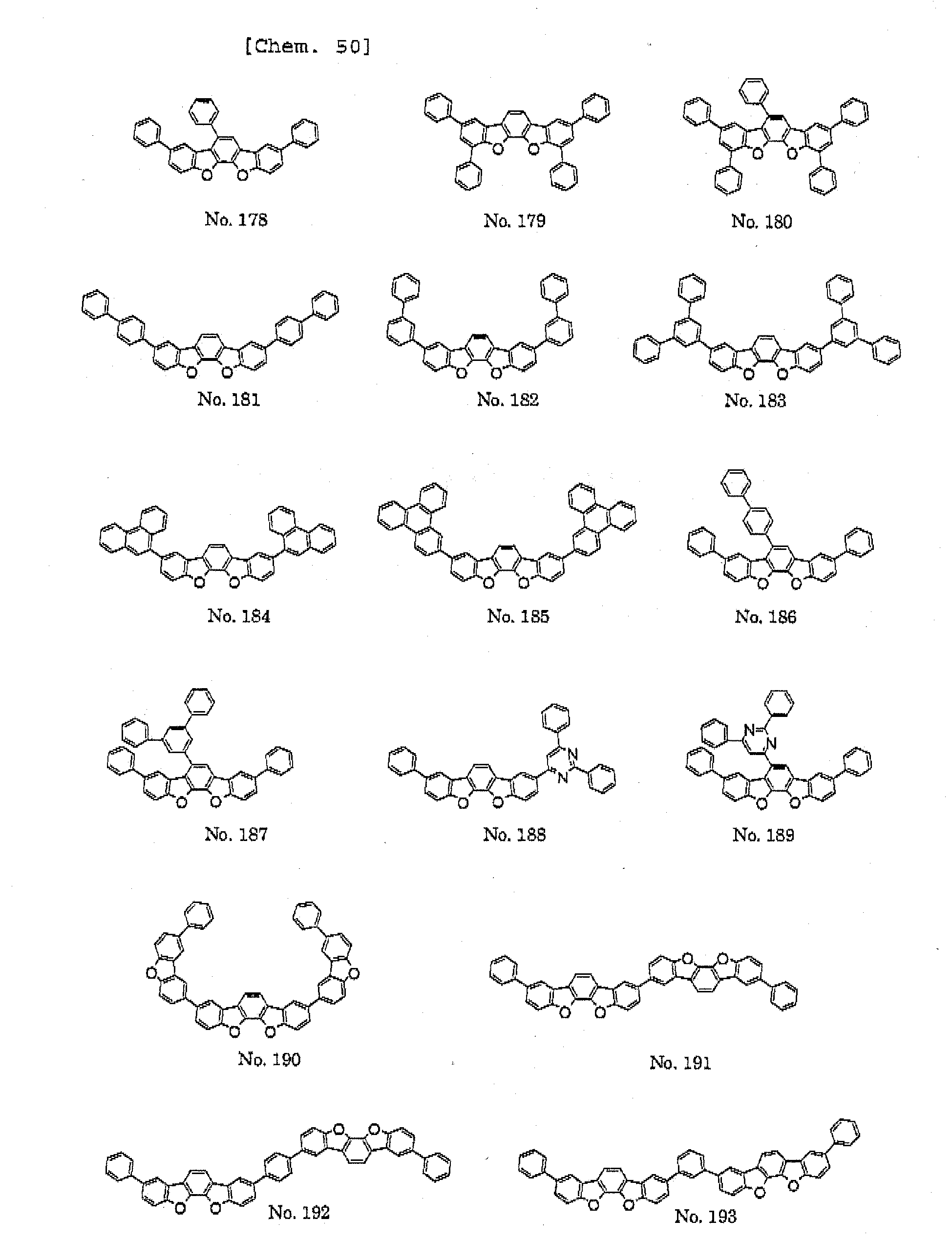

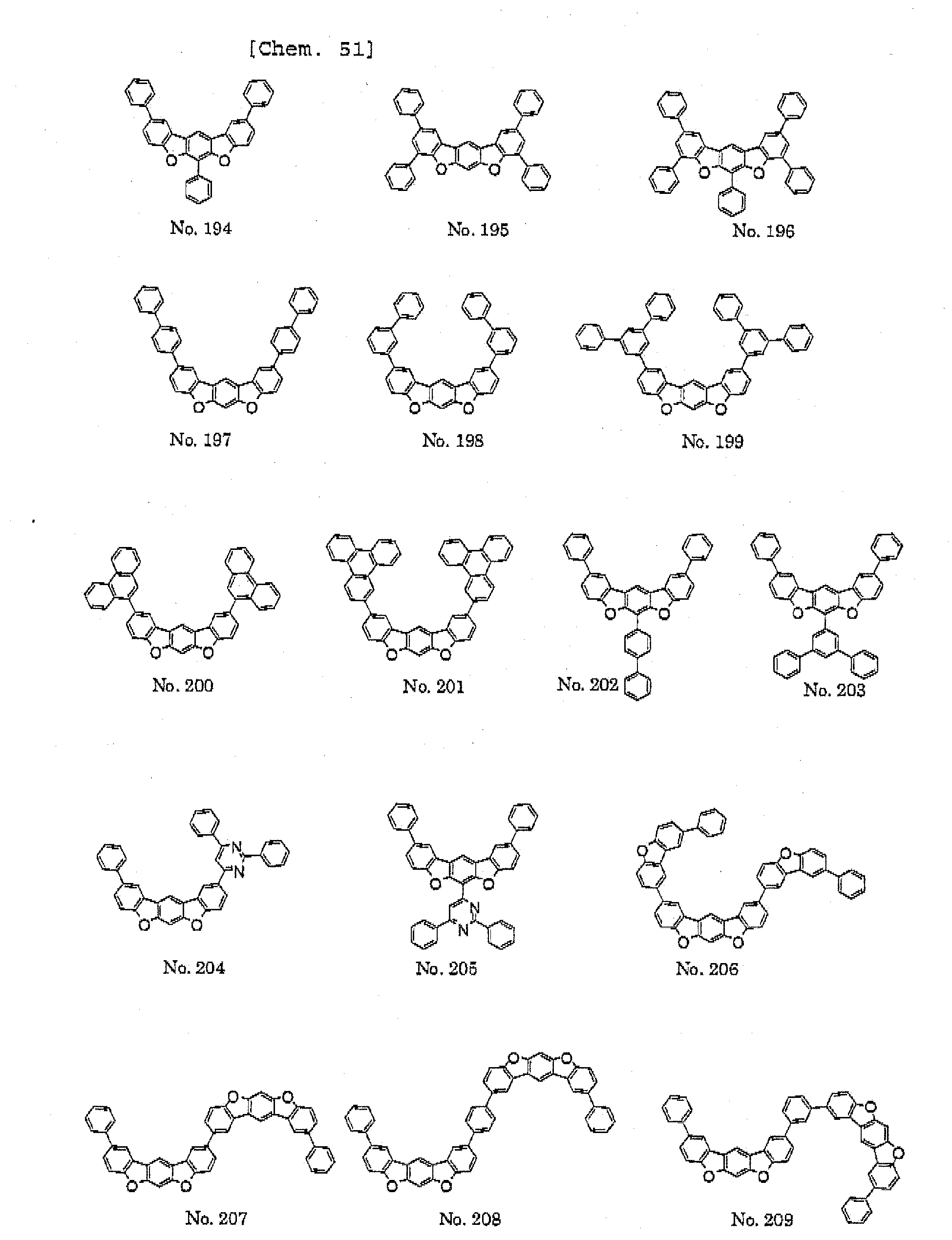

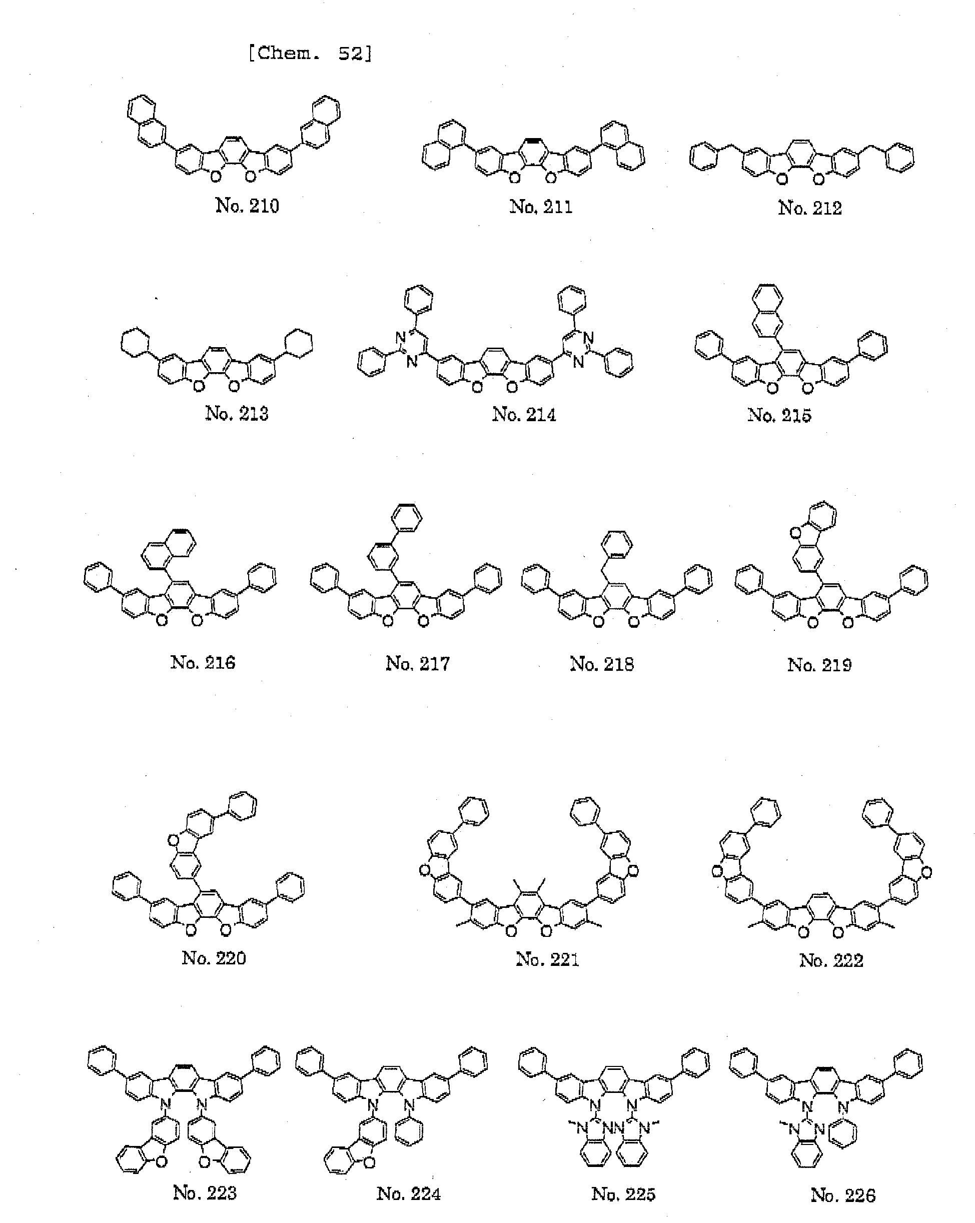

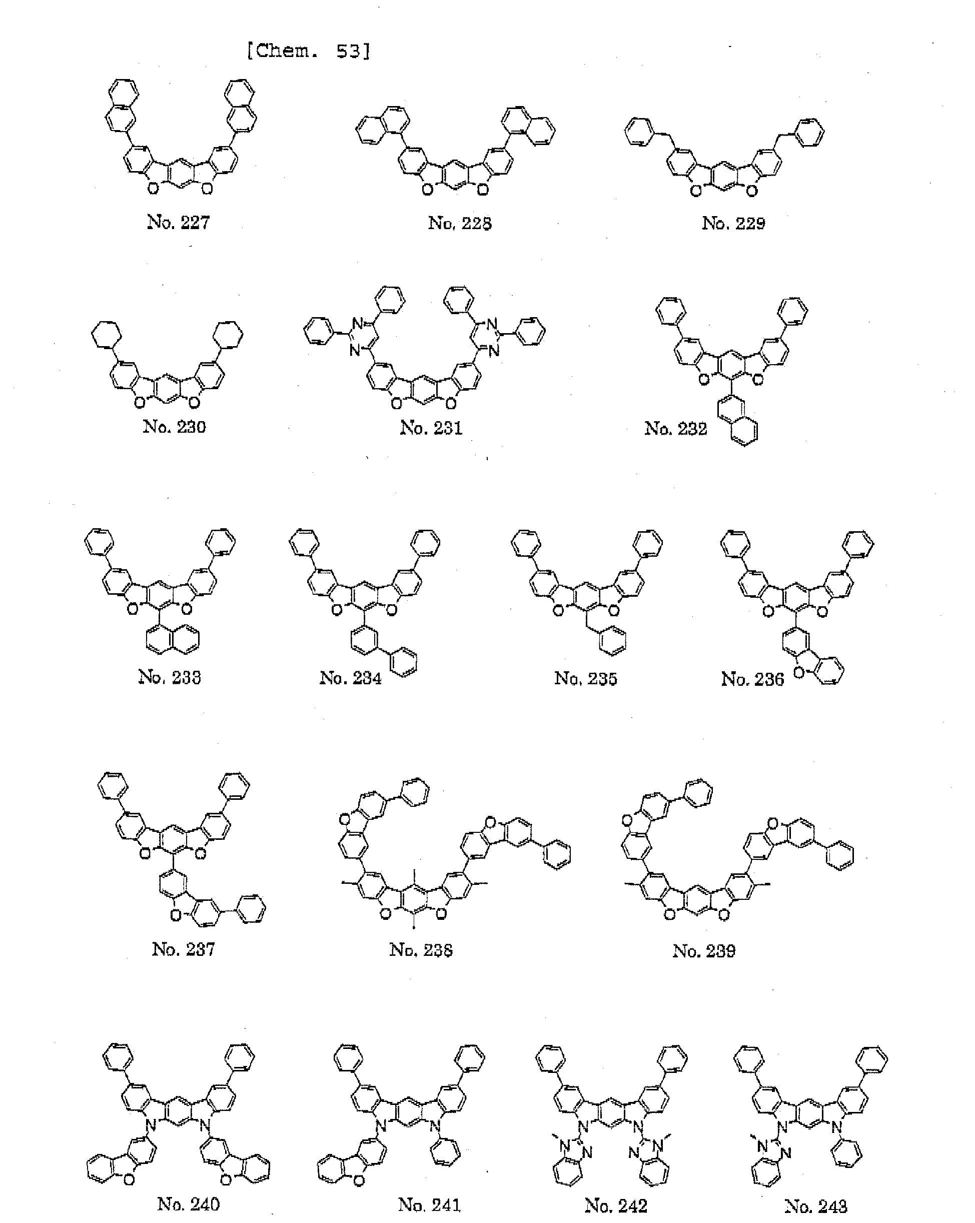

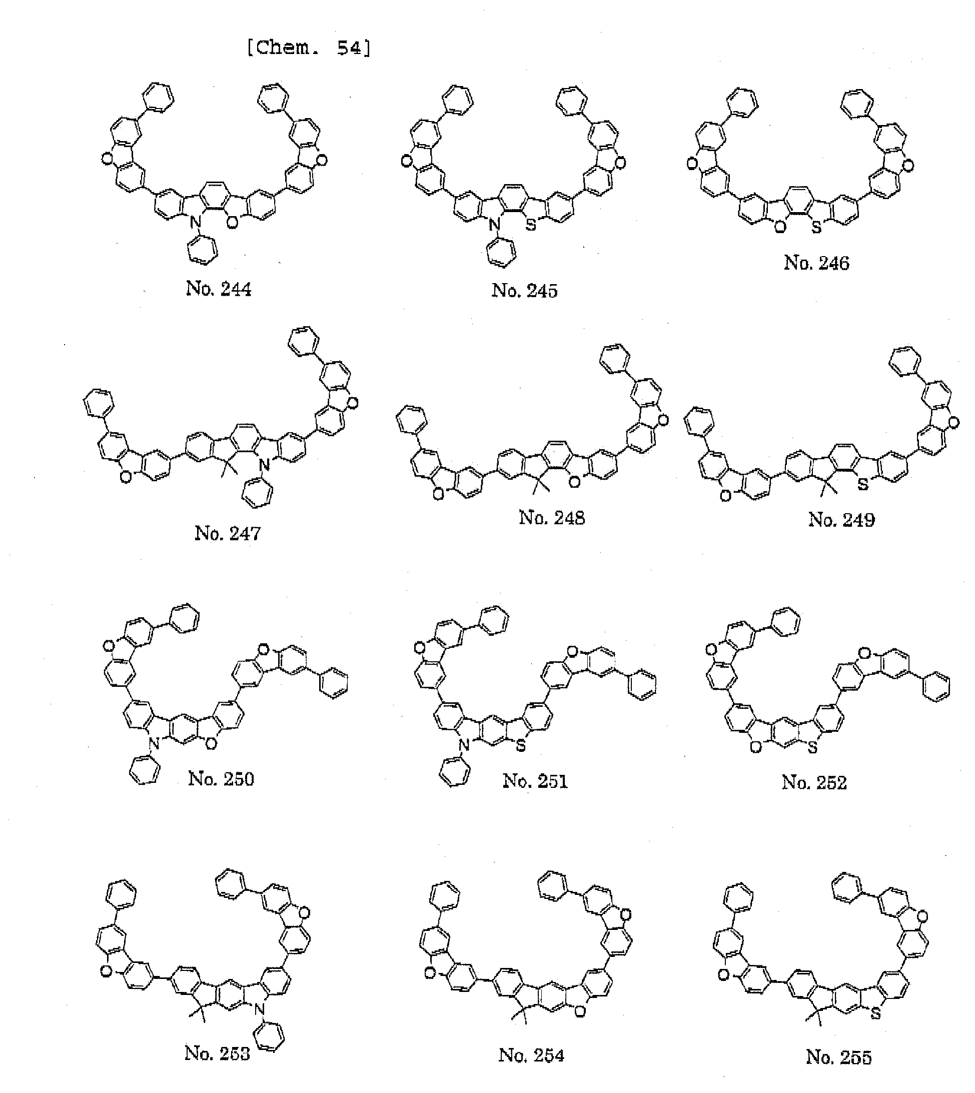

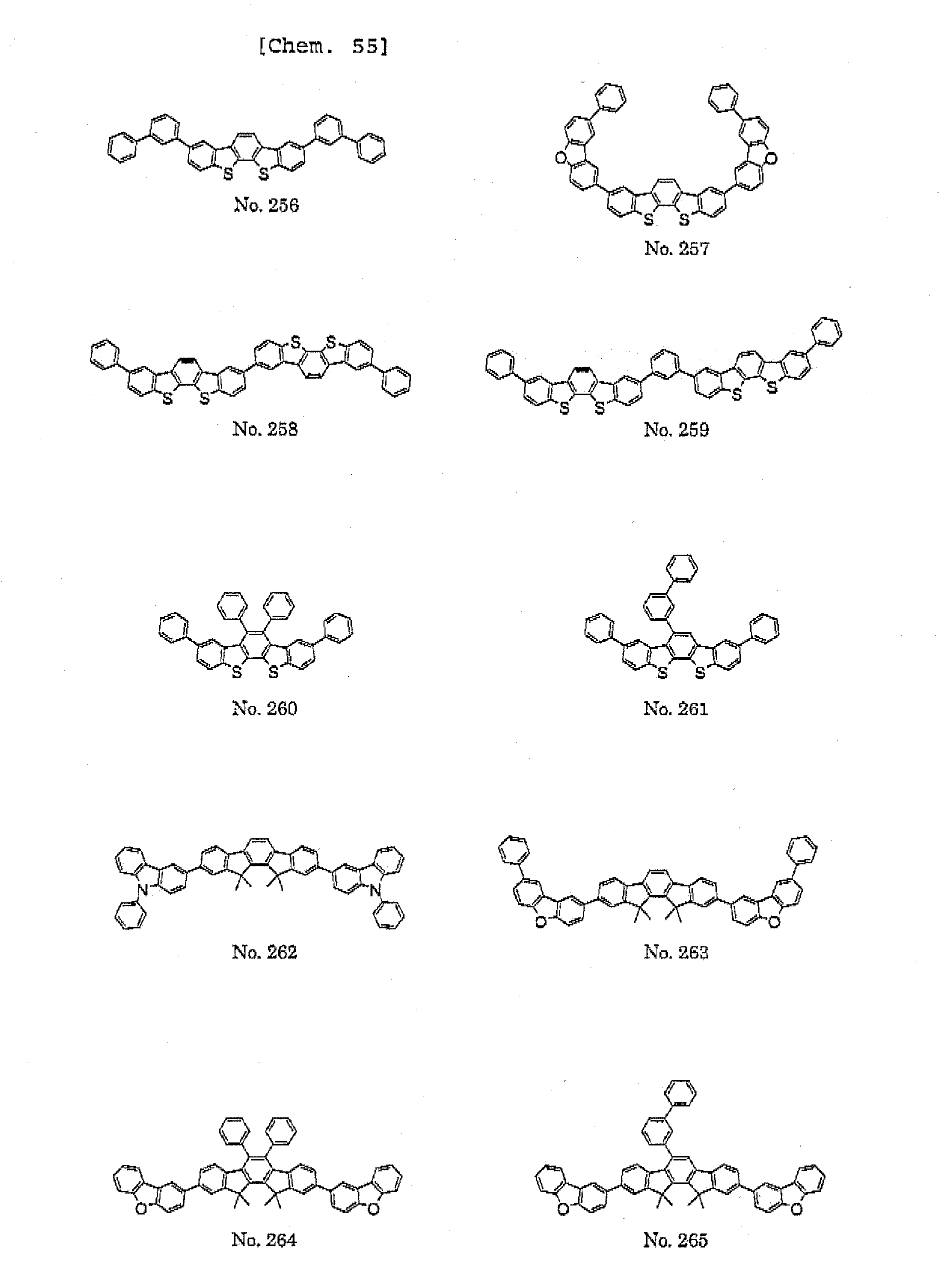

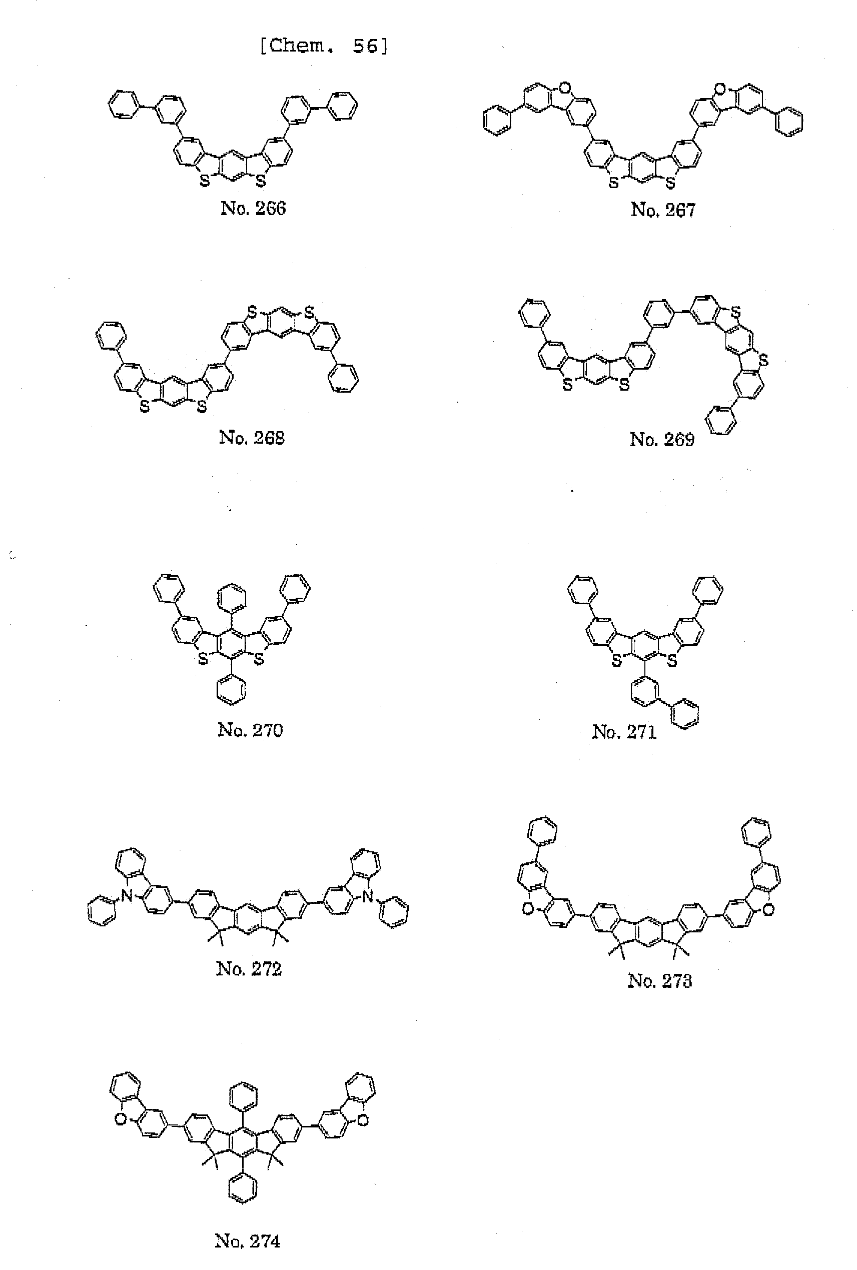

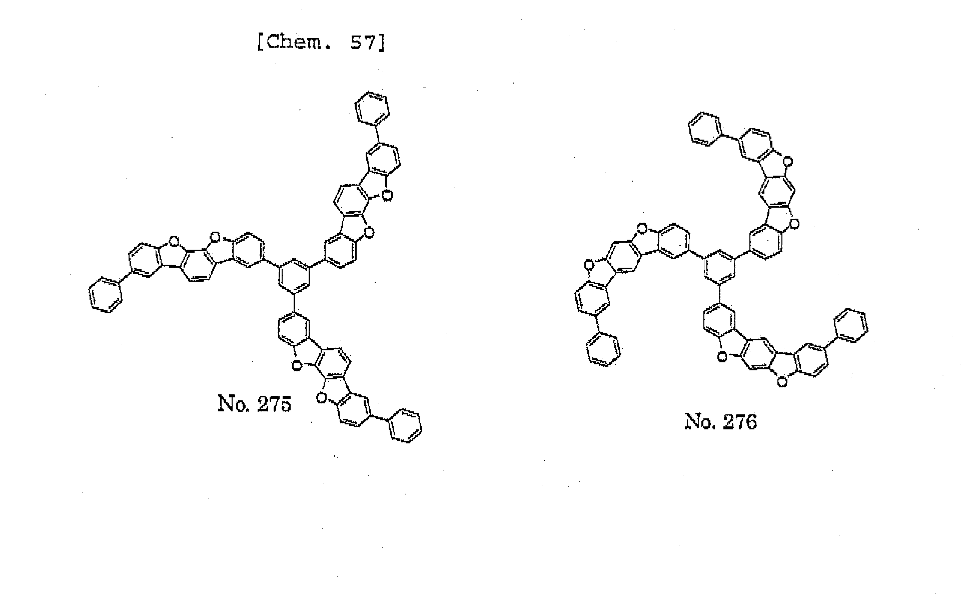

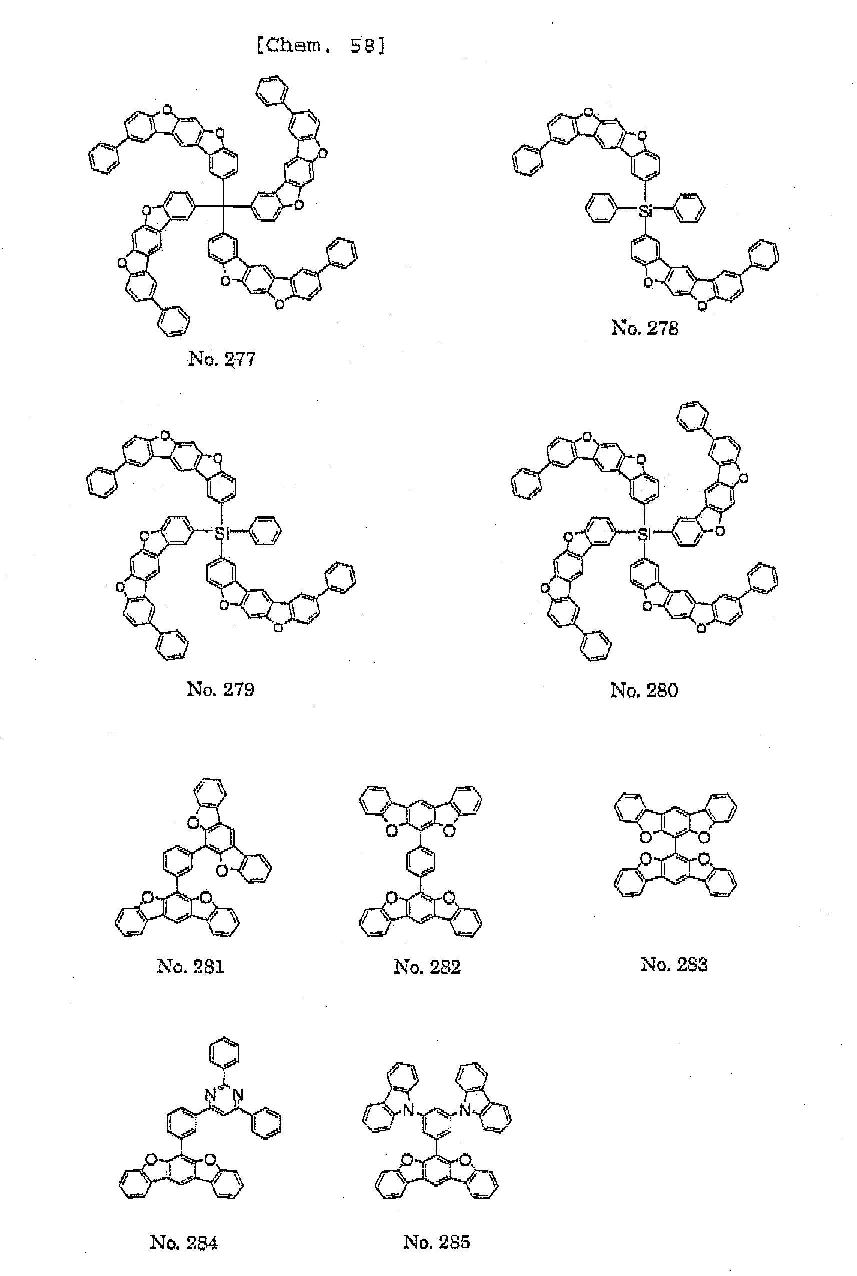

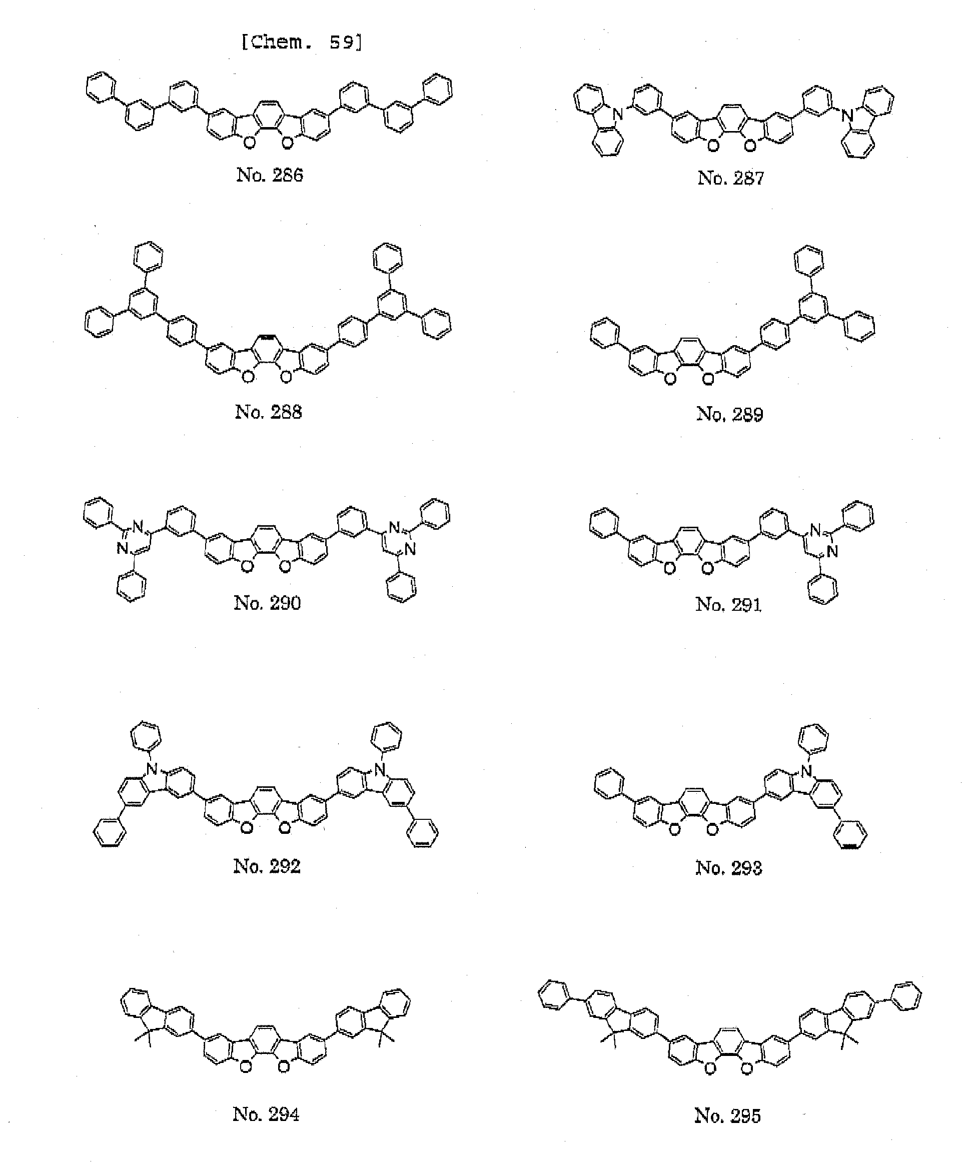

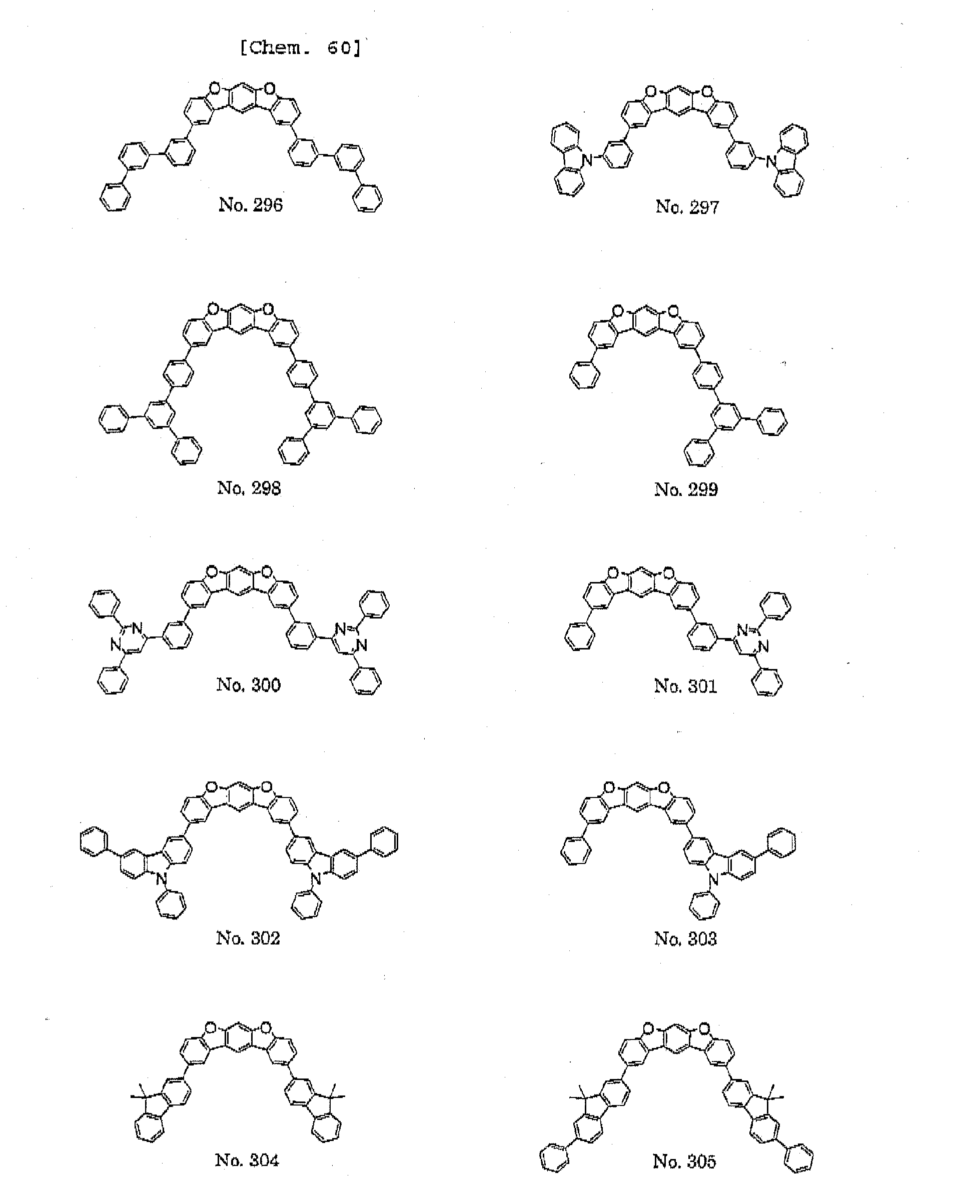

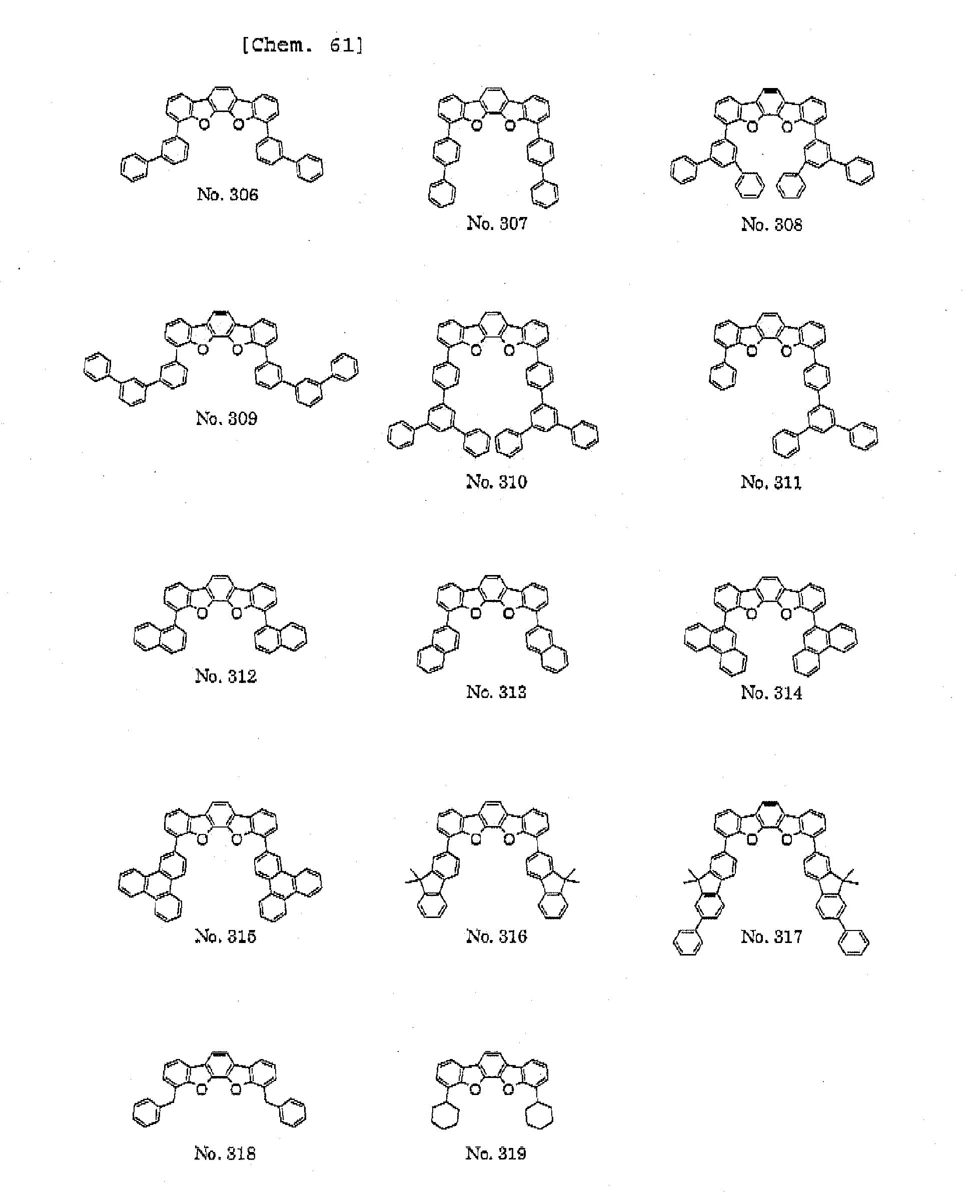

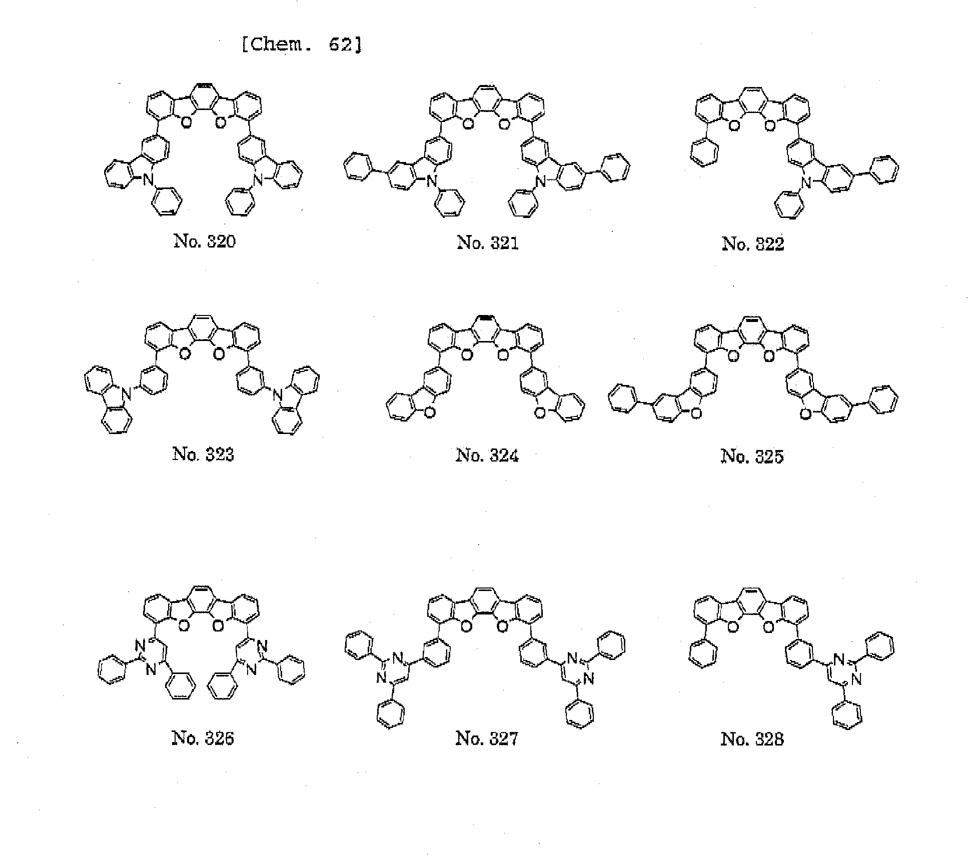

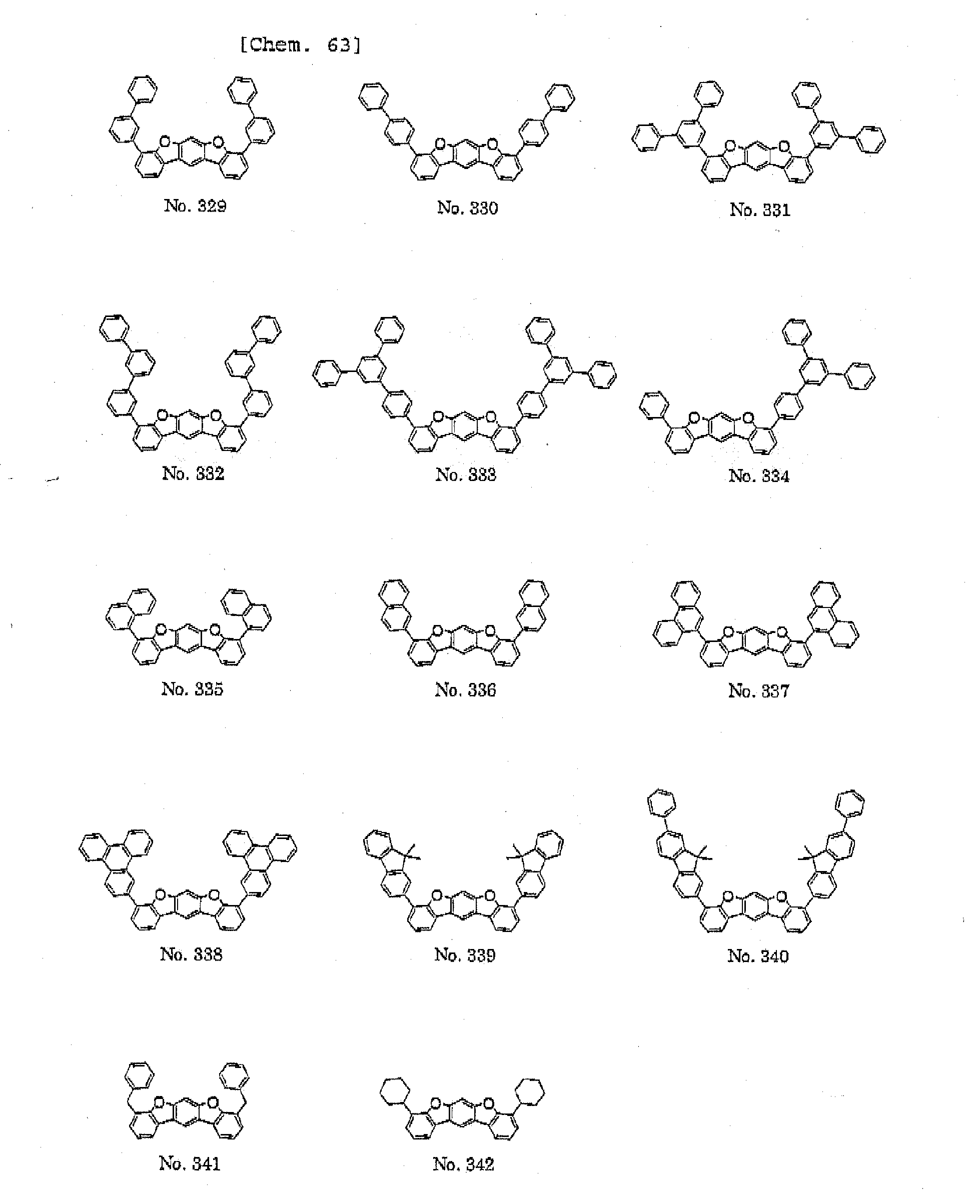

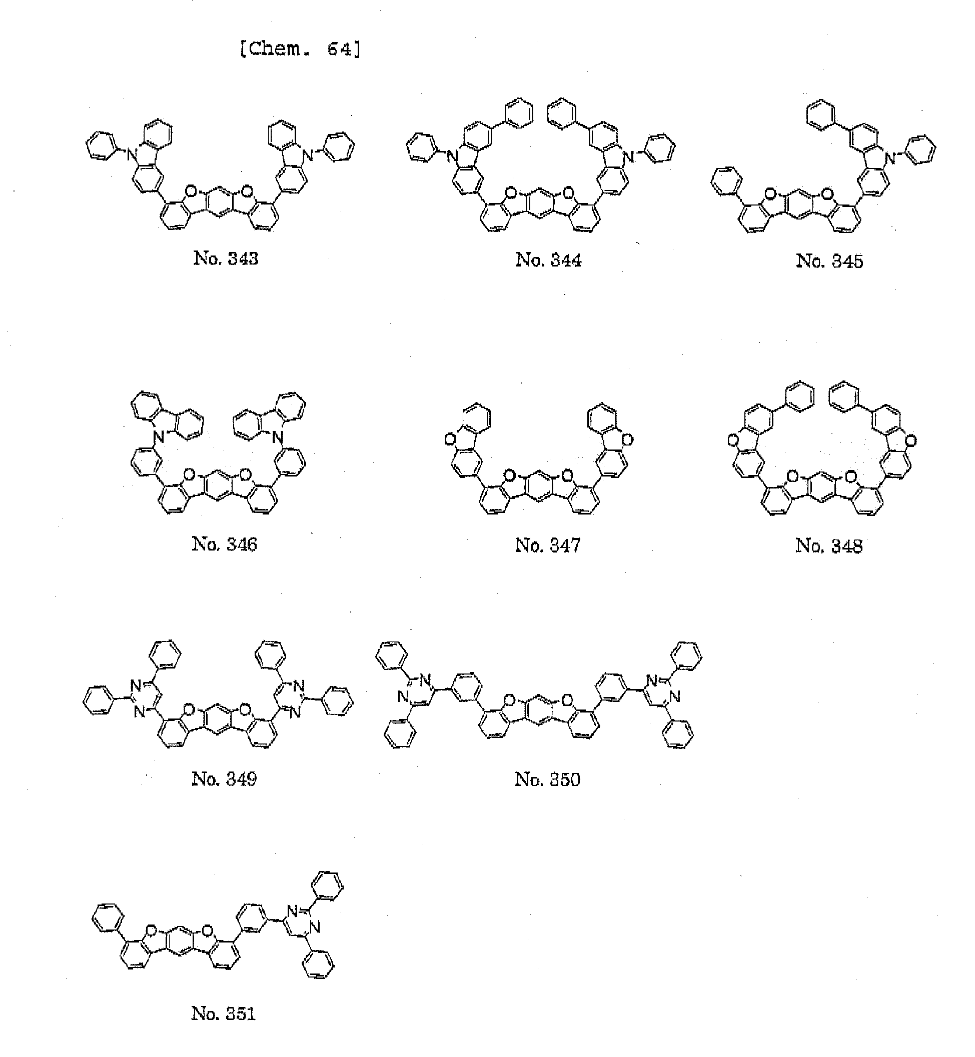

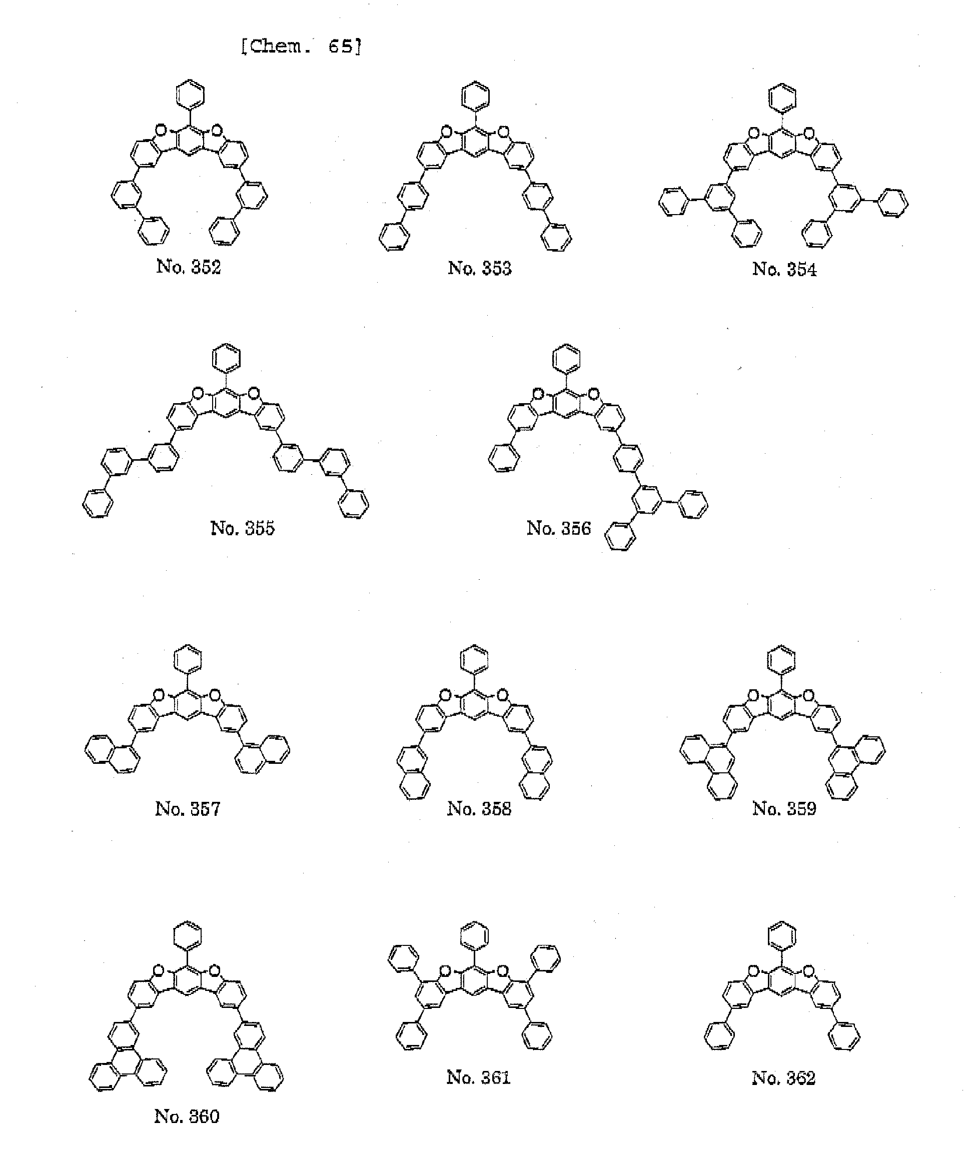

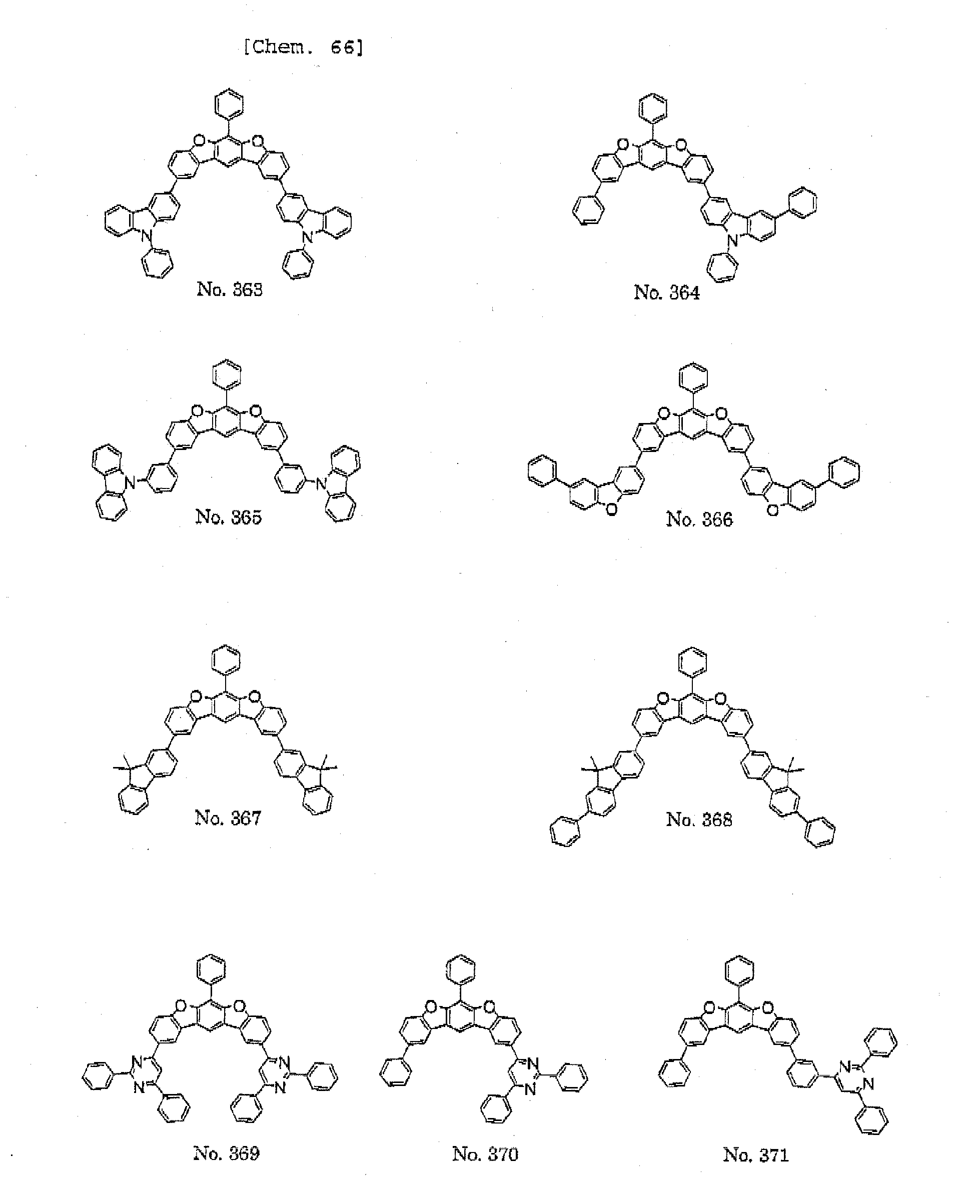

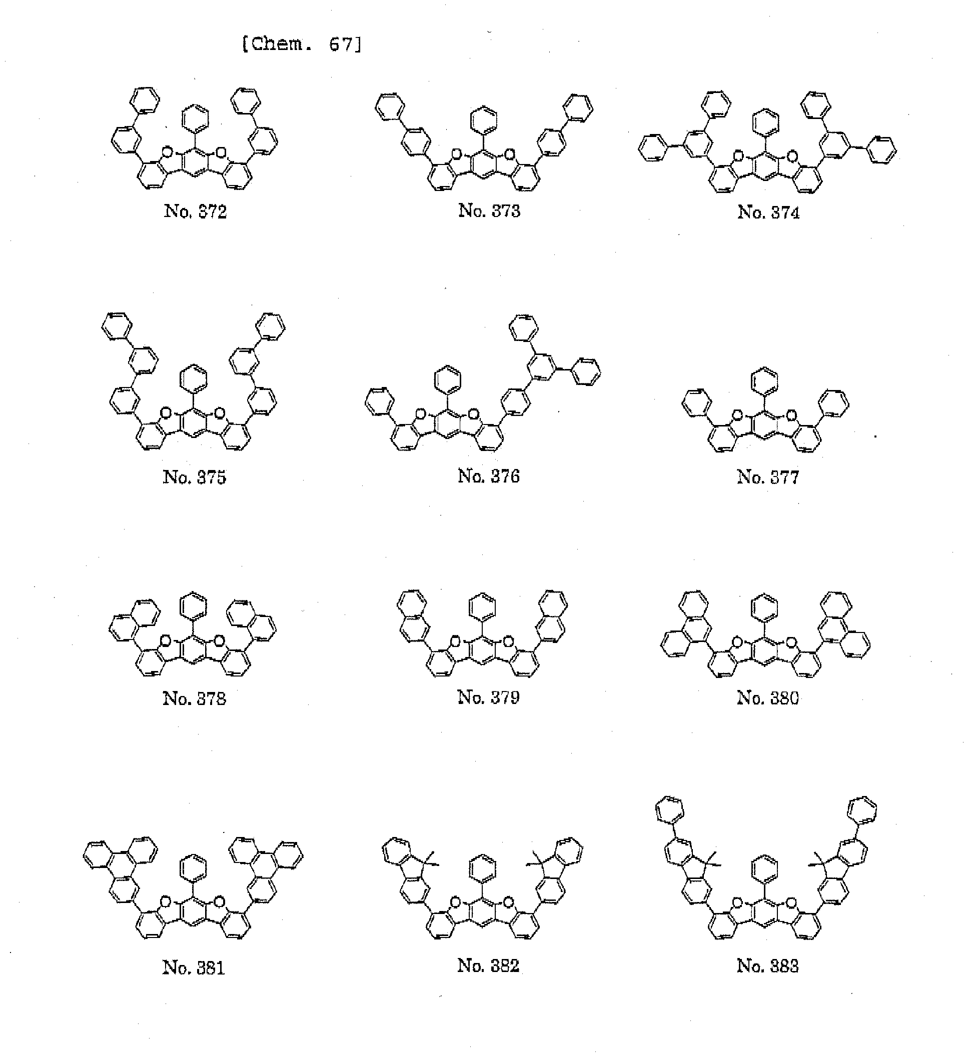

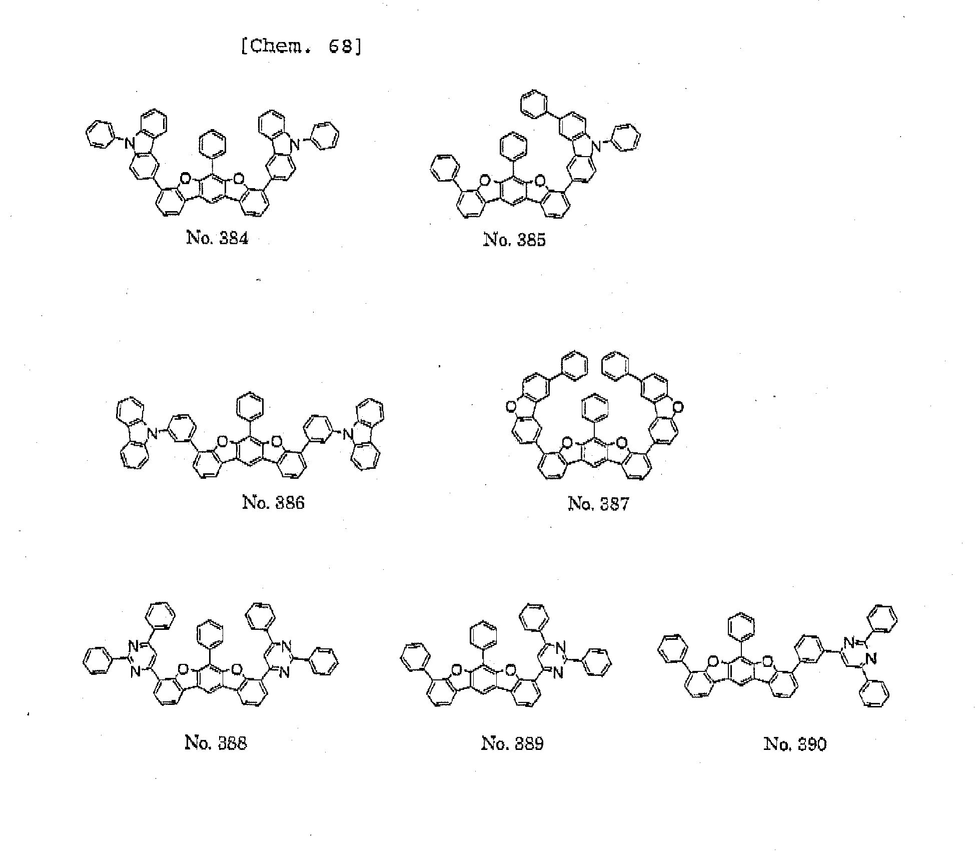

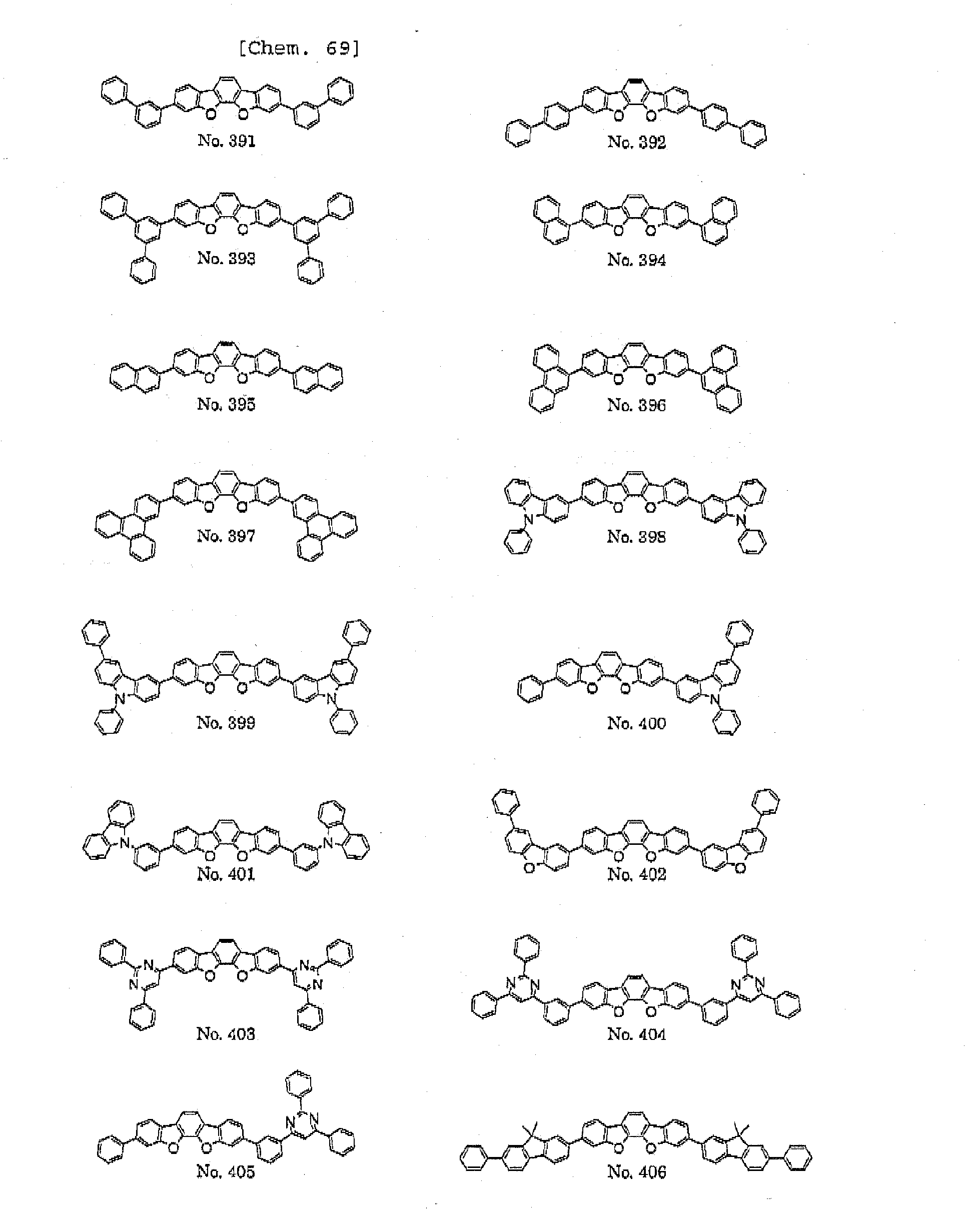

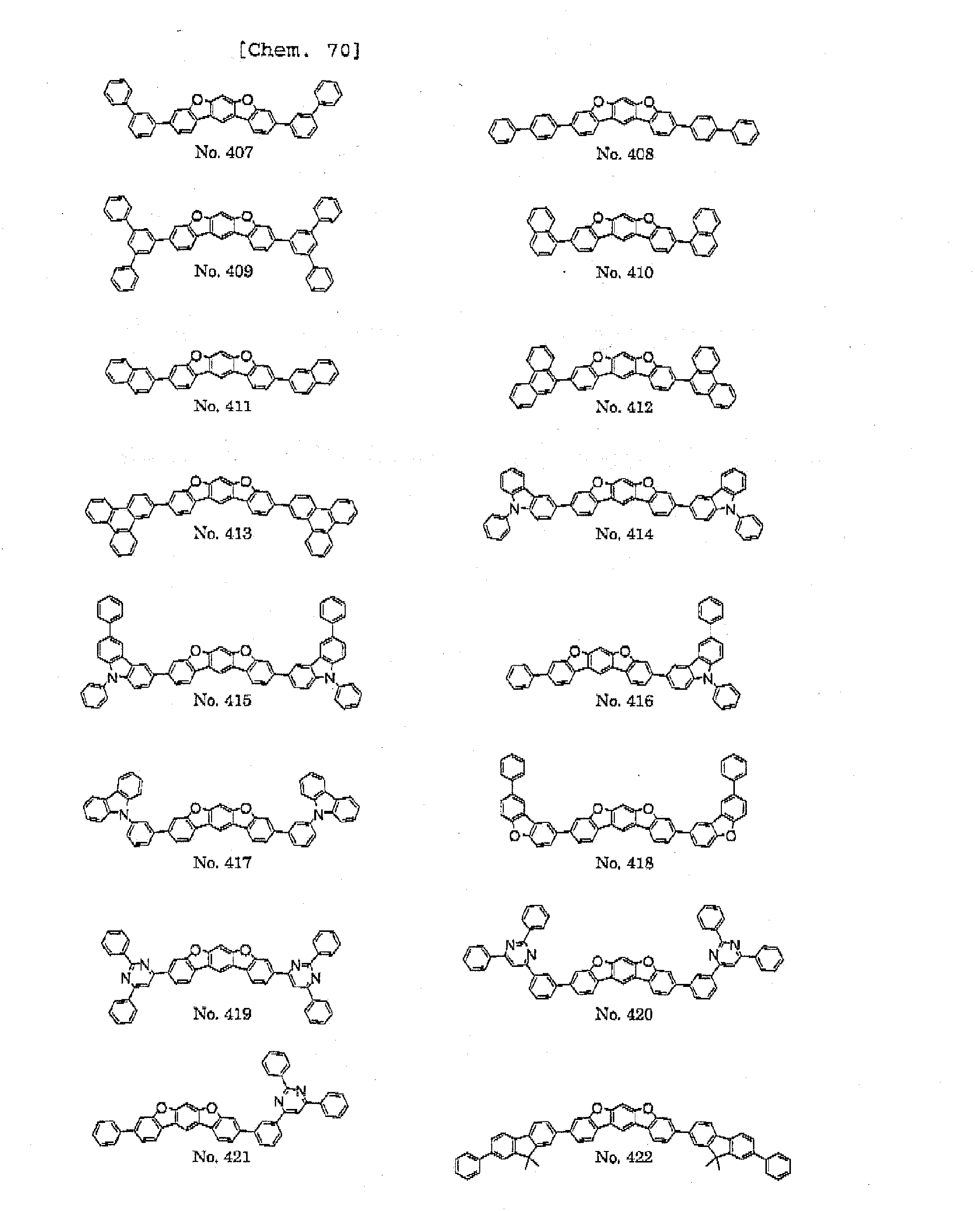

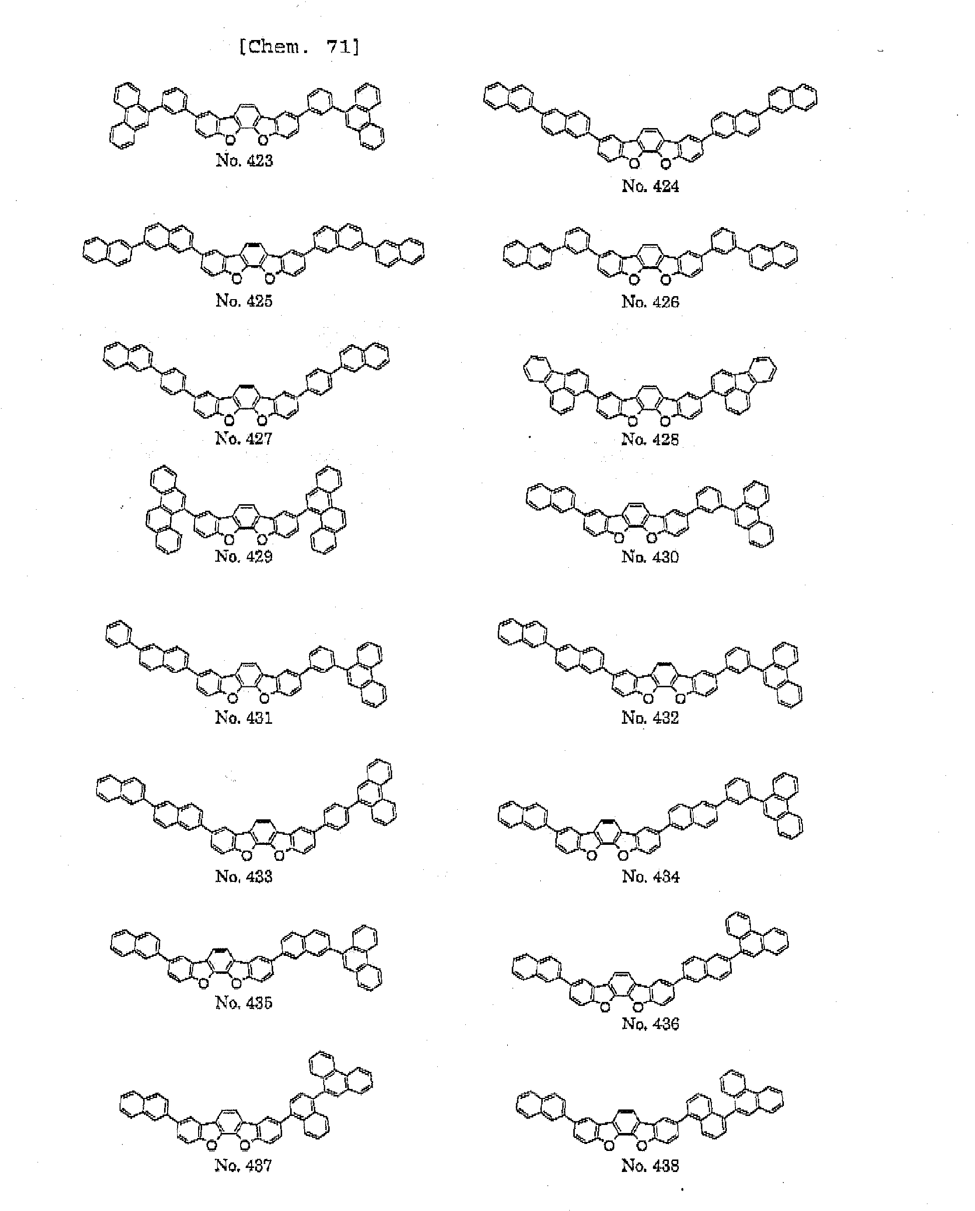

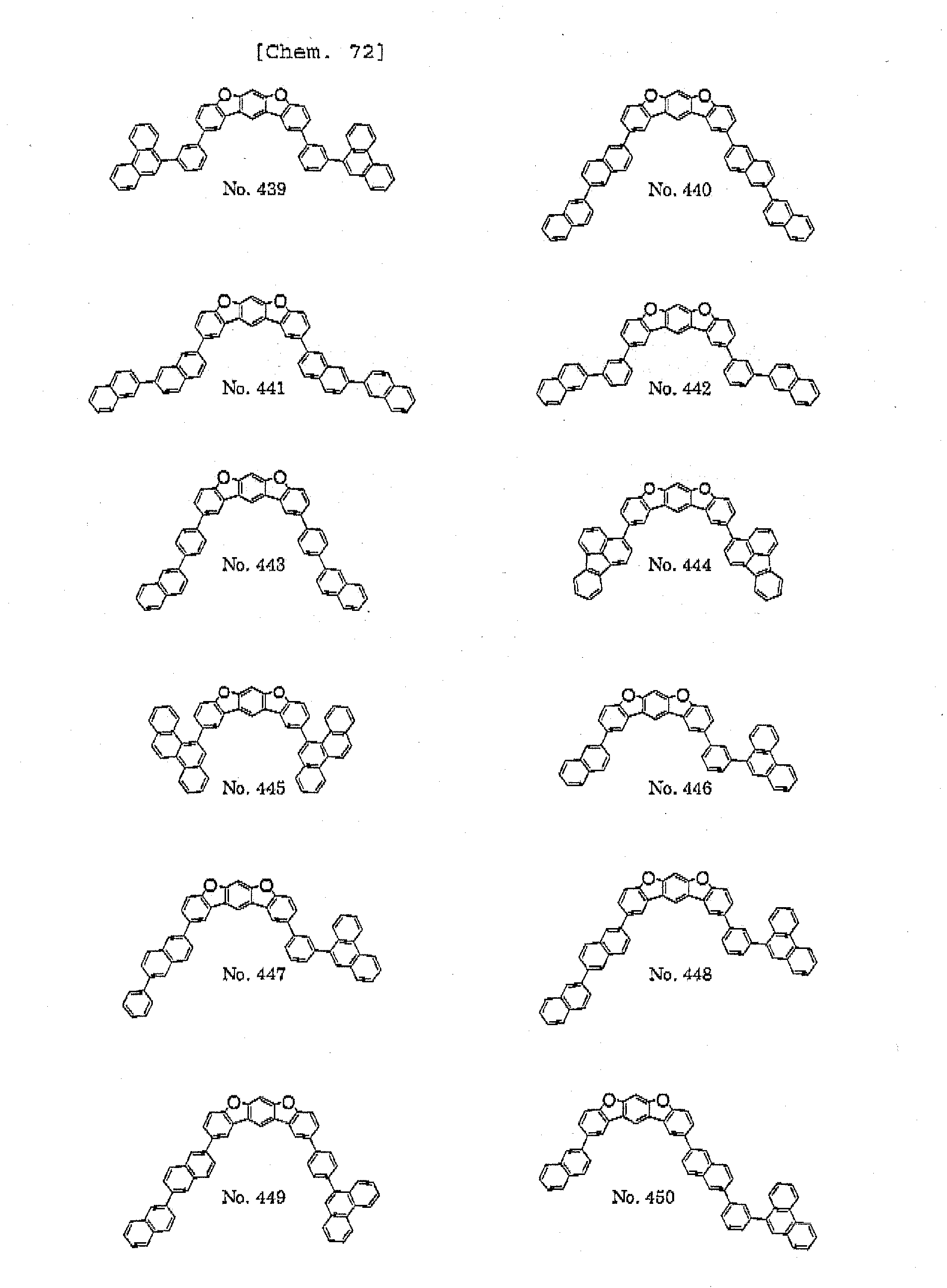

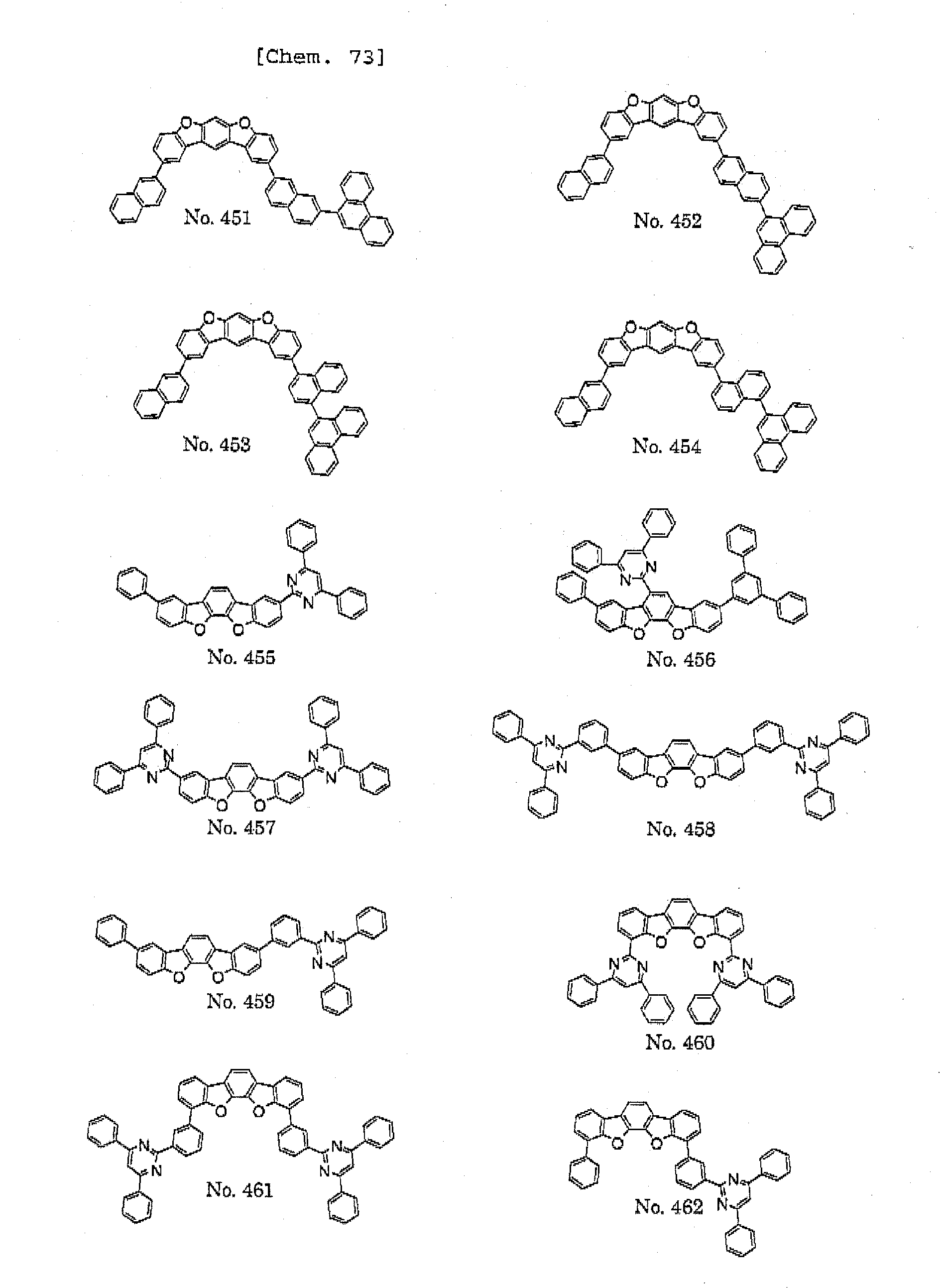

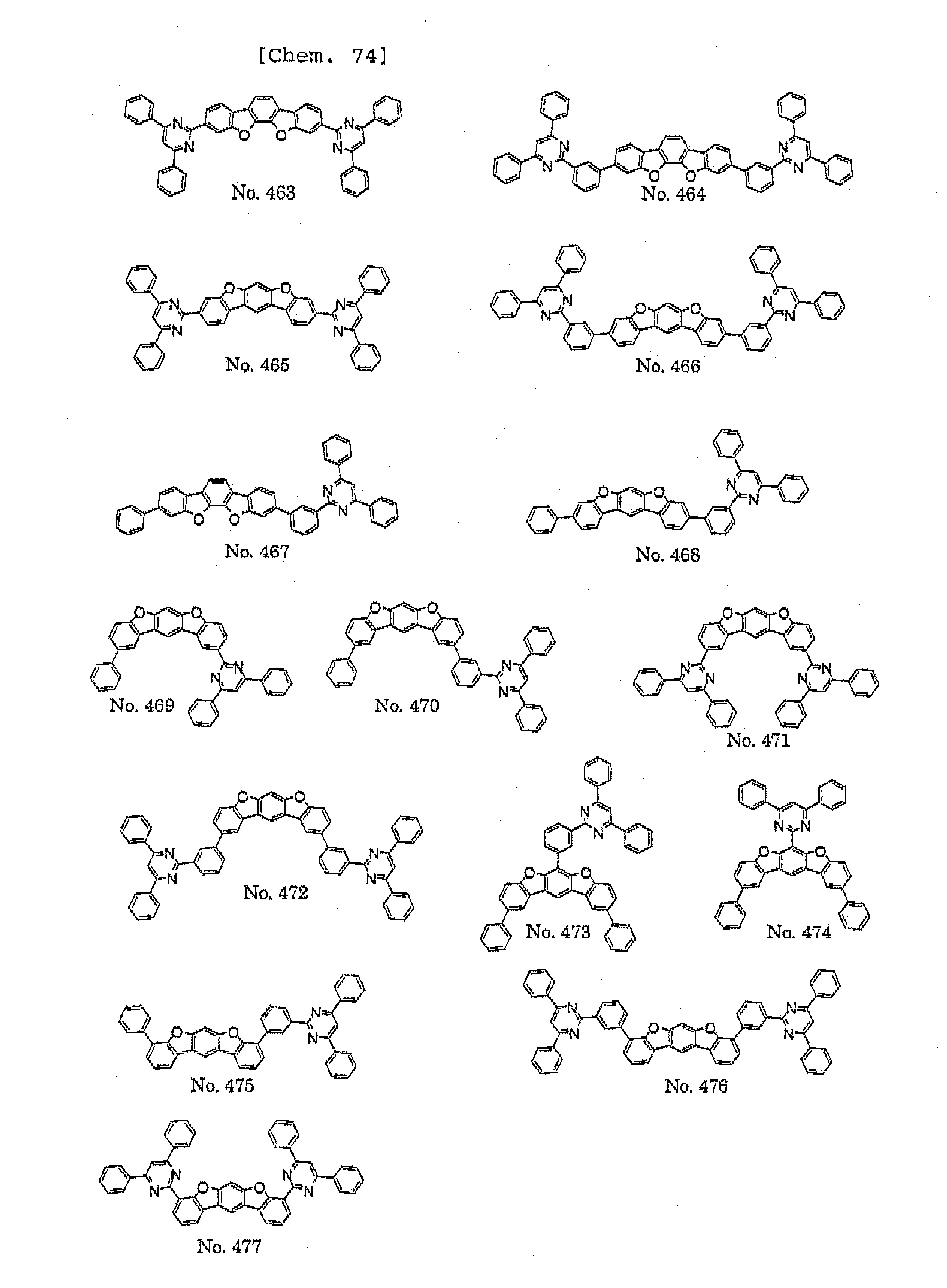

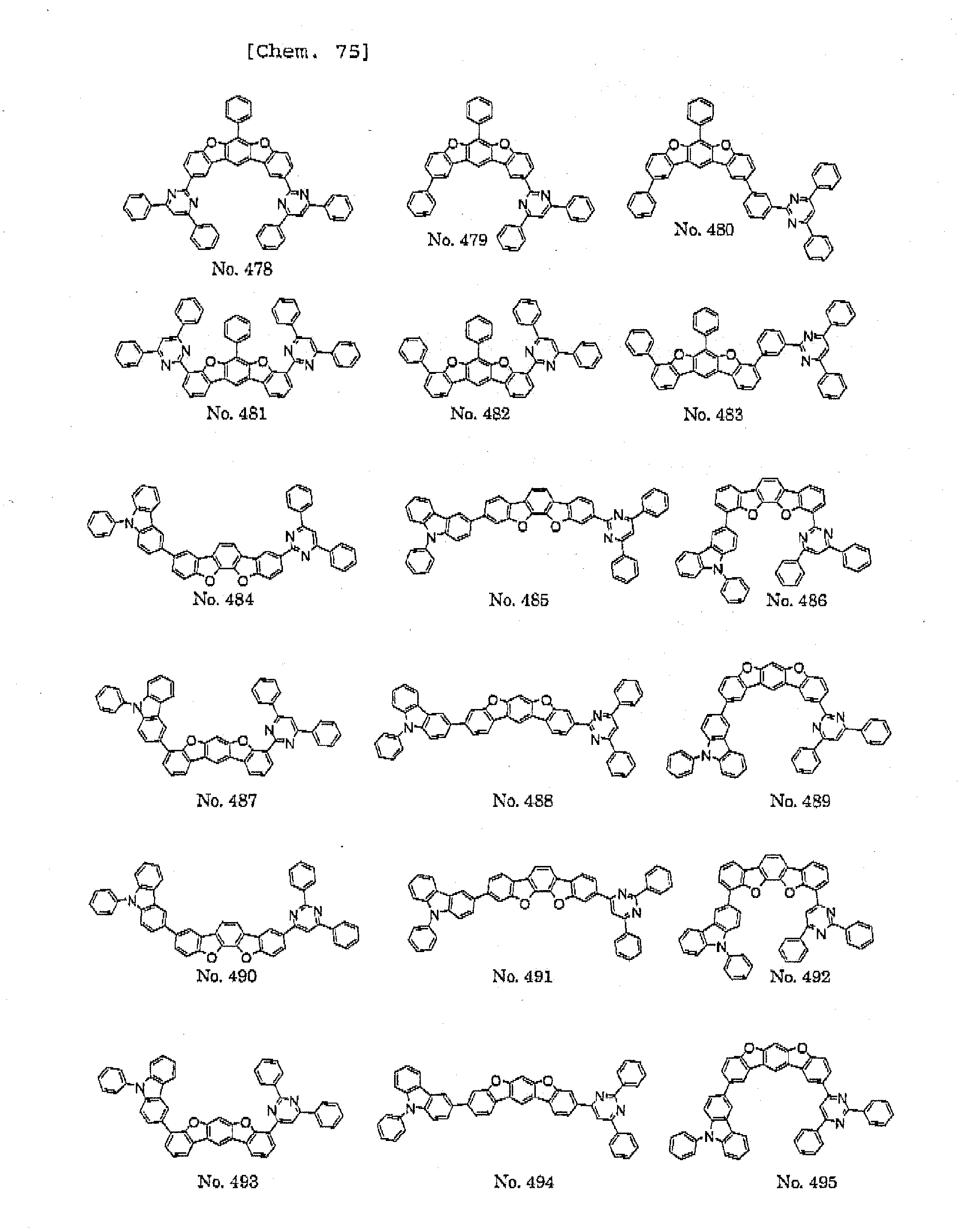

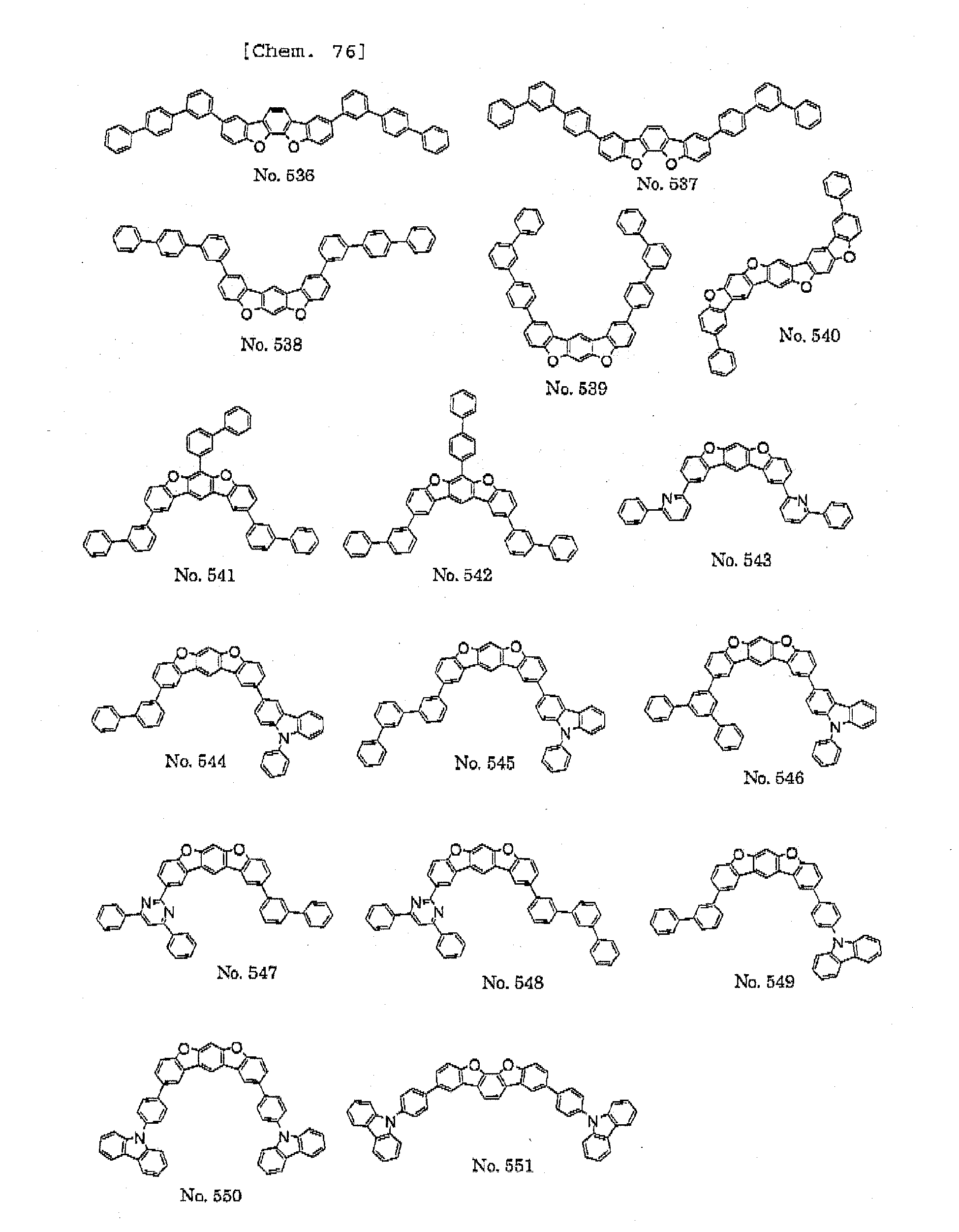

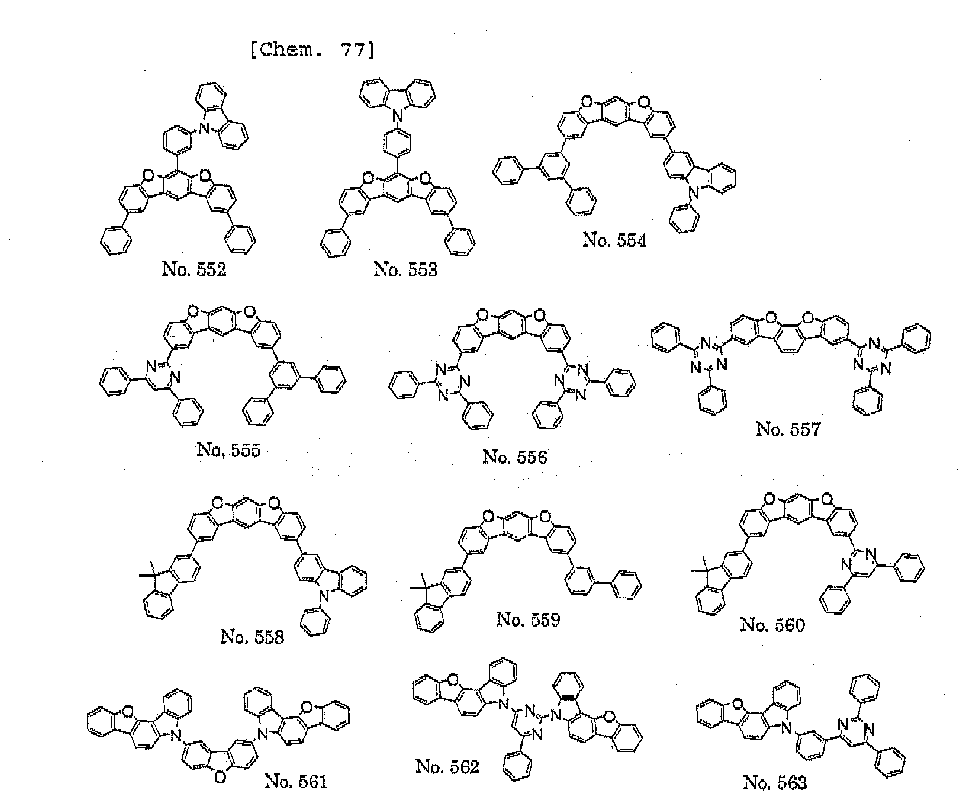

A multi-layer type organic EL device is obtained by laminating a plurality of layers; for example, the device is formed of an anode, a hole transporting layer (a hole injecting layer), a light emitting layer, and a cathode, of an anode, a light emitting layer, an electron transporting layer (an electron injecting layer), and a cathode, of an anode, a hole transporting layer (a hole injecting layer), a light emitting layer, an electron transporting layer (an electron injecting layer), and a cathode, or of an anode, a hole transporting layer (a hole injecting layer), a light emitting layer, a hole barrier layer, an electron transporting layer (an electron injecting layer), and a cathode.

In the organic EL device of the present invention, the light emitting layer preferably contains the polycyclic compound as a host material. In addition, it is preferred that the light emitting layer be formed of a host material and a phosphorescent material, and the host material be the polycyclic compound. In addition, the polycyclic compound may be a host material to be used together with a phosphorescent material, or may be an electron transporting material to be used together with a phosphorescent material. The material has a triplet energy of preferably 2.2 to 3.2 eV, or more preferably 2.5 to 3.2 eV.
The phosphorescent material is preferably a compound containing iridium (Ir), osmium (Os), ruthenium (Ru), or platinum (Pt) because the compound has a high phosphorescent quantum yield, and can additionally improve the external quantum efficiency of the light emitting device. The material is more preferably a metal complex such as an iridium complex, an osmium complex, a ruthenium complex, or a platinum complex. Of those, the iridium complex and the platinum complex are still more preferred, and an orthometalated iridium complex is most preferred. Specific examples of the metal complex such as an iridium complex, an osmium complex, a ruthenium complex, or a platinum complex are shown below.

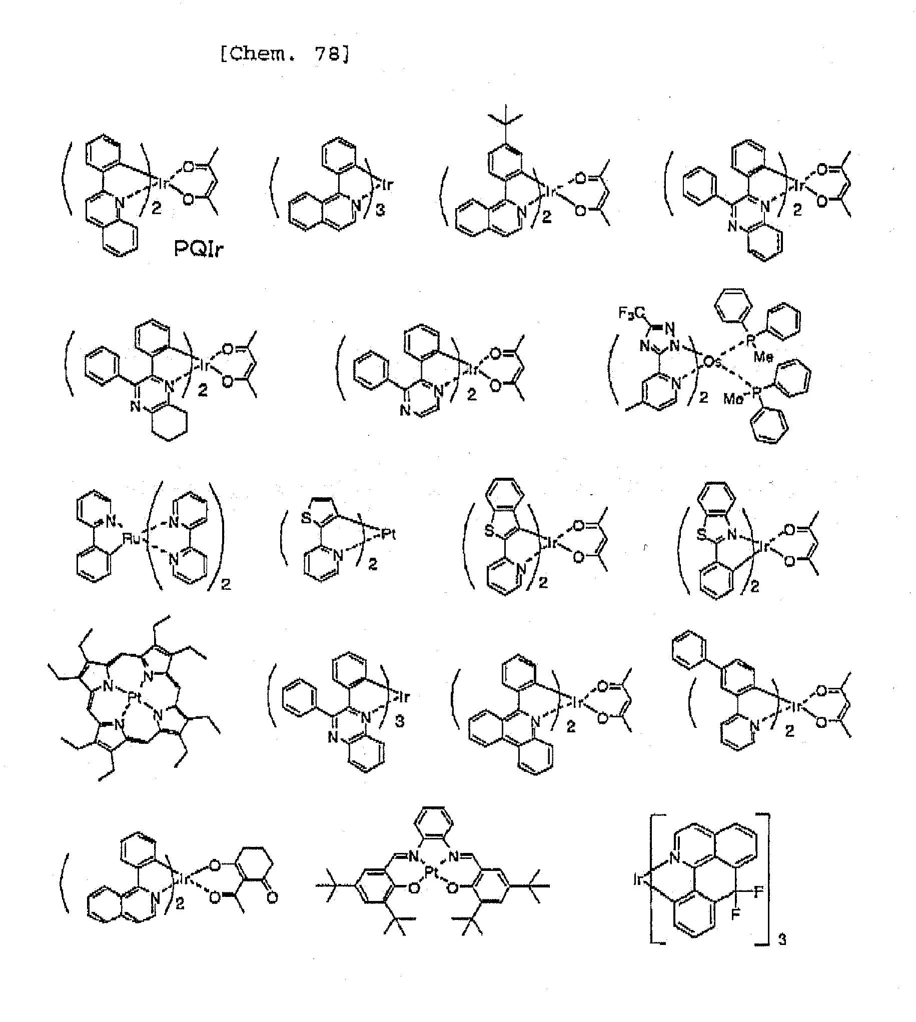

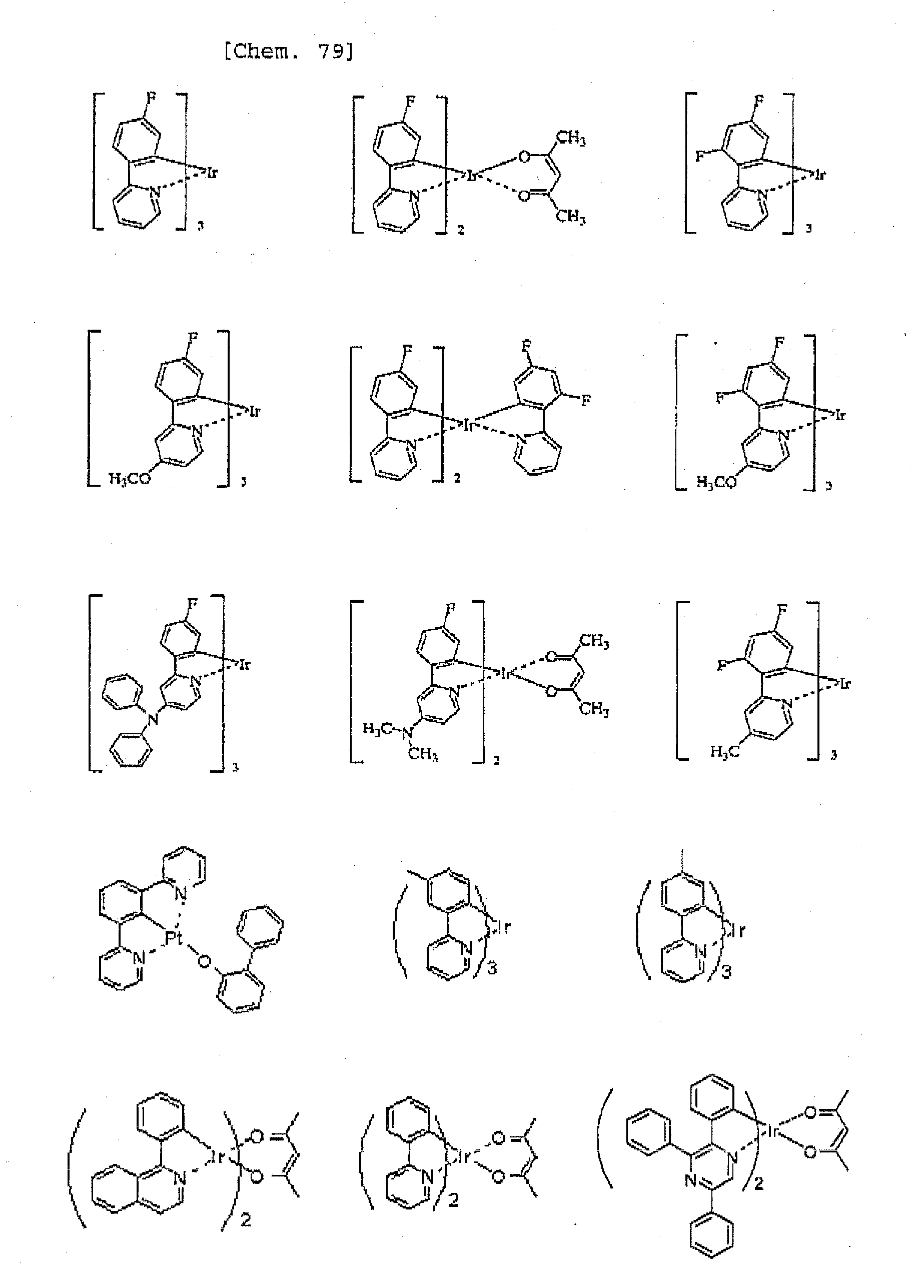

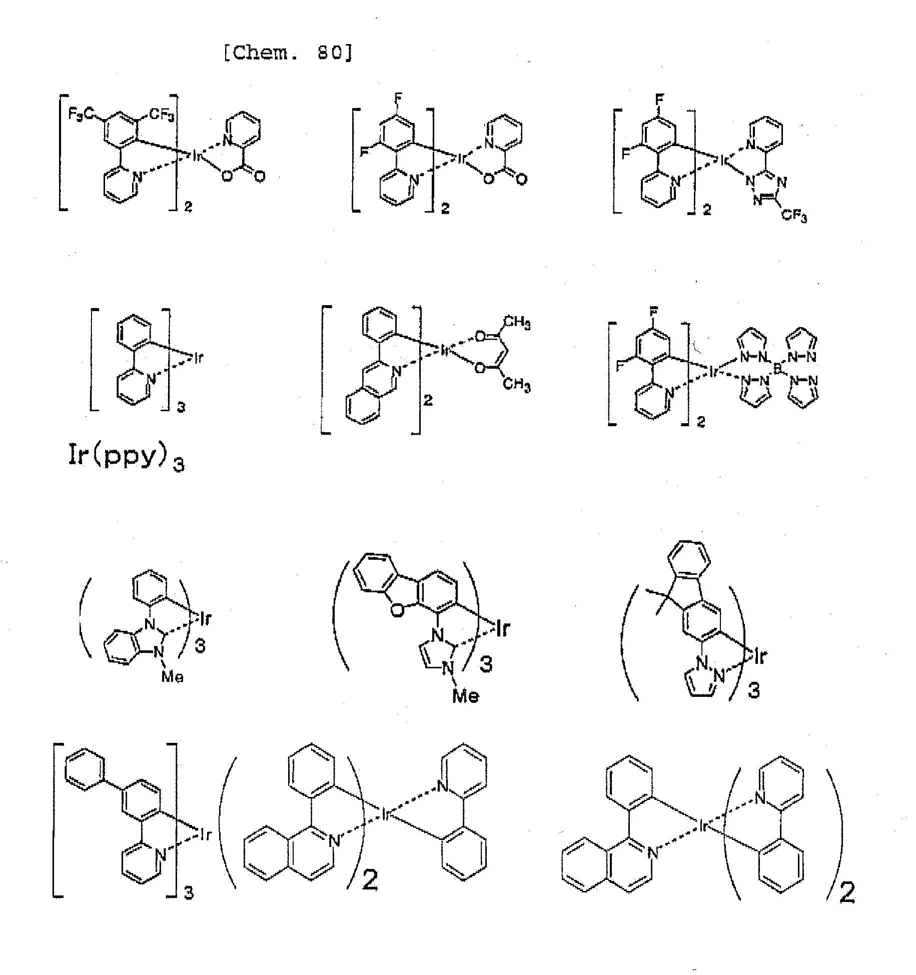

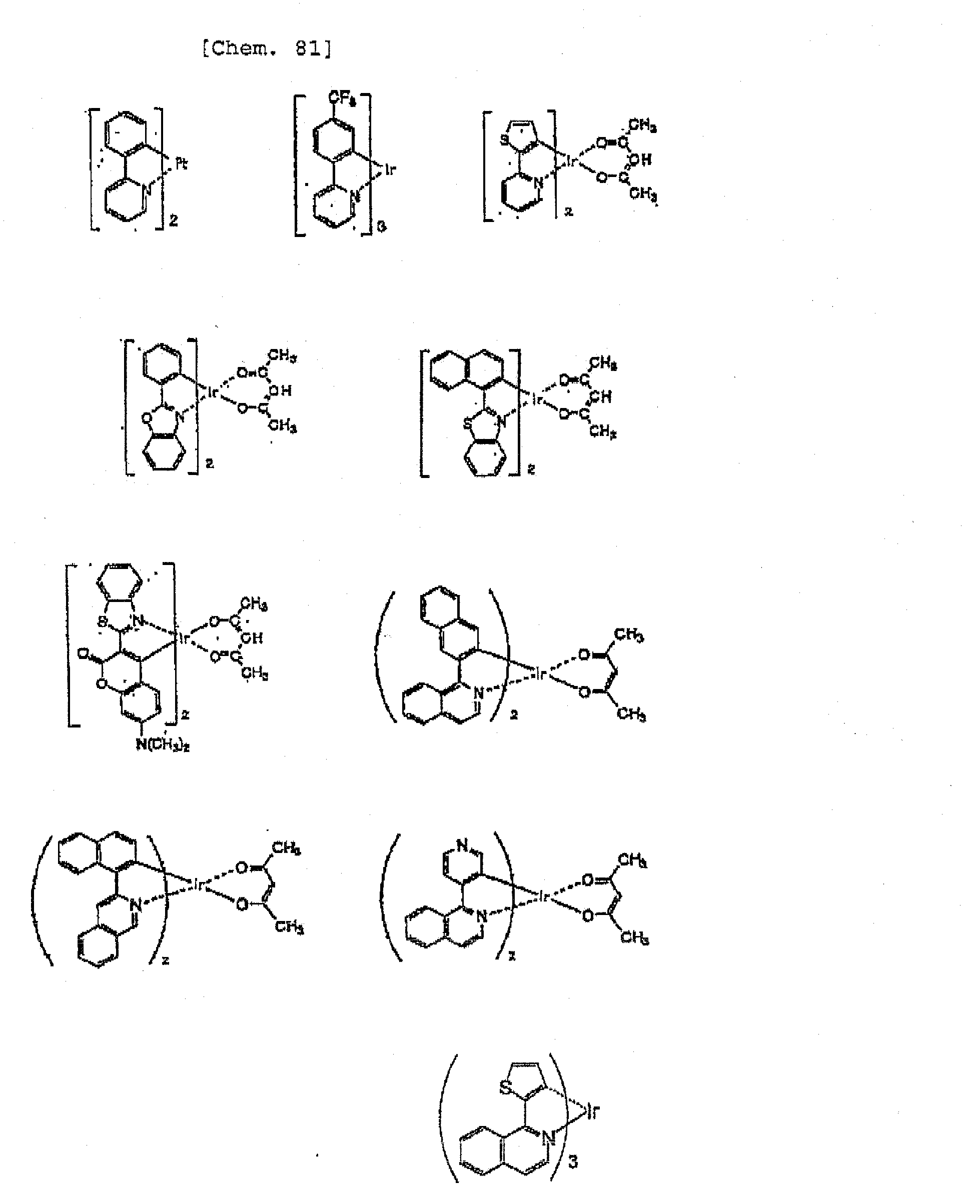

In addition, the organic EL device of the present invention is preferably such that the light emitting layer contains a host material and a phosphorescent material, and contains a metal complex having a local maximum luminous wavelength of 500 nm or less. Further, the material of the present invention can be used together with a fluorescent dopant. The material canbe used together with a blue, green, or red fluorescent dopant. In particular, the material can be more preferably used together with the blue or green fluorescent dopant. Further, the material can be preferably used also as an electron transporting material for a fluorescent organic EL device.

The organic EL device of the present invention preferably has a reductive dopant in an interfacial region between the cathode and an organic thin film layer (for example, an electron injecting layer or a light emitting layer). Examples of the reductive dopant include at least one kind selected from an alkali metal, an alkali metal complex, an alkali metal compound, an alkaline earth metal, an alkaline earth metal complex, an alkaline earth metal compound, a rare earth metal, a rare earth metal complex, and a rare earth metal compound.

Preferred examples of the alkali metal include an alkali metal having a work function of 2.9 eV or less, such as Na having a work function of 2.36 eV, K having a work function of 2.28 eV, Rb having a work function of 2.16 eV, and Cs having a work function of 1.95 eV. Of those, K, Rb, and Cs are more preferred, Rb or Cs is still more preferred, and Cs is most preferred.
Preferred examples of the alkali earth metal include an alkali earth metal having a work function of 2.9 eV or less, such as Ca having a work function of 2.9 eV, Sr having a work function of 2.0 to 2.5 eV, and Ba having a work function of 2.52 eV.
Preferred examples of the rare earth metal include a rare earth metal having a work function of 2.9 eV or less, such as Sc, Y, Ce, Tb, and Yb.
Of those metals, a preferred metal has a particularly high reductive ability, so improvement of light emission intensity and long life of organic EL device can be attained by adding a relatively small amount of the metal to an electron injecting region.

Examples of the alkali metal compound include an alkali oxide such as Li₂O, Cs₂O, or K₂O, and an alkali halide such as LiF, NaF, CsF, or KF. Of those, LiF, Li₂O, and NaF are preferred.
Examples of the alkali earth metal compound include BaO, SrO, CaO, admixtures thereof such as BaₘSr₁₋ₘO (0<m<1) and BaₘCa₁₋ₘO (0<m<1). Of those, BaO, SrO, and CaO are preferred.
Examples of the rare earth metal compound include YbF₃, ScF₃, ScO₃, Y₂O₃, Ce₂O₃, GdF₃, and TbF₃. Of those, YbF₃, ScF₃, and TbF₃ are preferred.

The alkali metal complex, alkali earth metal complex, and rare earth metal complex are not particularly limited as long as they each include as a metal ion at least one of alkali metal ions, alkali earth metal ions, and rare earth metal ions. Mean while, preferred examples of a ligand include, but not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzoimidazole, hydroxybenzotriazole, hydroxyfluborane, bipyridyl, phenanthroline, phthalocyanine, porphyrin, cyclopentadiene, β-diketones, azomethines, and derivatives thereof.

For the addition form of the reductive dopant, it is preferred that the reductive dopant be formed in a shape of a layer or an island in the interfacial region. A preferred example of the forming method includes a method in which an organic substance which is a light emitting material or an electron injecting material for forming the interfacial region is deposited at the same time as the reductive dopant is deposited by a resistant heating deposition method, thereby dispersing the reductive dopant in the organic substance. The disperse concentration by molar ratio of the organic substance to the reductive dopant is 100 :1 to 1:100 , and is preferably 5:1 to 1:5.
In a case where the reductive dopant is formed into the shape of a layer, the light emitting material or electron injecting material which serves as an organic layer in the interface is formed into the shape of a layer. After that, the reductive dopant is solely deposited by the resistant heating deposition method to form a layer preferably having a thickness of 0.1 to 15 nm.
In a case where the reductive dopant is formed into the shape of an island, the light emitting material or electron injecting material which serves as an organic layer in the interface is formed into the shape of an island. After that, the reductive dopant is solely deposited by the resistant heating deposition method to form an island preferably having a thickness of 0.05 to 1 nm.

When the organic EL device of the present invention has an electron injecting layer between the light emitting layer and the cathode, an electron transporting material to be used in the electron injecting layer is preferably an aromatic heterocyclic compound containing one or more hetero atoms in any one of its molecules, or particularly preferably a nitrogen-containing ring derivative.

The nitrogen-containing ring derivative is preferably, for example, a nitrogen-containing ring metal chelate complex represented by the following formula (A).

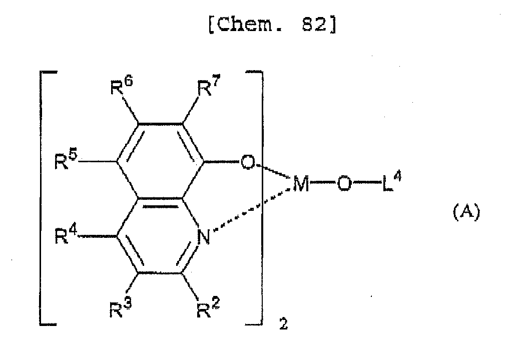

R² to R⁷ each independently represent a hydrogen atom, a halogen atom, an amino group, a hydrocarbon group having 1 to 40 carbon atoms, an alkoxy group, an aryloxy group, an alkoxycarbonyl group, or a heterocyclic group, each of which may be substituted.

M represents aluminum (Al), gallium (Ga), or indium (In). Indium is preferred.
L⁴ in the formula (A) is a group represented by the following formula (A') or (A").

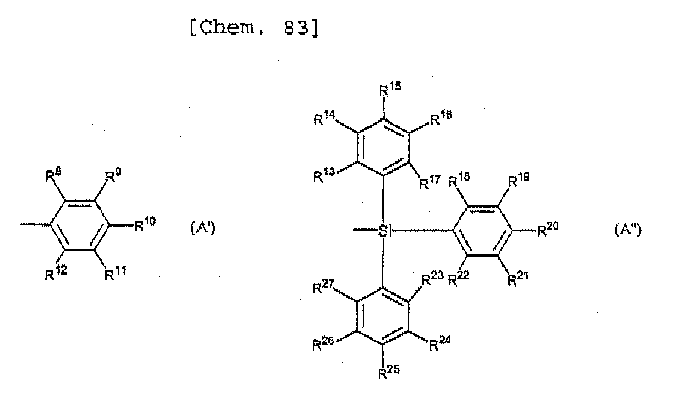

(In the formula, R¹ to R¹² each independently represent a hydrogen atom, or a substituted or unsubstituted hydrocarbon group having 1 to 40 carbon atoms, and adjacent groups may form a cyclic structure. In addition, R¹³ to R²⁷ each independently represent a hydrogen atom, or a substituted or unsubstituted hydrocarbon group having 1 to 40 carbon atoms, and adjacent groups may form a cyclic structure.)

A nitrogen-containing heterocyclic derivative is a nitrogen-containing heterocyclic derivative formed of an organic compound having any one of the following formulae, and a nitrogen-containing compound which is not a metal complex is also an example of the derivative. Examples of the derivative include a five- or six-membered ring containing a skeleton represented by the following formula (a) and a derivative of a structure represented by the following formula (b).

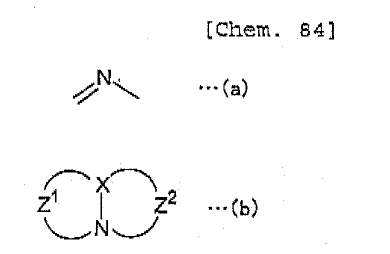

(In the formula (b), X represents a carbon atom or a nitrogen atom, and Z¹ and Z² each independently represent an atomic group capable of forming a nitrogen-containing heterocycle.)

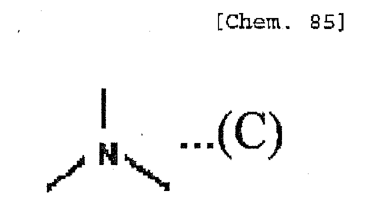

An organic compound having a nitrogen-containing' aromatic polycycle formed of a five- or six-memberedring is preferred. In the case of such nitrogen-containing aromatic polycycle having a plurality of nitrogen atoms, a nitrogen-containing aromatic polycyclic organic compound having a skeleton obtained by combining the above-mentioned formulae (a) and (b) or the abode-mentioned formulae (a) and (c) is more preferred.

The nitrogen-containing- group of the nitrogen-containing organic compound is selected from, for example, nitrogen-containing heterocyclic groups represented by the following formulae.

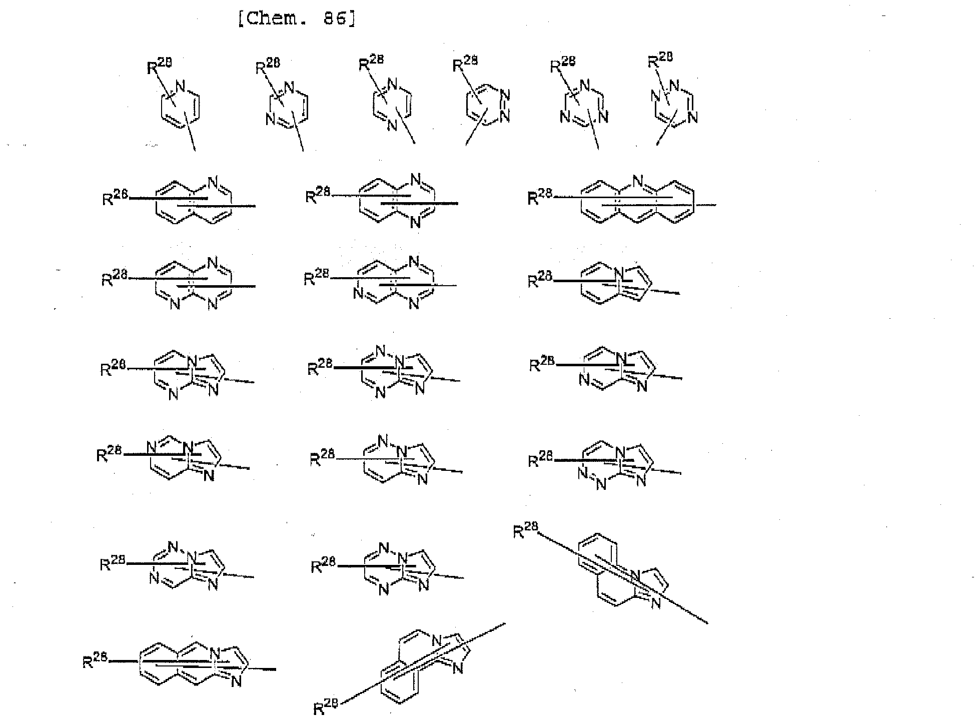

(In each of the formulae, R^{2B} represents an aryl group having 6 to 40 carbon atoms, a heteroaryl group having 3 to 40 carbon atoms, an alkyl group having 1 to 20 carbon atoms, or an alkoxy group having 1 to 20 carbon atoms, and when the number of R^{2B} is two or more, a plurality of R²⁸ₛ may be identical to or different from each other.)

Further, a preferred specific compound is, for example, a nitrogen-containing heterocyclic derivative represented by the following formula.

HAr^{a}-L⁶-Ar^{b}-Ar^{c} [Chem. 87]

(In the formula, HAr^{a} represents a nitrogen-containing heterocycle which has 3 to 40 carbon atoms and which may have a substituent, L⁶ represents a single bond, an arylene group which has 6 to 40 carbon atoms and which may have a substituent, or a heteroarylene group which has 3 to 40 carbon atoms and which may have a substituent, Ar^{b} represents a divalent aromatic hydrocarbon group which has 6 to 40 carbon atoms and which may have a substituent, and Ar^{c} represents an aryl group which has 6 to 40 carbon atoms and which may have a substituent, or a heteroaryl group which has 3 to 40 carbon atoms and which may have a substituent.)

HAr^{a} is selected from, for example, the following group.

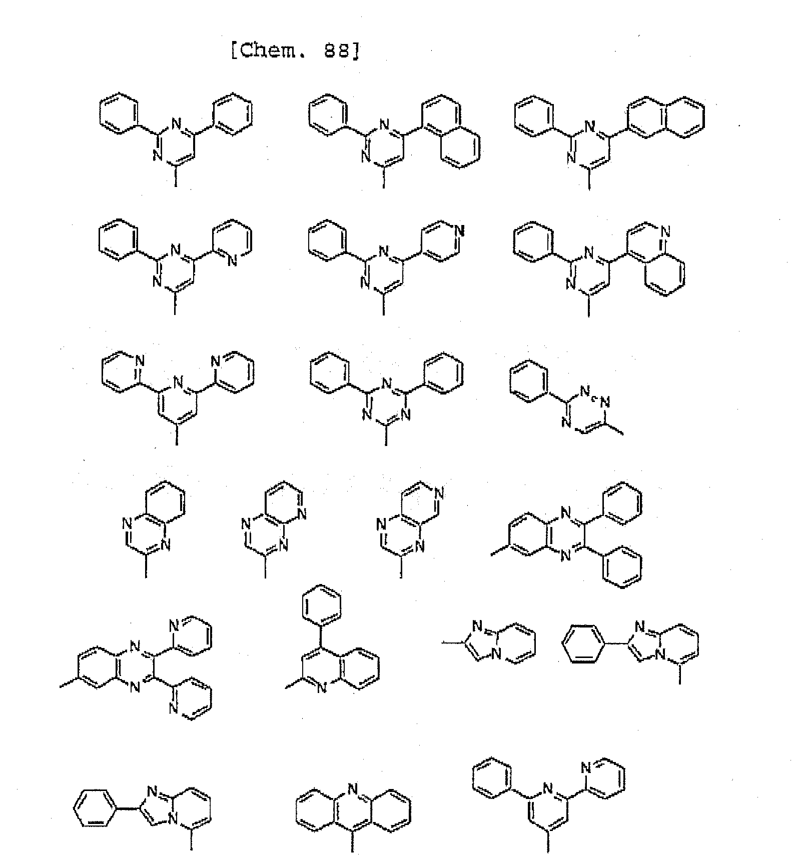

L⁶ is selected from, for example, the following group.

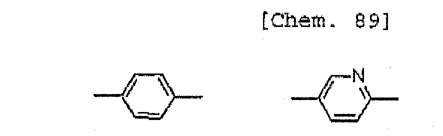

Ar^{c} is selected from, for example, the following groups.

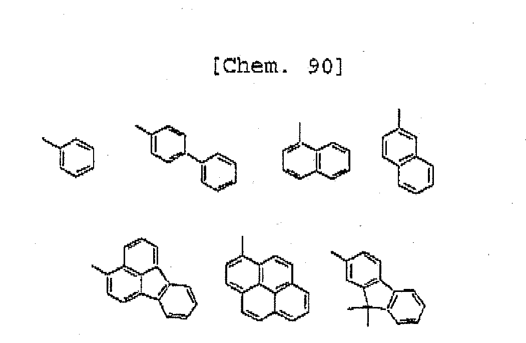

Ar^{b} is selected from, for example, the following arylanthranil groups.

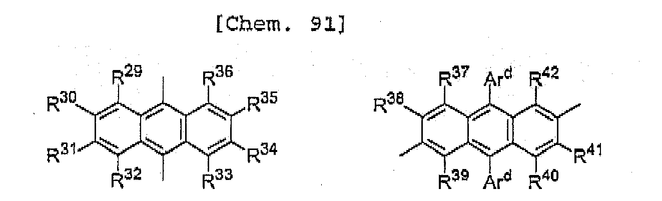

(In the formula, R²⁹ to R⁴² each independently represent a hydrogen atom, a halogen atom, an alkyl group having 1 to 20 carbon atoms, an alkoxy group having 1 to 20 carbon atoms, an aryloxy group having 6 to 40 carbon atoms, an aryl group which has 6 to 40 carbon atoms and which may have a substituent, or a heteroaryl group having 3 to 40 carbon atoms, and Ar^{d} represents an aryl group which has 6 to 40 carbon atoms and which may have a substituent, or a heteroaryl group having 3 to 40 carbon atoms.)
In addition, a nitrogen-containing heterocyclic derivative in which R²⁹ to R³⁶ in Ar^{b} represented by the above-mentioned formula each represent a hydrogen atom is preferred.

In addition to the foregoing, the following compound (see JP 09-3448 A) is also suitably used.

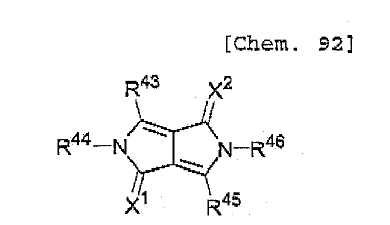

(In the formulae, R⁴³ to R⁴⁶ each independently represent a hydrogen atom, a substituted or unsubstituted aliphatic group, a substituted or unsubstituted alicyclic group, a substituted or unsubstituted carbocyclic aromatic ring group, or a substituted or unsubstituted heterocyclic group, and X¹ and X² each independently represent an oxygen atom, a sulfur atom, or a dicyanomethylene group.)

in addition, the following compound (see JP 2000-173774 A) is also suitably used.

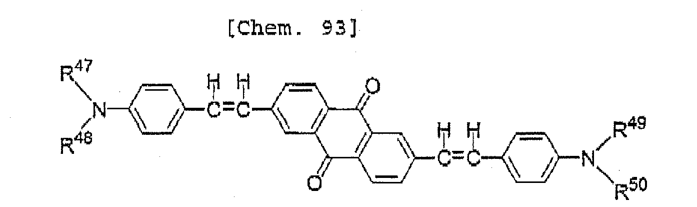

In the formula, R⁴⁷, R⁴⁸ R⁴⁵, and R⁵⁰ represent groups identical to or different from one another, and each represent an aryl group represented by the following formula.

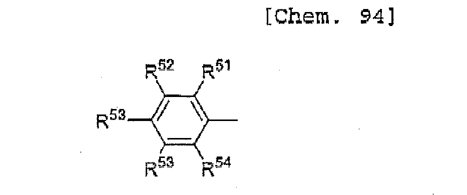

(In the formula, R⁵¹, R⁵², R⁵³, R⁵⁴, and R⁵⁵ represent groups identical to or different from one another, and each represent a hydrogen atom, or at least one of them may represent a saturated or unsaturated alkoxyl, alkyl, amino, or alkylamino group.)

Further, a polymer compound containing the nitrogen-containing heterocyclic group or nitrogen-containing heterocyclic derivative is also permitted.

In addition, the electron transporting layer preferably contains at least one of the nitrogen-containing heterocyclic derivatives represented by the following formulae (201) to (203).

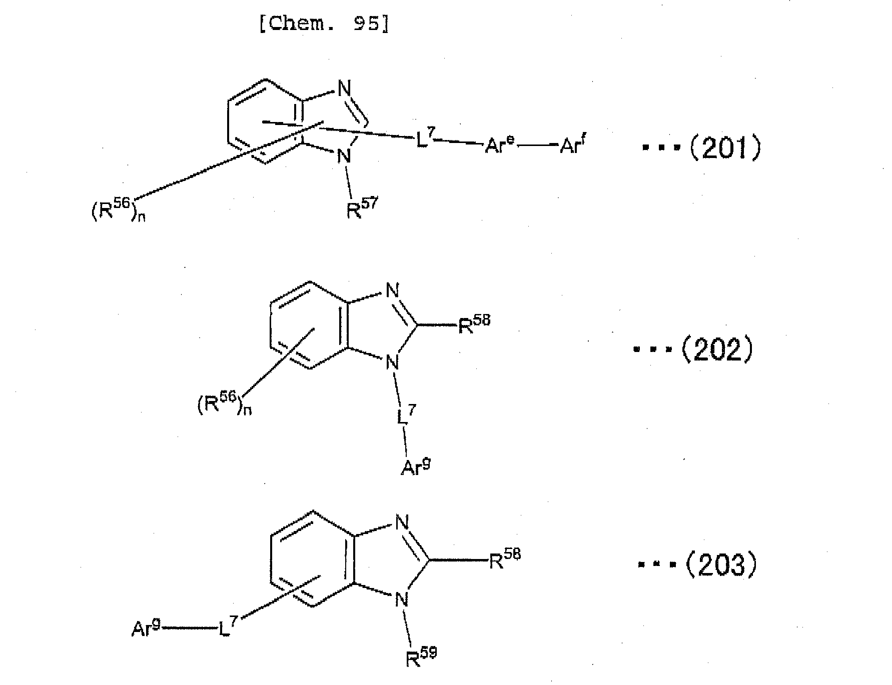

In the formulae (201) to (203), R⁵⁶ represents a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent, n represents an integer of 0 to 4, R⁵⁷ represents an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group having 1 to 20 carbon atoms, R⁵⁸ and R⁵⁹ each independently represent a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent, L⁷ represents a single bond, an arylene group which has 6 to 60 carbon atoms and which may have a substituent, a pyridinylene group which may have a substituent, a quinolinylene group which may have a substituent, or a fluorenylene group which may have a substituents, Ar^{e} represents an arylene group which has 6 to 60 carbon atoms and which may have a substituents, a pyridinylene group which may have a substituent, or a quinolinylene group which may have a substituent, and Ar^{r} represents a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent.
Ar^{g} represents an aryl group which has 6 to 60 carbon atoms and which may have a substituent, a pyridyl group which may have a substituents, a quinolyl group which may have a substituent, an alkyl group which has 1 to 20 carbon atoms and which may have a substituent, an alkoxy group which has 1 to 20 carbon atoms and which may have a substituent, or a group represented by -Ar^{e}-Ar^{f} (Ar^{e} and Ar^{f} each have the same meaning as that described above) .

In addition, as the nitrogen-containing ring derivative, a nitrogen-containing five-membered ring derivative is preferably exemplified. Examples of the nitrogen-containing five-membered ring include an imidazole ring, a triazole ring, a tetrazole ring, an oxadiazole ring, a thiadiazole ring, an oxatriazole ring, and a thiatriazole ring. Examples of the nitrogen-containing five-membered ring derivative include a benzoimidazole ring, a benzotriazolering, apyridinoimidazolering, apyrimidinoimidazole ring, and a pyridazinoimidazole ring. Particularly preferred is the compound represented by the following formula (B).

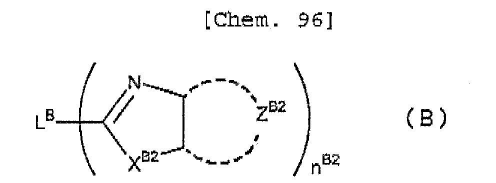

In the formulae (B), L^{B} represents a divalent or more linking group. Examples thereof include a carbon atom, a silicon atom, a nitrogen atom, a boron atom, an oxygen atom, a sulfur atom, metal atoms (for example, a barium atom and a beryllium atom), aromatic hydrocarbon rings, and aromatic heterocycles.

Of the nitrogen-containing five-membered ring derivatives each represented by the formula (B), a derivative represented by the following formula (B') is more preferred.

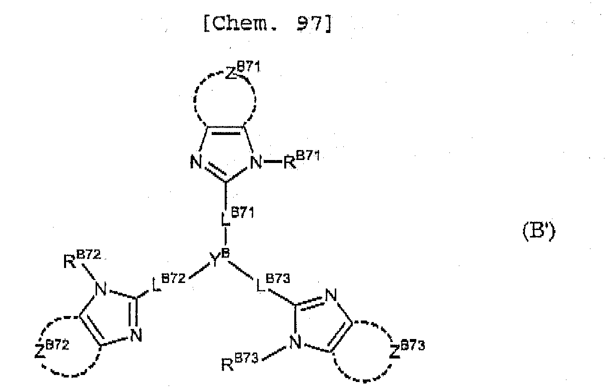

In the formula (B') , R^{B71}, R^{B72}, and R^{B73} each have the same meaning as that of R^{B2} in the formula (B).
Z^{B71}, Z^{B72}, and Z^{B73} each have the same meaning as that of Z^{a2} in the formula (B).
L^{B71}, L^{B72}, and L^{B73} each represent a linking group, and examples of the linking group include examples obtained by making the examples of L^{B} in the formula (B) divalent. The linking group is preferably a single bond, a divalent aromatic hydrocarbon ring group, a divalent aromatic heterocyclic group, or a linking group formed of a combination of two or more of them, or is more preferably a single bond. L^{B71}, L^{B72}, and L^{B73} may each have a substituent. Examples of the substituent include the same examples as those described for the substituent of the group represented by L^{B} in the formula (B) .
Y^{B} represents a nitrogen atom, a 1,3,5-benzenetriyl group, or a 2,4,6-triazinetriyl group.

A compound forming each of the electron injecting layer and the electron transporting layer is, for example, a compound having a structure obtained by combining an electron-deficient, nitrogen-containing five-membered ring skeleton or electron-deficient, nitrogen-containing six-membered ring skeleton and a substituted or unsubstituted, indole skeleton, substituted or unsubstituted carbazole skeleton, or substituted or unsubstituted azacarbazole skeleton as well as the polycyclic compound of the present invention. In addition, a suitable electron-deficient, nitrogen-containing five-membered ring skeleton or electron-deficient, nitrogen-containing six-membered ring skeleton is a molecular skeleton such as a pyridine, pyrimidine, pyrazine, triazine, triazole, oxadiazole, pyrazole, imidazole, quinoxaline, or pyrrole skeleton, or benzimidazole or imidazopyridine obtained when two or more of them fuse with each other. Of those combinations, a preferred combination is, for example, a combination of a pyridine, pyrimidine, pyrazine, or triazine skeleton and a carbazole, indole, azacarbazole, or quinoxaline skeleton. The skeleton may be substituted or unsubstituted.

Each of the electron injecting layer and the electron transporting layer may be of a monolayer structure formed of one or two or more kinds of the materials, or may be of a multi-layered structure formed of the plurality of layers identical to or different from each other in composition. Materials for those layers each preferably have a n-electron-deficient, nitrogen-containing heterocyclic group.

In addition, an insulator or semiconductor serving a an inorganic compound as well as the nitrogen-containing ring derivative is preferably used as a component of the electron injecting layer. When the electron injecting layer is formed of an insulator or semiconductor, current leakage can be effectively prevented, and the electron injecting property of the layer can be improved.
As the insulator, at least one metal compound selected from the group consisting of alkali metal chalcogenides, alkaline earth metal chalcogenide, alkali metal halides, and alkaline earth metal halides is preferably used. It is preferred that the electron injecting layer be formed of the alkali metal chalcogenide or the like since the electron injecting property can be further improved. To be specific, preferred examples of the alkali metal chalcogenide include Li₂O, K₂O, Na₂S, Na₂Se, and Na₂O, and preferred examples of the alkaline earth metal chalcogenide include CaO, BaO, SrO, BeO, BaS, and CaSe. In addition, preferred examples of the alkali metal halide include LiF NaF, KF, LiCl, KCl, and NaCl. Further, preferred examples of the alkaline earth metal halide include fluorides such as CaF₂, BaF₂, SrF₂, MgF₂, and BeF₂ and halides other than the fluorides.
In addition, examples of the semiconductor include oxides, nitrides, and oxynitrides containing at least one element selected from the group consisting of Ba, Ca, Sr, Yb, Al, Ga, In, Li, Na, Cd, Mg, Si, Ta, Sb, and Zn. One kind of them may be used alone, or two or more kinds of them maybe used in combination. In addition, it is preferred that the inorganic compound forming the electron injecting layer form a microcrystalline or amorphous insulating thin film. When the electron injecting layer is formed of the insulating thin film, a more uniform thin film can be formed, and defects of pixels such as dark spots can be decreased. It should be noted that examples of the inorganic compound include alkali metal chalcogenides, alkaline earth metal chalcogenides, alkali metal halides, and alkaline earth metal halides.
In addition, the reducing dopant can be preferably incorporated into the electron injecting layer in the present invention.
It should be noted that the thickness of each of the electron injecting layer and the electron transporting layer, which is not particularly limited, is preferably 1 to 100 nm.

An aromatic amine compound such as an aromatic amine derivative represented by the formula (I) is suitably used in the hole injecting layer or hole transporting layer (a hole injecting/transporting layer is also included in this category) .

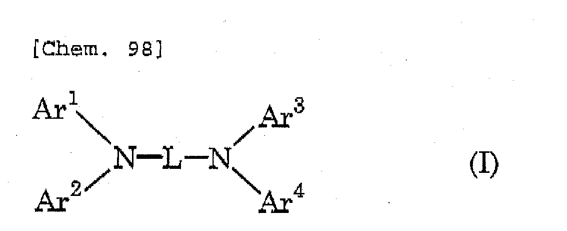

In the formula (I) Ar¹ to Ar⁴ each represent a substituted or unsubstituted aryl group having a ring formed of 6 to 50 carbon atoms, or a substituted or unsubstituted heteroaryl group having a ring formed of 5 to 50 atoms.

L represents a linking group, and specifically, a substituted or unsubstituted arylene group having a ring formed of 6 to 50 carbon atoms, a substituted or unsubstituted heteroarylene group having a ring formed of 5 to 50 atoms, or a bivalent group in which two or more arylene groups or heteroarylene groups are bonded by a single bond, an ether bond, a thioether bond, with an alkylene group having 1 to 20 carbon atoms, an alkenylene group having 2 to 20 carbon atoms, and an amino group.

In addition, an aromatic amine represented by the following formula (II) is also suitably used in the formation of the hole injecting layer or hole transporting layer.

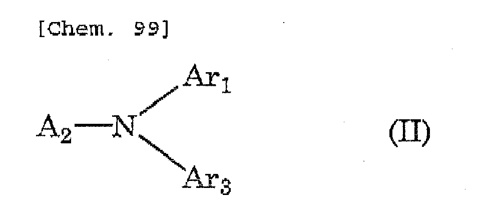

In the formula (II), the definition of Ar₁ to Ar₃ is the same as that of Ar¹ to Ar⁴ in the formula (I).

The compound of the present invention can be used in each of the hole injecting layer, the hole transporting layer, the electron injecting layer, and the electron transporting layer because the compound can transport both a hole and an electron.

In the present invention, the anode in the organic EL device has the function of injecting holes into the hole transporting layer or the light emitting layer. It is effective that the anode has a work function of 4.5 eV or more. Specific examples of the material for the anode used in the present invention include indium tin oxide alloys (ITO), tin oxide (NESA), gold, silver, platinum, and copper. In addition, as the cathode, a material having a small work function is preferred in view to inject an electron into an electron-injecting layer or a light-emitting layer. Examples of the cathode material are not particularly limited, and specifically, indium, aluminum, magnesium, a magnesium-indium alloy, a magnesium-aluminum alloy, an aluminum-lithium alloy, an aluminum-scandium-lithium alloy, and a magnesium-silver alloy may be used.

The method of forming the layers in the organic EL device of the present invention is not particularly limited. A conventionally known process such as the vacuum vapor deposition process or the spin coating process can be used. The organic thin film layer which is used in the organic EL device of the present invention and includes the compound represented by the formula (1) can be formed in accordance with a known process such as the vacuum vapor deposition process or the molecular beam epitaxy process (MBE process) or, using a solution prepared by dissolving the compounds into a solvent, in accordance with a coating process such as the dipping process, the spin coating process, the casting process, the bar coating process, or the roll coating process.
The thickness of each layer in the organic thin film layer in the organic EL device of the present invention is not particularly limited. In general, an excessively thin layer tends to have defects such as pin holes, whereas an excessively thick layer requires a high applied voltage to decrease the efficiency. Therefore, a thickness in the range of several nanometers to 1 µm is typically preferred.

### Examples

Next, the present invention is described in more detail by way of examples, but the present invention is not limited to the following examples. It should be noted that, in the synthetic examples below, DMF refers to dimethylformamide, THF refers to tetrahydrofuran, DME refers to dimethoxyethane, NBS refers to N-bromosuccinimide, Ph refers to a phenyl group, AcOEt refers to ethyl acetate, and NMP refers to N-methyl pyrrolidone.

### Synthesis Example 1 (Synthesis of Compound No.1)

### (1) Synthesis of Compound 1

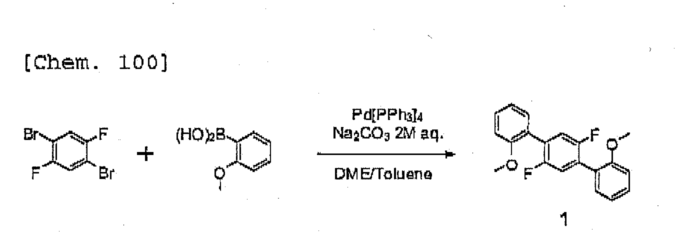

1,4-dibromo-2,5-difluorobenzene (49.3 g, 181.5mmol), 2-methoxyphenylboronic acid (66.2 g, 435,6 mmol), a 2 M aqueous solution of Na₂CO₃ (363 mL, 726 mmol)), DME (360 mL), toluene (360 mL), and Pd [PPh₃]₄ (20. 8 g, 18.0 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (500 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 38.5 g in 65% yield. FD-MS analysis C₂₀H₁₆F₂O₂: theoretical value 326, observed value 326

(2) Synthesis of Compound 2

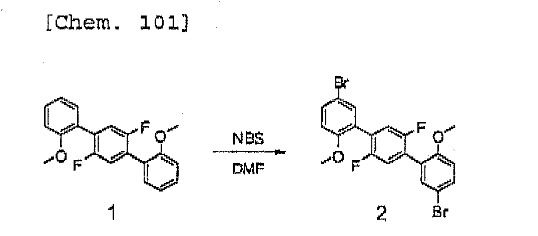

Compound 1 (36.6 g, 112.2 mmol) , NBS (39.9g, 224 mmol), and DMF (1000 mL) were loaded into a three-necked flask, and the mixture was stirred under an Ar atmosphere at room temperature for 8 hours. After the completion of the reaction, the resultant sample was transferred to a separating funnel, and water (1000 mL) was charged into the funnel. Then, the mixture was extracted with AcOEt. The resultant sample was purified by column chromatography, whereby a white solid was obtained in an amount of 38 g in 70% yield.
FD-MS analysis C₂₀H₁₄Br₂F₂O₂: theoretical value 484, observed value 484

(3) Synthesis of Compound 3

Compound 2 (37.2 g, 76.8 mmol)), a 1 M solution of BBr₃ in CH₂Cl₂ (180 mL, 180 mmol), and CH₂Cl₂ (500 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by column chromatography, whereby a white solid was obtained in an amount of 28 g in 80% yield.
FD-MS analysis CₐH₁₀Br₂F₂₀O₂: theoretical value 456, observed value 456

(4) Synthesis of Compound 4

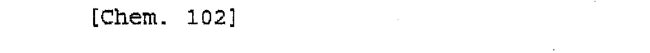

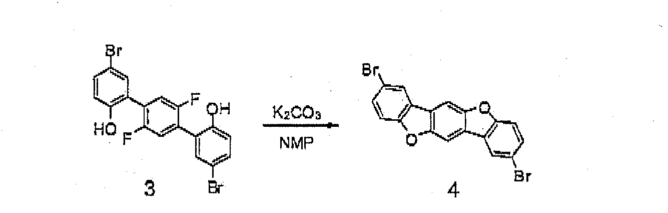

(Compound 3 (27.4 g, 60.1 mmol), K₂CO₃ (18.2g, 132mmol), and NMP (250 mL) were loaded into a three-necked flask, and the mixture was stirred under an Ar atmosphere at 150°C for 8 hours. After the completion of the reaction, the resultant was cooled to room temperature. The resultant sample was transferred to a separating funnel, and water (500 mL) was charged into the funnel. Then, the mixture was extracted with AcOEt. The resultant sample was purified by column chromatography, whereby a white solid was obtained in an amount of 20 g in 80% yield.
FD-MS analysis C₁₃H₈Br₂O₂ theoretical value 416, observed value 416

(5) Synthesis of Compound No. 1

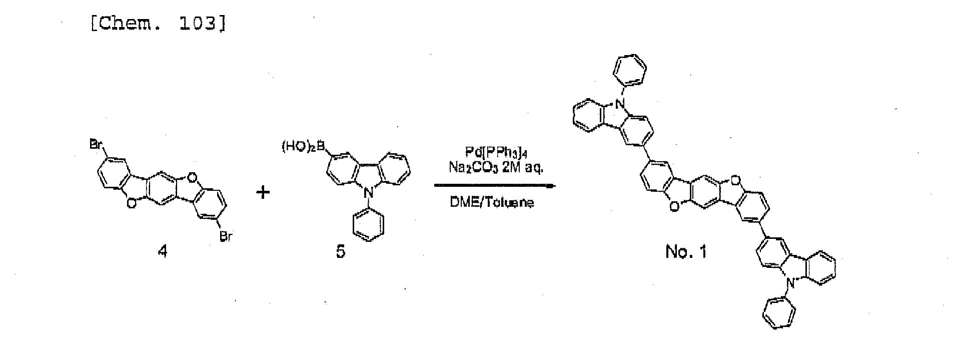

Compound 4 (2.5 g, 6.0 mmol), Compound 5 (3.8 g, 13.2 mmol)) , a 2 M aqueous solution of Na₂CO₃ (12 mL, 24 mmol), DME (12 mL), toluene (12 mL), and Pd [PPh₃]₄ (0.35 g, 0,3 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No.1) was obtained. The powder was purified by sublimation, whereby a white solid was obtained in an amount of 2.0 g in 45% yield.
FD-MS analysis C₅₄H₃₂N₂O₂: theoretical value 740, observed value 740

Synthesis Example 2 (Synthesis of Compound No. 11)

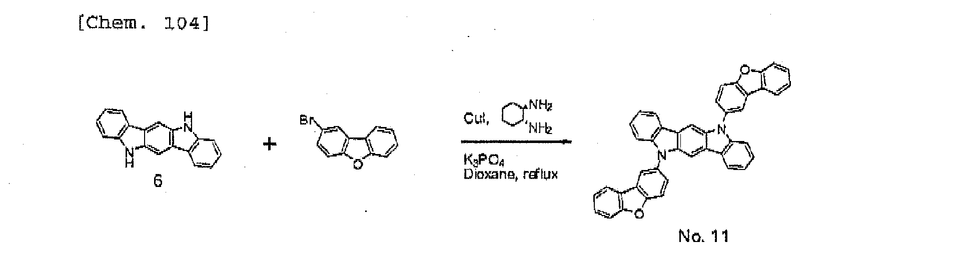

Compound 6 (2.6g, 10 mmol), 2-bromodibenzofuran (5.0 g, 20 mmol), CuI (1.9 g, 10 mmol), trans-cyclohexane-1,2-diamine (3.4 g, 30 mmol), K₃PO₄ (8.5 g, 40 mmol), and 1,4-dioxane (10 mL) were loaded into a three-necked flask, and the mixture was refluxed under an argon atmosphere for 10 hours.
After the completion of the reaction, the resultant was cooled to room temperature. The resultant sample was transferred to a separating funnel, and water (50 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 11) was obtained. The powder was purified by sublimation, whereby a white solid was obtained in an amount of 2.1 g in 35% yield.
FD-MS analysis C₄₂H₂₄N₂O₂: theoretical value 588, observed value 588

Synthesis Example 3 (Synthesis of Compound No. 22)

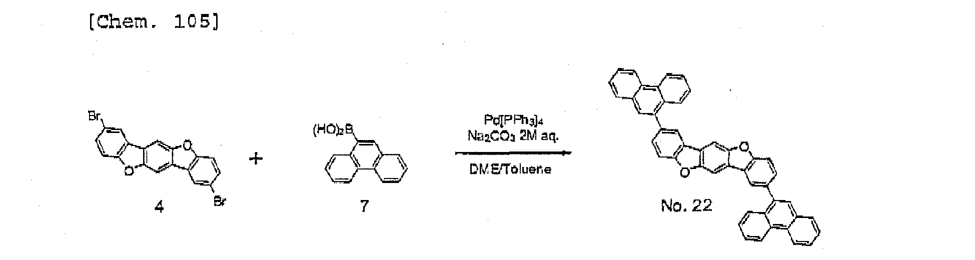

Compound 4 (2.5 g, 6.0 mmol) , Compound 7 (2.9 g, 13.2 mmol), a 2 M aqueous solution of Na₂CO₃ (12 mL, 24 mmol), DME (12 mL), toluene (12 mL), and Pd [PPh₃]₄ (0.35 g, 0.3 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 22) was obtained. The powder was purified by sublimation, whereby a white solid was obtained in an amount of 1.3 g in 35% yield.
FD-MS analysis C₄₆H₂₆O₂: theoretical value 610, observed value 610

(Synthesis Example 4 (Synthesis of Compound No. 28)

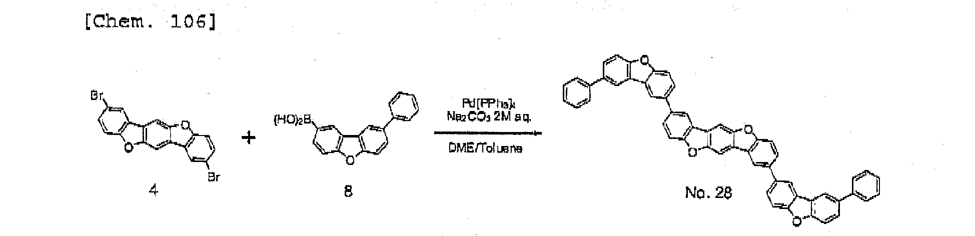

Compound 4 (2.5 g, 6.0 mmol), Compound 8 (3.8 g, 13,2 mmol) , a 2 M aqueous solution of Na₂CO₃ (12 mL, 24 mmol), DME (12 mL)), toluene (12 mL), and Pd [PPh₃]₄ (0.35 g, 0.3 mmol) were loaded into a three-neeked flask, and the mixture was refluxed under an Ar atmosphere for 8 hours.
After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 28) was obtained. The powder was purified by sublimation, whereby a white solid was obtained in an amount of 2.1 g in 47% yield.
FD-MS analysis C₅₄H₃₀O₄: theoretical value 742, observed value 742

Synthesis Example 5 (Synthesis of Compound No. 39)

### (1) Synthesis of Compound 9

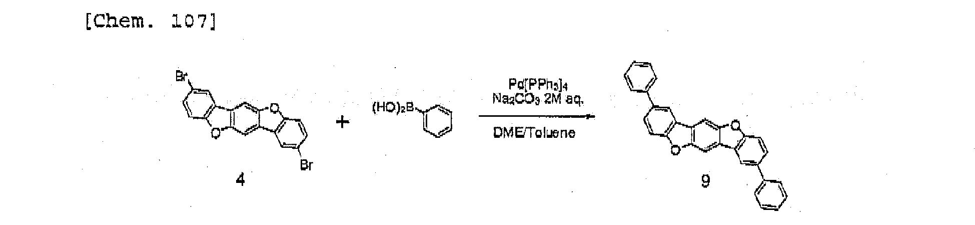

Compound 4 (10. 0 g, 24.0 0 mmol), phenylboronic acid (6.4 g, 52.8 mmol), a 2 M aqueous solution of Na₂CO₃, (48 mL, 96 mmol), DME (48 mL), toluene (48 mL), and Pd[PPh₃]₄ (1.4 g, 1.2 mmol) were loaded into a three-necked flask, and the mixture was refluxed under an Ar atmosphere for 8 hours.
After the completion, of the reaction, the resultant was cooled to room temperature. The resultant sample was transferred to a separating funnel, and water (300 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 7.5 g in 76% yield.
FD-MS analysis C₃₀H₁₉O₂; theoretical value 410, observed value 410

(2) Synthesis of Compound 10

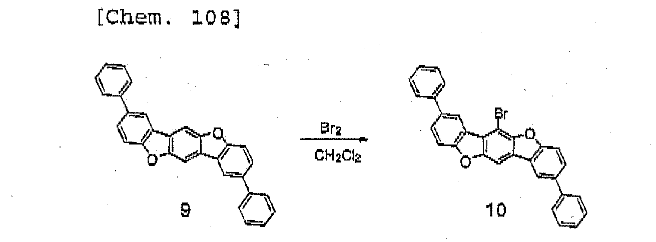

Compound 9 (7.5 g, 18.3 mmol) and CH₂Cl₂ (100 mL) were loaded into a three-necked flask, and bromine (2.9 g, 18.3 mmol) was dropped thereto under an Ar atmosphere at 0°C. After that, the mixture was stirred at room temperature for 8 hour. After the completion of the reaction, the resultant sample was transferred to a separating funnel, and water (50 mL) was charged into the funnel. Then, the mixture was extracted with CH₂Cl₂. The organic layer was washed with a saturated aqueous solution of NaNO₂ (50 mL), dried with MgSO₄, and then filtered and concentrated. The resultant sample was purified by column chromatography, whereby a white solid was obtained in an amount of 5.4 g in 60% yield.
FD-MS analysis C₃₀H₁₇BrO₂: theoretical value 489, observed value 489

(3) Synthesis of Compound No. 39

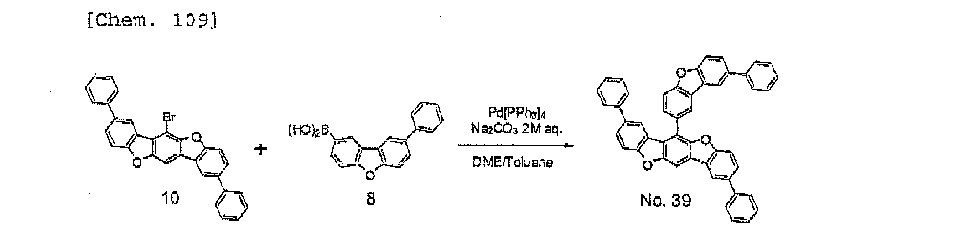

Compound 10 (4.9g, 10.0 mmol), Compound 8 (3.2g, 11.0 mmol), a 2 M aqueous solution of Na₂CO₃ (10 mL, 20 mmol), DME (20 mL), toluene (20 mL), and Pd [PPh₃]₄ (0.58 g, 0.5 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (100 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 39) was obtained. The powder was purified by sublimation, whereby a white solid was obtained in an amount of 1.8 g in 28% yield.
FD-MS analysis C₄₈H₂₀O₃: theoretical value 652, observed value 652

Synthesis Example 6 (Synthesis of Compound No. 57)

### (1) Synthesis of Compound 11

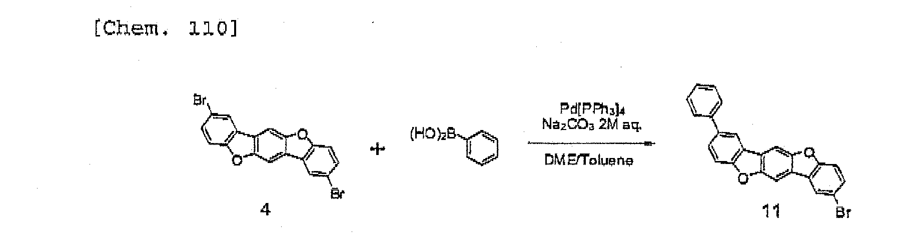

Compound 4 (16.6 g, 40 mmol), phenylboronic acid (4.9 g, 40 mmol), a 2 M aqueous solution of Na₂CO₃ (40 mL, 80 mmol), DME (80 mL), toluene (80 mL), and Pd[PPh₃]₄ (2.3g ,20 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (300 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 10.7 g in 65% yield. FD-MS analysis C₂₄H₁₃BrO₂: theoretical value 413, observed value 413

(2) Synthesis of Compound 12

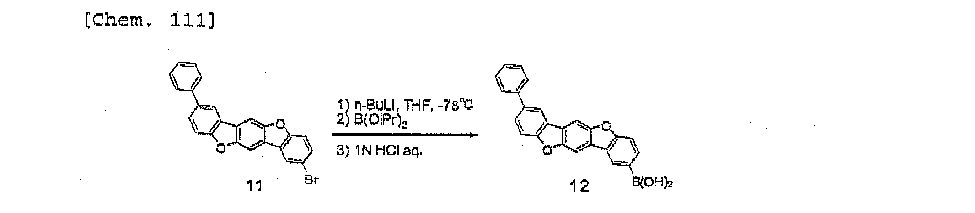

Compound 11 (10 g, 24.2mmol) and THF (240mL) were loaded into a three-necked flask, and the mixture was cooled to -78°C. Then, n-BuLi (1.65 M solution in n-hexane, 16.1 mL, 26.6 6 mmol) was added dropwise to the flask, and the resultant mixture was stirred at -78°C for 20 minutes. Triisopropyl borate (13.7 g, 72.6 mmol) was added to the resultant, and the mixture was stirred at -78°C for 1 hour. After that, the resultant was left to stand at room temperature overnight. Then, 1 N HCl (100 mL) was charged into the resultant, and the mixture was stirred at room temperature for 1 hour. The resultant sample was concentrated, and was then transferred to a separating funnel. Water (100 mL) was charted into the funnel, and the mixture was extracted with CH₂Cl₂ The extract was dried with MgSO₄, and was then filtered and concentrated. The resultant sample was purified by recrystallization (toluene-hexane), whereby a white solid was obtained in an amount of 5.5 g in 60% yield.

(3) Synthesis of Compound No. 57

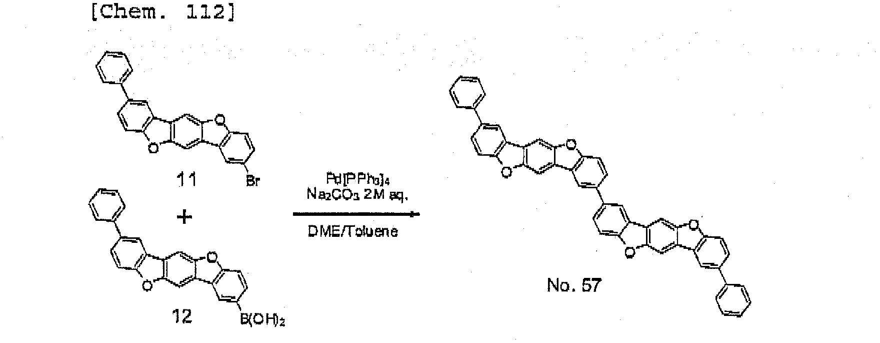

Compound 11 (2.5 g, 6.0 mmol), Compound 12 (2.5 g, 6.6 mmol), a 2 M aqueous solution, of Na₂CO₃ (6 mL, 12 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (100 mL) was charged into the funnel. Then, the mixture was extracted with CH₂Cl₂, The extract was dried with MgSO₄, and was then filtered and concentrated. The resultant sample was purified by silica gel column chromatography. The purified product was concentrated to dryness, and was then recrystallized twice, whereby a white powder (Compound No. 57) was obtained. The powder was purified by sublimation, whereby a white solid was obtained in an amount of 1.6 g in 40% yield.
FD-MS analysis C₄₈H₂₆O₄- theoretical value 666, observed value 666

Synthesis Example 7 (synthesis of Compound No. 58)

### (1) Synthesis of Compound 14

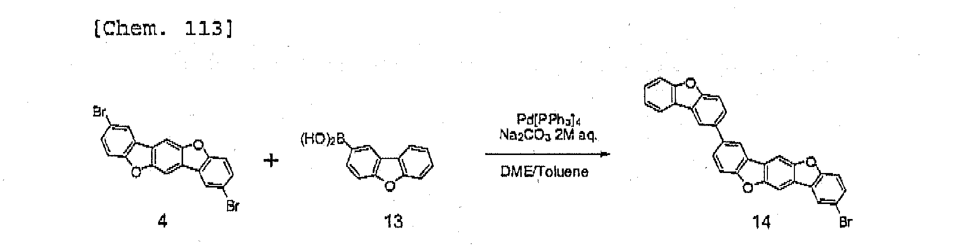

Compound 4 (16.6 g, 40 mmol), Compound 13 (8.5 g, 40 mmol), a 2 M aqueous solution of Na₂CO₃ (40 mL, 80 mmol), DME (80 mL), toluene (80 mL), and Pd[PPh₃]₄ (2.3 g, 2.0 mmol) were loaded into a three-necked flask, and the mixture was refluxed under an Ar atmosphere for 8 hours.
After the completion, of the reaction, the resultant was cooled to room temperature. The resultant sample was transferred to a separating funnel, and water (300 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 11.1 g in 55% yield.
FD-MS analysis C₃₀H₁₅BrO₃: theoretical value 503, observed value 503

(2) Synthesis of Compound 15

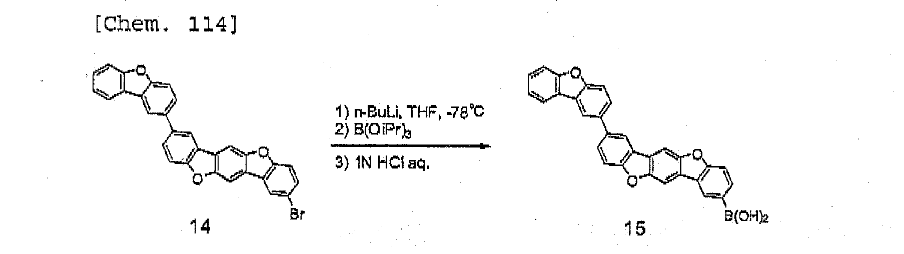

(Compound 14 (11 g, 21.9 mmol) and THF (220 mL) were loaded into a three-necked flask, and the mixture was cooled to -78°C. Then, n-BuLi (1.65 M solution in n-hexane, 14.5 mL, 24.0 mmol)) was added dropwise to the flask, and the resultant mixture was stirred at -78°C for 20 minutes. Triisopropyl borate (12.4 g, 65.6 mmol) was added to the resultant, and the mixture was stirred at -78°C for 1 hour. After that, the resultant was left to stand at room temperature overnight. Then, 1 NHCl (100 mL) was charged into the resultant, and the mixture was stirred at room temperature for 3. hour. The resultant sample was concentrated, and was then transferred to a separating funnel. Water (100 mL) was charged into the funnel, and the mixture was extracted with CH₂Cl₂. The extract was dried with MgSO₄, and was then filtered and concentrated. The resultant sample was purified by recrystallization (toluene -hexane), whereby a white solid was obtained in an amount of 6.4 g in 62% yield.

(3) Synthesis of Compound No. 58

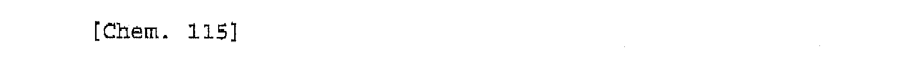

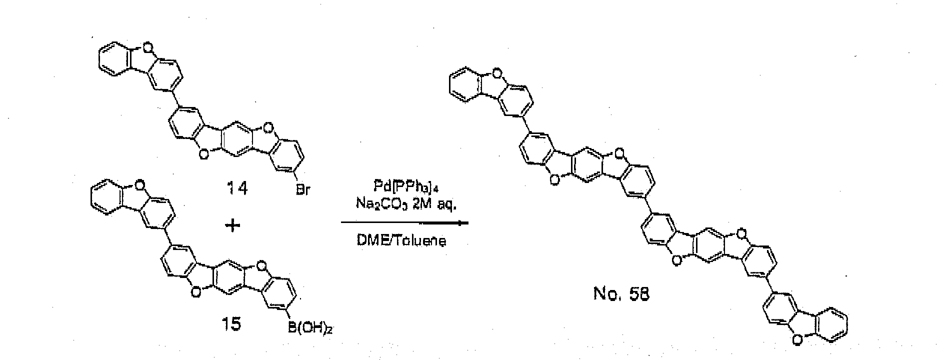

Compound 14 (3.0 g, 6.0 mmol), Compound 15 (3.1 g, 6.6 mmol), a 2 M aqueous solution of Na₂CO₃ (6 mL, 12 mmol), DME (12 mL), toluene (12 mL), and Pd[PPh₃]₄ (0.35 g, 0.3 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (100 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound No. 58) was obtained. The powder was purified by sublimation, whereby a white solid was obtained in an amount of 1.3 g in 26% yield.
FD-MS analysis C₆₀H₃₀O₆: theoretical value 846, observed value 846

Synthesis Example 8 (Synthesis of Compound NO. 60)

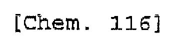

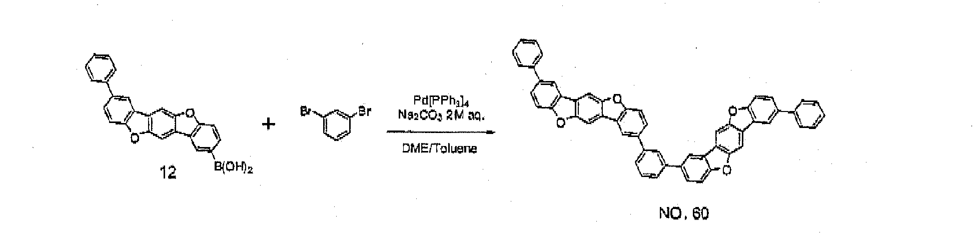

Compound 12 (5.5g, 14.5mmol), 1,3-dibromobenzene (1.7 g, 7.3 mmol), a 2 M aqueous solution of Na₂CO₃ (15 mL, 30 mmol), DME (15 mL), toluene (15 mL), and Pd [PPh₃]4 (0.42 g, 0.37 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound NO. 60) was obtained. The powder was purified by sublimation, whereby a white solid was obtained in an amount of 1.6 g in 29% yield.
FD-MS analysis C₅₄H₃₀O₄: theoretical value 742, observed value 742

Synthesis Example 9 (Synthesis of Compound No. 62)

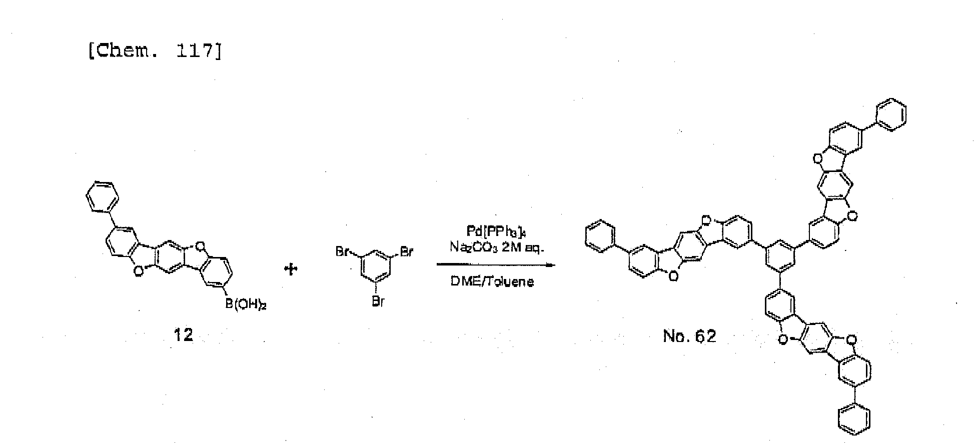

Compound 12 (8.3 g, 21.9 mmol), 1,3,5-tribromobenzene (2.3 g, 7.3 mmol), a 2 M aqueous solution of Na₂CO₃ (22.5 mL, 45 mmol), DME (15 mL), toluene (15 mL), and Pd[PPh₃]₄ (0.63 g, 0.56 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (150 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (No. 62) was obtained. The powder was purified by sublimation, whereby a white solid was obtained in an amount of 1.1 g in 14% yield.
FD-MS analysis C₇₈H₄₂O₆: theoretical value 1075, observed value 1075

Synthesis Example 10 (Synthesis of Compound No. 20)

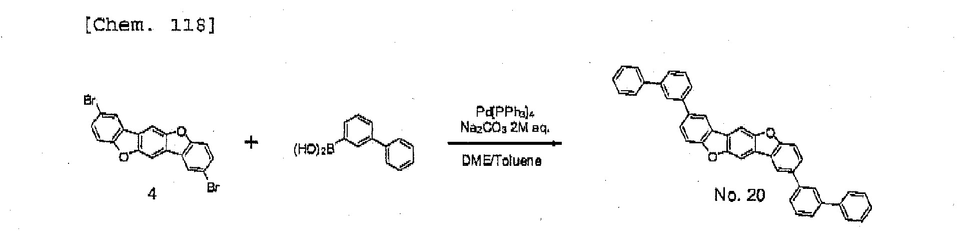

Compound 4 (2.5 g, 6.0 mmol), 3-biphenylboronic acid (2.6 g, 13.2 mmol), a 2 M aqueous solution of Na₂CO₃ (12 mL, 24 mmol), DME (12 mL), toluene (12 mL), and Pd [PPh₃]₄ (0.35 g, 0.3 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound NO. 20) was obtained. The powder was purified by sublimation, whereby a white solid was obtained in an amount of 1.3 g in 39% yield.
FD-MS analysis C₄₂H₂₆O₂: theoretical value 562, observed value 562

Synthesis Example 11 (Synthesis of Compound No. 67)

### (1) Synthesis of Compound 17

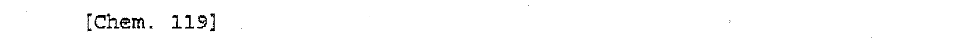

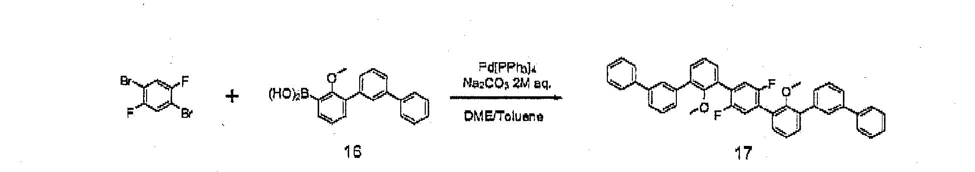

1,4-dibromo-2,5-difluorobenzene (5.4 g, 20.0 mmol) Compound 16 (12.8 g, 42.0 mmol), a 2 M aqueous solution of Na₂CO₃ (40 mL, 80.0 mmol), DME (40 mL), toluene (40 mL), and Pd[PPh₃] (1.2 g, 1.0 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (100 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 8.8 g in 70% yield.
FD-MS analysis C₄₄H₃₂F₂O₂: theoretical value 630, observed value 630

(2) Synthesis of Compound 18

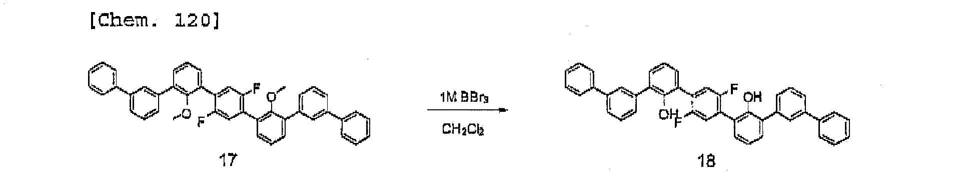

Compound 17 (8.8 g, 14.0 mmol), a 1 M solution of BBr₃ in CH₂Cl₂ (34 mL, 34.0 mmol), and CH₂Cl₃ (140 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by silica gel column chromatography, whereby a white solid was obtained in an amount of 7.8 g in 93% yield.
FD-MS analysis C₄₂H₂₈F₂O₂: theoretical value 602, observed value 602

(3) Synthesis of Compound No. 67

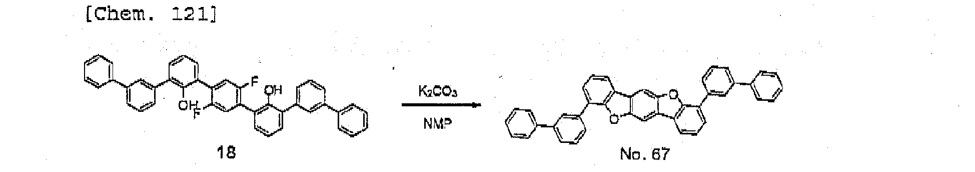

Compound 18 (7.8 g, 12.9mmol), K₂CO₃ (7.2g, 51.8mmol), and NMP (50 mL) were loaded into a three-necked flask, and the mixture was stirred under an Ar atmosphere at 200°C for 3 hours. After the completion of the reaction, the resultant was cooled to room temperature. Toluene (500 mL) was charged into the resultant sample. The mixture was transferred to a separating funnel, and was washed with water. The washed product was dried with MgSO₄, and was then filtered and concentrated. The resultant sample was purified by silica gel column chromatography. The purified product was concentrated to dryness, and was then recrystallized twice, whereby a white powder (compound No. 67) was obtained. The powder was purified by sublimation, whereby a white solid was obtained in an amount of 2.5 g in 35% yield.
FD-MS analysis C₄₂H₂₆O₂: theoretical value 562, observed value 562

Synthesis Example 12 (Synthesis of Compound NO. 455)

### (1) Synthesis of Compound 19

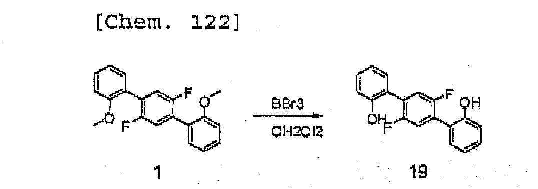

Compound (30.0 g, 91.9mmol) and CH₂Cl₂ (500mL) were loaded into a three-necked flask, and the mixture was cooled to 0°C. Then, 1 M BBr₃ (220 mL, 220 mmol) was added to the flask, and the resultant mixture was stirred at room temperature for 24 hours.
After the completion of the reaction, the obtained solution was cooled to -78°C, and quenched with methanol (50 mL) and water (100 mL). The sample was transferred to a separating funnel, and the mixture was extracted with CH₂Cl₂. The extract was dried with MgSO₄, and was then filtered and concentrated. The obtained sample was purified by silica gel column chromatography. The purified product was concentrated to dryness, whereby a white solid was obtained in an amount of 24.7 g in 90% yield.
FD-MS analysis C₁₈H₂F₂O₂: theoretical value 298, observed value 298

(2) Synthesis of Compound 20

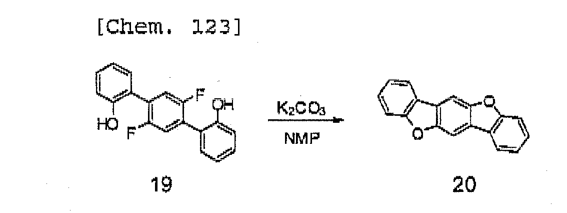

Compound 19 (20.0 g, 67.1mmol) and NMP (700 mL) were loaded into a three-necked flask, and Compound 40 was dissolved completely. K₂CO₃ (37.1 g, 268.2 mmol) was added to the flask, followed by stirring at 200°C for 2 hours.
After the completion of the reaction, the obtained solution was cooled to room temperature. The sample was transferred to a separating funnel and toluene (2 L) was added thereto. The resultant sample was washed with water. The washed product was concentrated to dryness, and was then recrystallized, whereby a white solid was obtained in an amount of 12.7 g in 73% yield.
FD-MS analysis C₁₈H₁₀O₂: theoretical value 258, observed value 258

(3) Synthesis of Compound 21

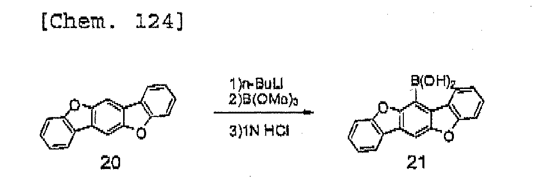

Compound 20 (12.0 g, 46.5 mmol) and THF (500 mL) were loaded into a three-necked, flask, and the mixture was cooled to -78°C. Then, n-BuLi (2.63 M solution in hexane, 18.6 ml, 48.8mmol) was added to the flask, and the resultant mixture was stirred at room temperature under an Ar atmosphere for 1 hour. Next, the resultant was cooled to -78°C again, trimethyl borate (10.4 g, 100 mmol) was added to the resultant. After being stirred at -78°C for 10 minutes, the mixture was stirred at room temperature for 1 hour. After the completion of the reaction, the resultant was concentrated to about the half volume. 1 N HCl (200 mL) was added to the concentrated resultant, followed by stirring at room temperature for 1 hour. The resultant sample was transferred to a separating funnel, and the mixture was extracted with ethyl acetate. The extract was dried with MgSO₄, and was then concentrated. The concentrated product was subjected to dispersion washing with a toluene/hexane mixed solvent, whereby a white solid was obtained in an amount of 12.6 g in 90% yield.

(4) Synthesis of Compound No. 455

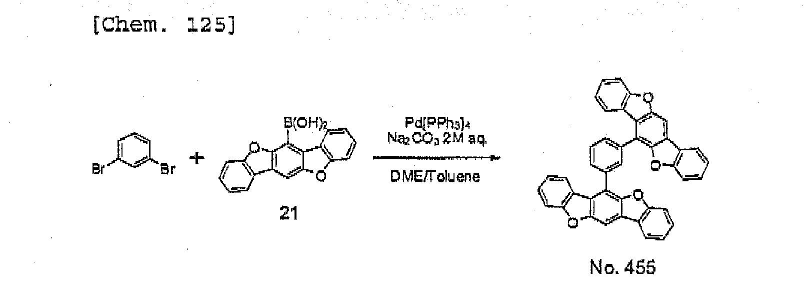

Compound 21 (3.8g, 12.6mmol), 1,3-dibromobenzene (1.4 g, 6.0 mmol), a 2 M aqueous solution of Na₂CO₃ (12 mL, 24 mmol), DME (12 mL), Toluene (12 mL), and Pd[PPh₃]₄ (0.35 g, 0.30 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (50 mL) was charged into the funnel. Then, the mixture was extracted with CH₂Cl₂. The extract was dried with MgSO₄, and was then filtered and concentrated. The resultant sample was purified by silica gel column chromatography. The purified product was concentrated to dryness, and was then recrystallized twice, whereby a white powder (Compound NO. 455) was obtained. The powder was purified by sublimation, whereby a white solid was obtained in an amount of 1.8 g in 52% yield.
FD-MS analysis C₄₂H₂₂O₄: theoretical value 590, observed value 590

Synthesis Example 13 (Synthesis of Compound No. 461)

### (1) Synthesis of Compound 22

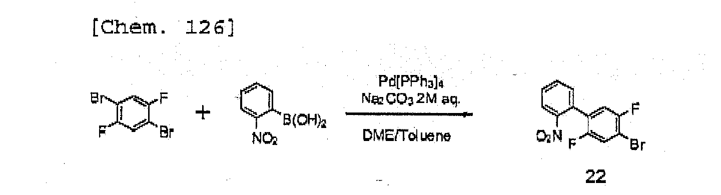

1,4-dibromo-2,5-difluorobenzene (37.5 g, 138.0 mmol), 2-nitrophenylboronic acid (23.0 g, 138.0 mmol), a 2 Maqueous solution of Na₂CO₃ (138 mL, 267 mmol), DME (275 mL), toluene (275 mL), and Pd[PPh₃]₄ (8.0 g, 6.9 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (250 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 26.1 g in 60% yield. FD-MS analysis C₁₂H₆BrF₂NO₂: theoretical value 314, observed value 314

(2) Synthesis of Compound 23

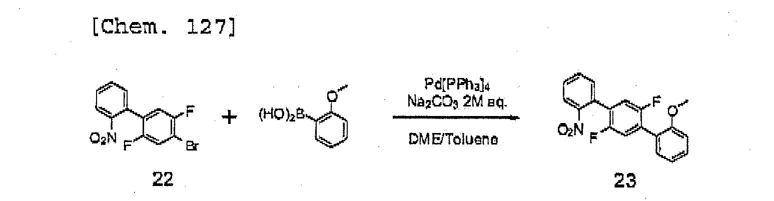

Compound 22 (26.1g, 83. 0 mmol), 2-methoxyphenylboronic acid (15.2 g, 99.6 mmol), a 2 M aqueous solution of Na₂CO₃ (83 mL, 166 mmol), DME (165 mL), toluene (165 mL), and Pd[PPh₃]₄ (4.8 g, 4.2 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (200 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 22.1 g in 78% yield. FD-MS analysis C₁₉H₁₃F₂NO₃: theoretical value 341, observed value 341

(3) Synthesis of Compound 24

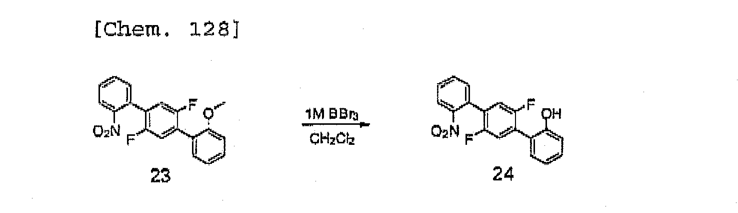

Compound 23 (22,1g, 64.8 mmol), a 1M solution of BBr₃ in CH₂Cl₂ (163 mL, 163 mmol), and CH₂Cl₂ (500 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by column chromatography, whereby a white solid was obtained in an amount of 20.2 g in 95% yield.
FD-MS analysis C₁₈H₁₁F₂NO₃: theoretical value 327, observed value 327

(4) Synthesis of Compound 25

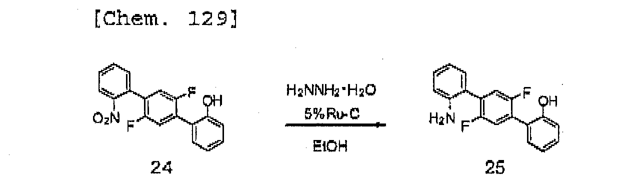

Compound 24 (20.2 g, 61.6 mmol), 5% Ru-C (2.47 g), and ethanol (230 mL) were loaded into a three-necked flask, and the mixture was stirred under an Ar atmosphere at 70°C. Hydrazine monohydrate (18.6 g, 371.9 mmol) was dissolved in ethanol (20 mL), and the solution was added dropwise to the flask. After that, the reaction mixture was refluxed for 8 hours. After the completion of the reaction, the mixture was cooled to room temperature. The resultant sample was filtered under reduced pressure, and the filtrate was concentrated. The resultant sample was purified by silica gel column chromatography, whereby a white solid was obtained in an amount of 15.6 g in 85% yield.
FD-MS analysis C₁₈H₁₃F₂NO: theoretical value 297, observed value 297

(5) Synthesis of Compound 26

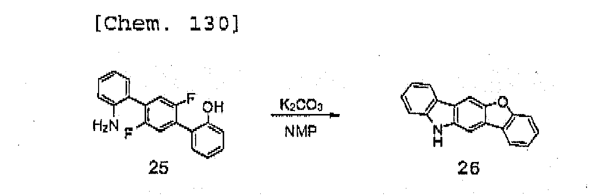

Compound 25 (15.6 g, 52.3 mmol) K₂CO₃ (28.9 g, 208.9 mmol), and NMP (500 mL) were loaded into a three-necked flask, and the mixture was stirred under an Ar atmosphere at 150°C for 8 hours. After the completion of the reaction, the resultant was cooled to room temperature. Water (500 mL) was charged into the resultant sample. Then, the solid precipitate was collected by filtration. The resultant sample was purified by column chromatography, and was then recrystallized twice, whereby a white solid was obtained in an amount of 10.8 g in 80% yield.
FD-MS analysis C₁₈H₁₁NO: theoretical value 257, observed value 257

(6) Synthesis of compound No. 461

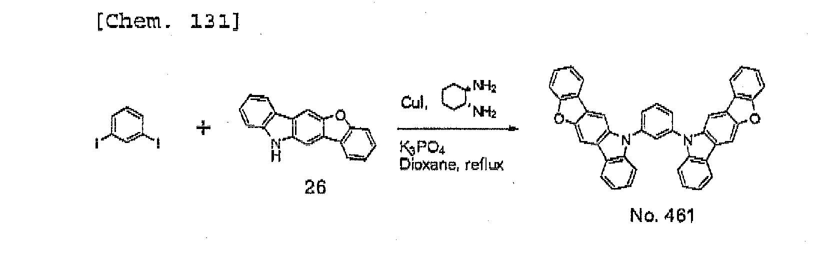

1,3-diiodobenzene (2.1 g, 6.5 mmol), Compound 26 (4.0 g, 15.6mmol), Cul (1.3 g, 6.5 mmol), trans-cyclohexane-1,2-diamine (2.2g, 19.5 mmol), K₃PO₄ (5.5g, 25.9 mmol), and 1,4-dioxane (7.5 mL) were loaded into a three-necked flask, and the mixture was refluxed under an Ar atmosphere for 10 hours.
After the completion of the reaction, the resultant was cooled to room temperature. The resultant sample was transferred to a separating funnel, and water (100 mL) was charged into the funnel. Then, the mixture was extracted with CH₂Cl₂. The resultant sample was purified by column chromatography. The purified product was concentrated to dryness, and was then recrystallized twice, whereby a white powder (Compound No. 461) was obtained. The powder was purified by sublimation, whereby a white solid was obtained in an amount of 1.3 g in 35% yield.
FD-MS analysis C₄₂H₂₄N₂O₂: theoretical value 588, observed value 588

synthesis Example 14 (Synthesis of Compound No. 87)

### (1) Synthesis of Compound 28

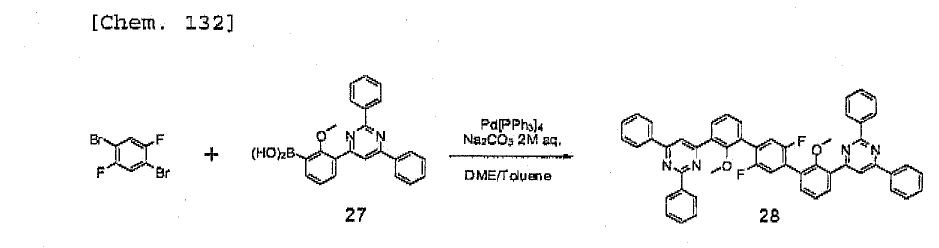

1,4-dibromo-2,5-difluorobenzene (5.4 g, 20.0 mmol), Compound 27 (16.1 g, 42. 0 mmol), and a 2 M aqueous solution of Na₂CO₃ (40 mL, 80.0 mmol) DME (40 mL), toluene (40ML), and Pd [PPh₃]₄ (1.2 g, 1.0 mmol) were loaded into a three-necked flask, and the mixture was refluxed under an Ar atmosphere for 8 hours.
After the completion of the reaction, the resultant was cooled to room temperature. The resultant sample was transferred to a separating funnel, and water (100 mL) was charged into the funnel. Then, the mixture was extracted with CH₂Cl₂. The extract was dried with MgSO₄, and was then filtered and concentrated. The resultant sample was purified by silica gel column chromatography, whereby a white solid was obtained in an amount of 11.8 g in 75% yield. FD-MS analysis C₅₂H₃₆F₂N₄O₂: theoretical value 786, observed value 786

(2) Synthesis of Compound 29

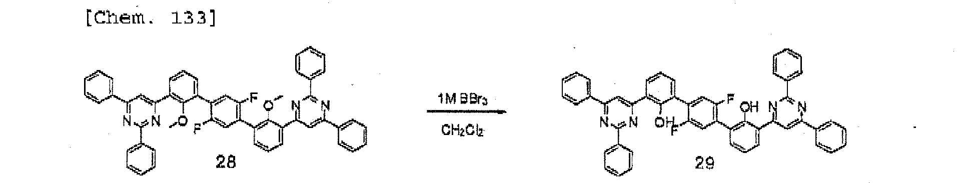

Compound 28 (11.8 g, 15.0 mmol), a 1 M solution of BBr₃ in CH₂Cl₂ (37 mL, 37.0 mmol), and CH₂Cl₂ (150 mL) were loaded into a three-necked flask, and the mixture was stirred under an Ar atmosphere at 0°C for 8 hours. After that, the mixture was left to stand at room temperature overnight. After the completion of the reaction, the resultant was neutralized with a saturated aqueous solution of NaHCO₃. The resultant sample was transferred to a separating funnel, and was extracted with CH₂Cl₂. The resultant sample was purified by silica gel column chromatography, whereby a white solid was obtained in an amount of 10.2g in 90% yield. FD-MS analysis C₅₀H₃₂F₂N₄O₂: theoretical value 758, observed value 758

(3) Synthesis of Compound NO. 87

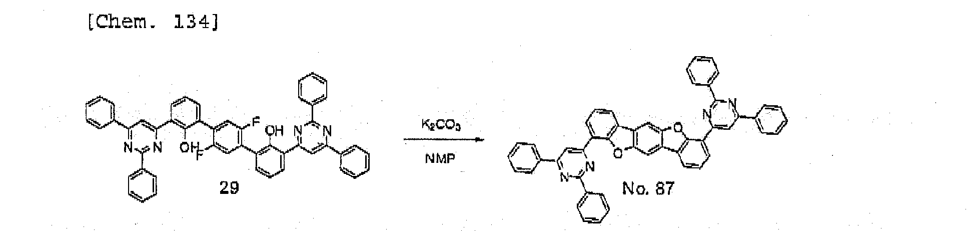

Compound 29 (10.2 g, 13.4mmol), K₂CO₃ (7.4g, 53.8 mmol), and NMP (60 mL) were loaded into a three-necked flask, and the mixture was stirred under an Ar atmosphere at 200°C for 3 hours. After the completion of the reaction, the resultant was cooled to room temperature. Toluene (500mL) was charged into the resultant sample. The mixture was transferred to a separating funnel, and was washed with water. The washed product was dried with MgSO₄, and was then filtered and concentrated. The resultant sample was purified by silica gel column chromatography. The purified product was concentrated to dryness, and was then recrystallized twice, whereby a white powder (Compound No. 87) was obtained. The powder was purified by sublimation, whereby a white solid was obtained in an amount of 2.2 g in 23% yield.
FD-MS analysis C₅₀CH₃₀N₄O₂: theoretical value 718, observed value 718

An apparatus and measurement conditions adopted for field desorption mass spectrometry (FD-MS) in each of Synthesis Examples 1 to 14 are shown below.
- Apparatus:: HX110 (manufactured by JEOL Ltd.)
- Conditions:: accelerating voltage 8 kv scan range m/z=50 to 1500 emitter kind: carbon emitter current: to mA→2 rnA/min→40 mA (held for 10 minutes)

### Example 1

### (Production of organic EL device)

A glass substrate provided with an IT0 transparent electrode measuring 25 mm by 75 mm by 1.1 mm (manufactured by GEOMATEC Co. Ltd.) was subjected to ultrasonic cleaning in isopropyl alcohol for 5 minutes. Further, the substrate was subjected to ultraviolet (UV)-ozone cleaning for 30 minutes.

The glass substrate provided with a transparent electrode thus cleaned was mounted on a substrate holder of a vacuum deposition apparatus. First, Compound A was deposited from the vapor onto the surface of the glass substrate on the side where a transparent electrode line was formed so as to cover the transparent electrode, whereby a hole transporting layer having a thickness of 30 nm was obtained.

Compound No. 1 as a host for phosphorescence and Ir(Ph-ppy)₃ as a dopant for phosphorescence were co-deposited from the vapor onto the hole transporting layer, whereby a phosphorescent layer having a thickness of 30 nm was obtained. The concentration of Ir(Ph-Ppy)₃ was 5 mass%.

Subsequently, Compound B having a thickness of 10 nm, Compound C having a thickness of 20 nm, LiF having a thickness of 1 nm, and metal Al having a thickness of 80 nm were sequentially laminated on the phosphorescent layer, whereby a cathode was obtained. It should be noted that LiF as an electron injectable electrode was formed at a rate of 1 Å/min.

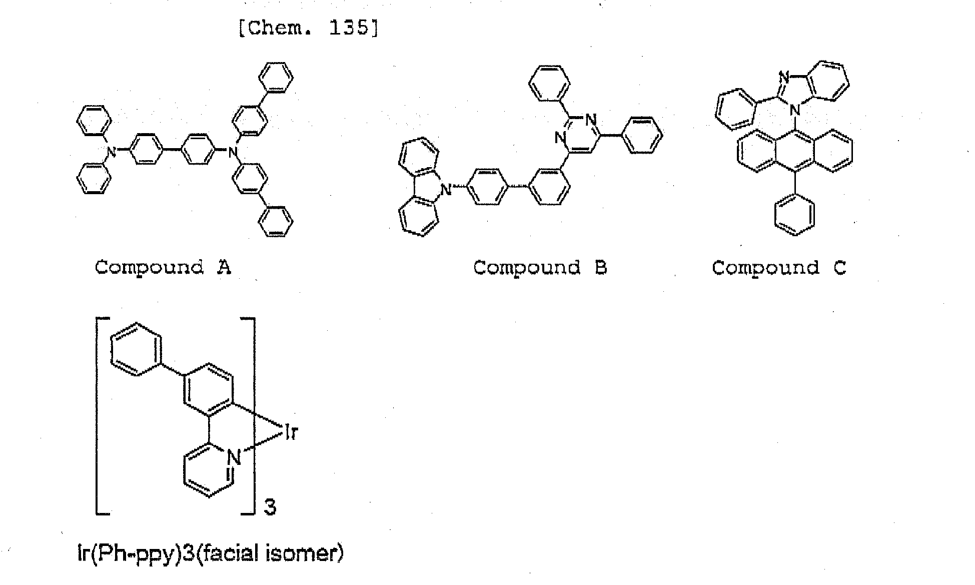

### (Evaluation of organic EL device for light emitting performance)

The organic EL device thus produced was caused to emit light by being driven with a direct current. The luminance (L) and the current density were measured. Then, the current efficiency (L/J). at a luminance of 1000 cd/m² was determined. Further, the lifetime of the device at a luminance of 20,000 cd/m² was determined. Table 1 shows the results.

### Examples 2 to 12

Organic EL devices were each produced in the same manner as in Example 1 except that a host material listed in Table 1 was used instead of Host Compound No. 1 in Example 1, and the devices were each evaluated in the same manner as in Example 1. Table 1 shows the results of the evaluation for light emitting performance.

### Comparative Examples 1 and 2

Organic EL devices were each produced in the same manner as in Example 1 except that the following compounds (a) and (b) described in EP 0908787 A were each used as a host material instead of Host Compound No. 1 in Example 1, and the devices were each evaluated in the same manner as in Example 1. Table 1 shows the results of the evaluation for light emitting performance.

### Comparative Examples 3 and 4

Organic EL devices were each produced in the same manner as in Example 2 except that the following compounds (c) and (d) described in WO 2006-122630 Al were each used as a host material instead of Host Compound No.1 in Example 1, and the devices were each evaluated in the same manner as in Example 1. Table 1 shows the results of the evaluation for light emitting performance.

### Comparative Example 5

An organic EL device was produced in the same manner as in Example 1 except that the following compound (e) described in WO 2007-063754 Al was used as a host material instead of Host Compound No. 1 in Example 1, and the device was evaluated in the same manner as in Example 1. Table 1 shows the results of the evaluation for light emitting performance.

### Comparative Example 6

An organic EL device was produced in the same manner as in Example 1 except that the following compound (f) described in JP 2008-81494 A was used as a host material instead of Host Compound No. 1 in Example 1, and the device was evaluated in the same manner as in Example 1. Table 1 shows the results of the evaluation for light emitting performance.

### Comparative Example 7

An organic EL device was produced in the same manner as in Example 1 except that the following compound (g) described in US 2002-0132134 and US 2003-0044646 was used as a host material instead of Host Compound No. 1 in Example 1, and the device was evaluated in the same manner as in Example 1. Table 1 shows the results of the evaluation for light emitting performance.

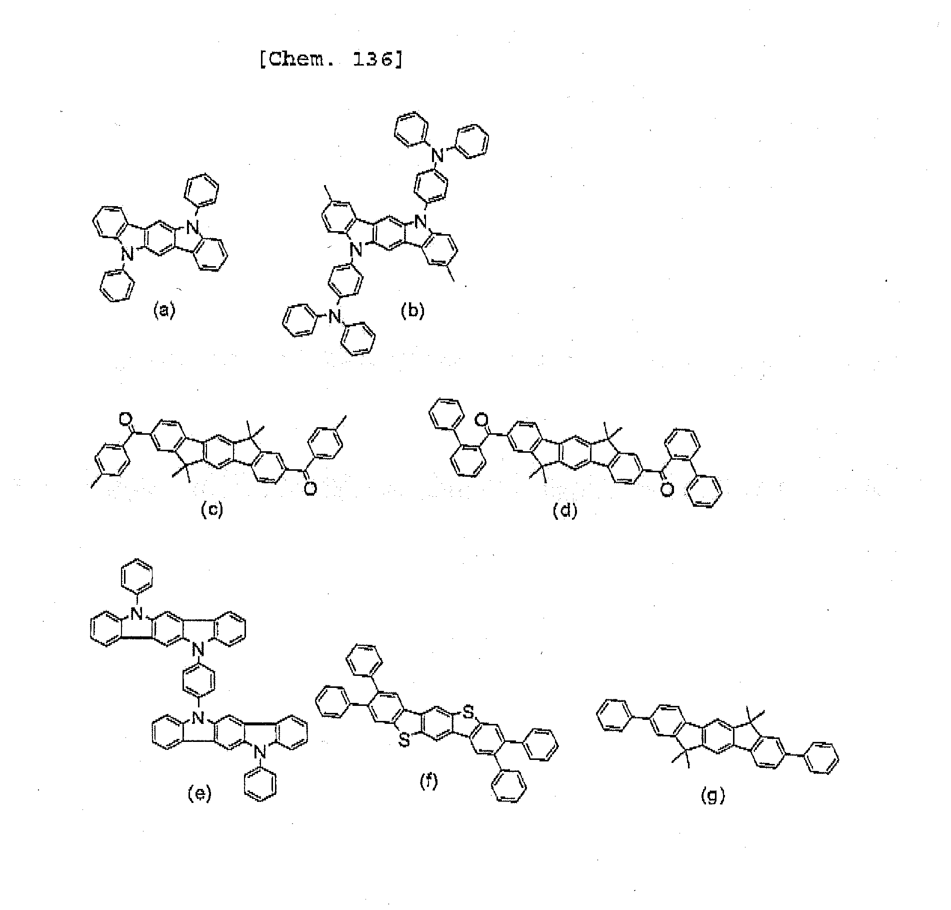

[Table 1]

**Table 1**

| | Host compound | Voltage (V) @20 mA/cm2 | Efficiency (cd/A) @1000 cd/m2 | Lifetime (hr) @20,000 cd/m2 |
|---|---|---|---|---|
| Example 1 | (1) | 5.8 | 52.3 | 350 |
| Example 2 | (11) | 4.5 | 45.8 | 110 |
| Example 3 | (22) | 6.0 | 50.8 | 320 |
| Example 4 | (28) | 5.2 | 57.6 | 400 |
| Example 5 | (39) | 5.3 | 57.5 | 300 |
| Example 6 | (57) | 5.2 | 56.8 | 410 |
| Example 7 | (58) | 5.0 | 50.5 | 350 |
| Example 8 | (60) | 5.4 | 57.8 | 370 |
| Example 9 | (62) | 5.3 | 54.3 | 500 |
| Example 10 | (455) | 4.7 | 58.5 | 380 |
| Example 11 | (461) | 4.6 | 56.5 | 390 |
| Example 22 | (87) | 4.6 | 52.1 | 230 |
| Comparative Example 1 | (a) | 4.6 | 26.5 | 50 |
| Comparative Example 2 | (b) | 4.2 | 17.6 | 30 |
| Comparative Example 3 | (c) | 4.9 | 37.5 | 50 |
| Comparative Example 4 | (d) | 4.7 | 35.9 | 60 |
| Comparative Example 5 | (e) | 4.3 | 17.3 | 30 |
| Comparative Example 6 | (f) | 5.5 | 38.2 | 50 |
| Comparative Example 7 | (g) | 5.4 | 28.7 | 60 |

### Example 13

### (Production of organic EL device)

The glass substrate provided with a transparent electrode cleaned in the same manner as described above was mounted on a substrate holder of a vacuum deposition apparatus. First, Compound A was deposited from the vapor onto the surface of the glass substrate on the side where a transparent electrode line was formed so as to cover the transparent electrode, whereby a hole transporting layer having a thickness of 30 nm was obtained.

Compound No. 198 as a host for phosphorescence and Ir(Ph-ppy)₃ as a dopant for phosphorescence were co-deposited from the vapor onto the hole transporting layer, whereby a phosphorescent layer having a thickness of 30 nm was obtained. The concentration of Ir(Ph-ppy)₃ was 10 mass%.

Subsequently, Compound No. 20 having a thickness of 10 nm, Compound C having a thickness of 20 nm, LiF having a thickness of 1 nm, and metal Al having a thickness of 80 nm were sequentially laminated on the phosphorescent layer, whereby a cathode was obtained. It should be noted that LiF as an electron injectable electrode was formed at a rate of 1 Å/min.

### (Evaluation of organic EL device for light emitting performance)

The organic EL device thus produced was caused to emit light by being driven with a direct current. The luminance (L) and the current density were measured. Then, the current efficiency (L/J) at a luminance of 1000 cd/m² was determined. Further, the lifetime of the device at a luminance of 20,000 cd/m² was determined. Table 2 shows the results.

### Examples 14 to 22

Organic EL devices were each produced in the same manner as in Example 13 except that a host compound and an electron transportable compound listed in Table 2 were used instead of Host Compound No. 198 and Electron Transportable Compound No. 20 in Example 13, and the devices were each evaluated in the same manner as in Example 13. Table 2 shows the results of the evaluation for light emitting performance.

### Comparative Example 8

An organic EL device was produced in the same manner as in Example 13 except that: CBP was used instead of Host Compound No. 198 in Example 13; and BAlq was used instead of Electron Transportable Compound No. 20 in Example 13. Then, the device was evaluated in the same manner as in Example 13. Table 2 shows the results of the evaluation for light emitting performance.

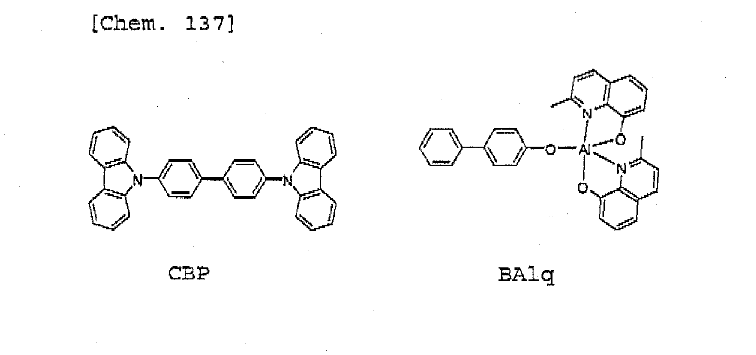

[Table 2]

**Table 2**

| | Host compound | Electron transporting compound | Voltage (V) @20 mA/cm2 | Efficiency (cd/A) @1000cd/m2 | Lifetime (hr) @20,000 cd/m2 |
|---|---|---|---|---|---|
| Example 13 | (198) | (20) | 4.7 | 65.1 | 500 |
| Example 14 | (198) | (67) | 4.5 | 63.9 | 550 |
| Example 15 | (137) | (67) | 4.5 | 57.1 | 350 |
| Example 16 | (193) | (67) | 5.2 | 61.8 | 400 |
| Example 17 | (209) | (67) | 5.3 | 63.8 | 380 |
| Example 18 | (198) | (87) | 4.5 | 63.5 | 540 |
| Example 19 | (137) | (87) | 4.2 | 59.5 | 580 |
| Example 20 | CBP | (20) | 5.3 | 47.2 | 100 |
| Example 21 | CBP | (67) | 5.1 | 48.5 | 100 |
| Example 22 | CBP | (87) | 4.8 | 42.8 | 110 |
| Comparative Example 8 | CBP | BAlq | 6.5 | 45.1 | 30 |

Each of the Organic EL devices of the comparative examples showed a lower current efficiency, was driven at a higher voltage, and had a shorter lifetime than those of each of the organic EL devices of the examples.

### Industrial Applicability

As described above in detail, the utilization of the polycyclic compound of the present invention as a material for an organic EL device can provide an organic EL device which shows high luminous efficiency, and has a long lifetime. Accordingly, the organic EL device of the present invention is extremely useful as, for example, a light source for various electronic instruments. In addition, the halogen compound of the present invention is suitable for an intermediate of the polycyclic compound. In addition, the polycyclic compound of the present invention can be effectively used also as a material for an organic electron device, and is extremely useful in an organic solar cell, organic semiconductor laser, a sensor using organic matter, or an organic TFT.

## Claims

1. A polycyclic compound, which is represented by the following formula (1) or (2): 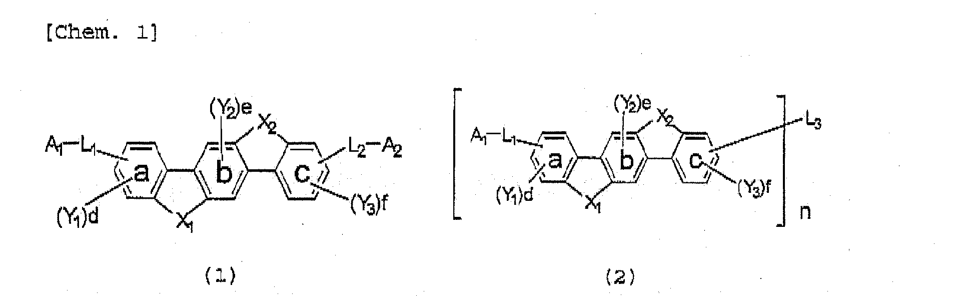 in the formulae (1) and (2), X₁ and X₂ each independently represent oxygen (O), N-R₁, or CR₂R₃, provided that a case where both X₁ and X₂ represent CR₂R₃ is excluded, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms;
in the formula (2), n represents 2, 3, or 4, and the compound represented by the formula (2) comprises a dimer using L₃ as a linking group for n=2, a trimer using L₃ as a linking group for n=3, or a tetramer using L₃ as a linking group for n=4;
in the formulae (1) and (2), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formula (1), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formula (2), when n represents 2, L₃ represents a single bond, an alkylene group having 1 to 20 carbon atoms, a substituted unsubstituted, cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond;
in the formulae (1) and (2) , A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon band, provided that, when L₁ represents an alkylene group having 1 to 20 carbon atoms, a case where A₁ represents a hydrogen atom is excluded;
in the formula (1), A₂ represents a hydrogen atom, substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formulae (1) and (2), Y₁, Y₂, and Y₃ each represent an alkyl group having 1 to 2 carbon atoms, a substituted or unsubstituted cycloalkyl group having a ring formed of 3 to 20 carbon atoms, an alkoxy group having 1 to 20 carbon atoms, an aralkyl group having 7 to 24 carbon, atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2,
provided that, when X₁ and X₂ each represent oxygen (O) or CR₂R₃, bothL₁ and L₂ represent single bonds, and both A₁ and A₂ represent hydrogen atoms, the benzene ring b has one or two Y₂ 's, and a case where Y₂ represents a methyl group or an unsubstituted phenyl group is excluded; and
in the formulae (1) and (2), A₁, A₂, L₁, L₂, and L₃ are each free of any carbonyl group.

2. The polycyclic compound according to claim 1, wherein A₁ and/or A₂ each represent/represents a pyrimidyl group having at least one substituent selected from a phenyl group, a biphenyl group, a terphenyl group, and a quaterphenyl group.

3. The polycyclic compound according to claim 1, wherein A₁ and/or A₂ each represent/represents a carbazolyl group having at least one substituent selected from a phenyl group, a biphenyl group, a terphenyl group, and a quaterphenyl group.

4. The polycyclic compound according to claim 1, wherein A₁ and/or A₂ each represent/represents a dibenzofuranyl group having at least one substituents selected from a phenyl group, a biphenyl group, a terphenyl group, and a quaterphenyl group.

5. The polycyclic compound according to claim 1, wherein A₁ and/or A₂ each represent/represents an aromatic hydrocarbon group having a structure in which two or more benzene rings are bonded to each other at meta positions.

6. The polycyclic compound according to claim 1, wherein the formula (1) is represented by one of the following formulae (1a) and (1b), and the formula (2) is represented by one of the following formulae (2a) and (2b): 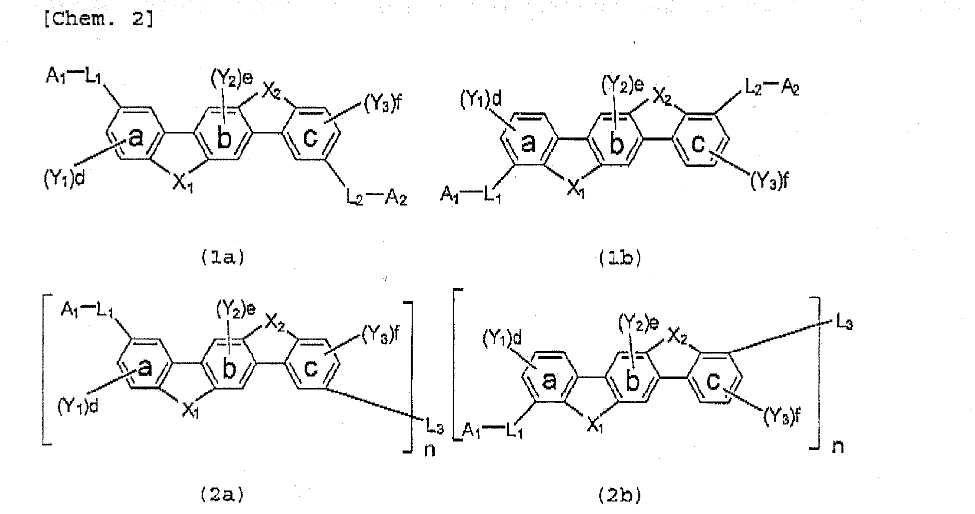 in the formulae (1a), (1b), (2a), and (2b), X₁, X₂, L₁, L₂, L₃, A₁, A₂, Y₁, Y₂, Y₃, n, d, f, and e each have the same meaning as that described above.

7. The polycyclic compound according to claim 1, wherein both X₁ and X₂ in the formula (1) or (2) represent N-R₁, and at least one R₁ represents a dibenzofuran, residue or a carbazole, residue.

8. The polycyclic compound according to claim 1, wherein both X₁ and X₂ in the formula (1) or (2) represent N-R₁, and N-R₁ represented by X₁ and N-R₁ represented by X₂ are different from each other.

9. The polycyclic compound according to claim 1, wherein at least one of X₁ and X₂ in the formula (1) or (2) represents an oxygen atom.

10. The polycyclic compound according to claim 1, wherein the polycyclic compound is represented by one of the following formulae (3) to (10); 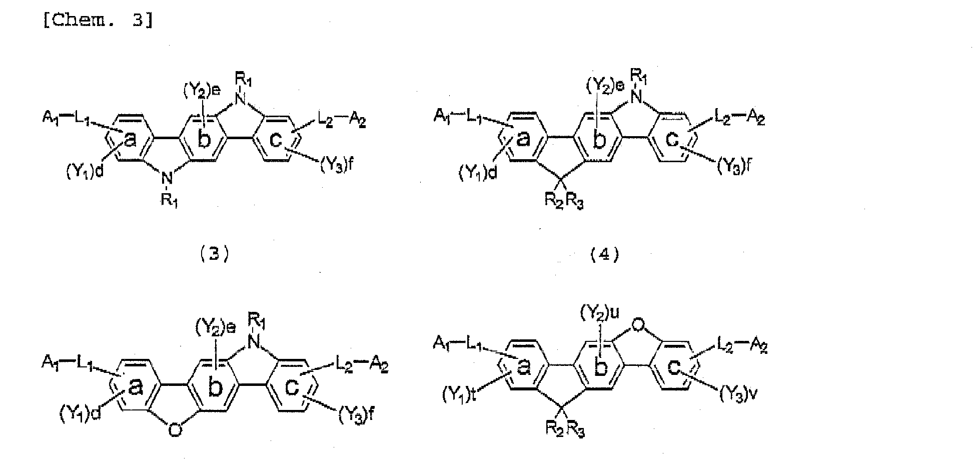 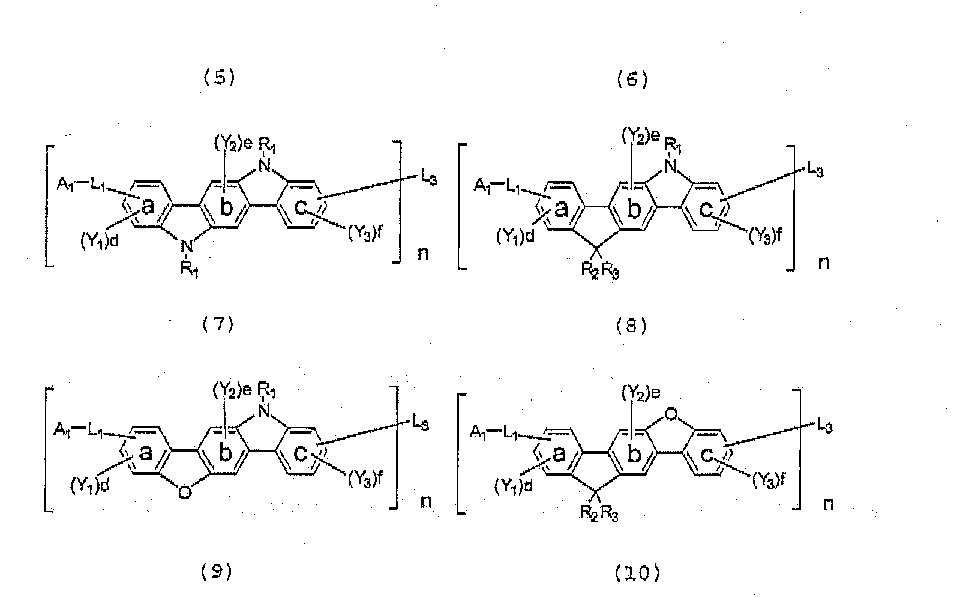 in the formulae (3) to (10), R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, in the formulae (3) and (7), at least one R₁ represents a substituted or unsubstituted, monovalent fused aromatic heterocyclic group having a ring formed of 8 to 24 atoms;
in the formulae (7) to (10), n represents 2, 3, or 4, and the compound represented by any one of the formulae (7) to (10) comprises a dimer using L₃ as a linking group for n=2, a trimer using L₃ as a linking group for n=3, or a tetramer using L₃ as a linking group for n=₄;
in the formulae (3) to (10), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formulae (3) to (6), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formulae (7) to (10), when n represents 2, L₃ represents a single bond, an alkylene group having 1 to 20 carbon atoms, a substituted unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, bivalent aromatic heterocyclic group which has a ring formed of 3 to 24 atoms and which is linked with the benzene ring c through a carbon-carbon bond, when n represents 3, L₃ represents a trivalent saturatedhydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group halving 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond;
in the formulae (3) to (10), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when Lit represents an alkylene group having 1 to 20 carbon atoms, a case where A₁ represents a hydrogen atom is excluded;
in the formulae (3) to (6), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formulae (3) to (10), Y₁, Y₂, and Y₃ each represent an alkyl group having 1 to 20 carbon atoms , a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 2 , or 2, provided that, when both L₁ and L₂ represent single bonds, and both A₁ and A₂ represent hydrogen atoms, the benzene ring b has one or two Y₂'s, and a case where Y₂ represents a methyl group or an unsubstituted phenyl group is excluded; and
in the formulae (3) to (10), A₁, A₃, L₁, L₂, and L₃ are each free of any carbonyl group.

11. The polycyclic compound according to claim 1, wherein the polycyclic compound is represented by one of the following formulae (11) and (12) : 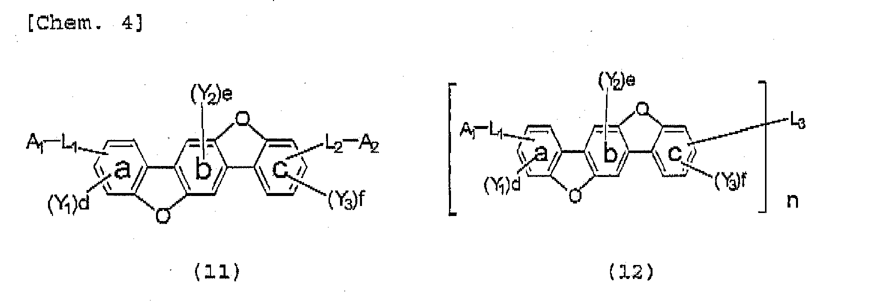 in the formula (12), n represents 2, 3, or 4, and the compound represented by the formula (12) comprises a dimer using L₃ as a linking group for n=2, a trimer using L₃ as a linking group for n=3, or a tetramer using L₃ as a linking group for n=4;
in the formulae (11) and (12), L₁ represents a single bond, an alkylene group having 1 to 20 carbon atoms, a substituted or unsubstituted, cycloalkylene group having a ring formed of 3 to 20 carbon atoms, a divalent silyl group or divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formula (11), L₂ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formula (12), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond;
in the formulae (11) and (12), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁. represents a hydrogen, atom is excluded;
in the formula (11), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formulae (11) and (12), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of to, 1, or 2, provided that, when both L₁ and L₂ represent single bonds, and both A₁ and A₂ represent hydrogen atom, the benzene ring b has one or two Y₂'s, and a case where Y₂ represents a methyl group or an unsubstituted phenyl group is excluded; and
in the formulae (11) and (12), A₁, A₂, L₁, L₂, and L₃ are each free of any carbonyl group.

12. The polycyclic compound according to claim 11, wherein A₁ in the formulae (11) and (12) represents a silyl group or a substituted silyl group having 3 to 20 carbon atoms, or an aromatic heterocyclic group which has a ring formed of 3 to 24 atoms and which is linked with L₁ through a carbon-carbon bond.

13. The polycyclic compound according to claim 11, wherein A₁ in the formulae (11) and (12) represents an aromatic heterocyclic group selected from pyridazine, pyrimidine, pyrazine, 1,3,5-triazine, carbazole, dibenzofuran, dibenzothiophene, phenoxazine, phenothiazine, and dihydroacridine, the aromatic heterocyclic group being linked with L₁ through a carbon-carbon bond.

14. The polycyclic compound according to claim 1, wherein the polycyclic compound is represented by one of the following formulae (13) and (14): 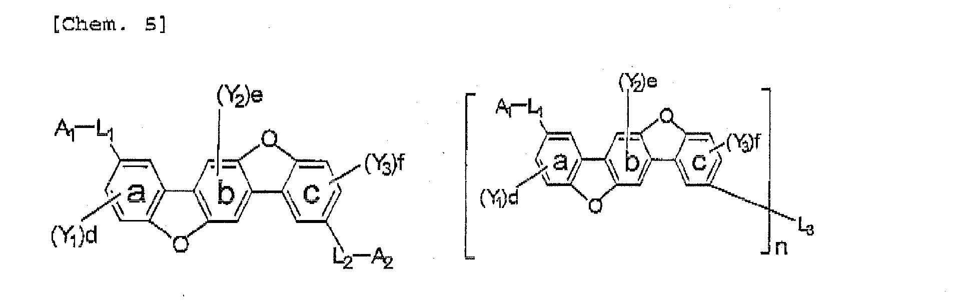 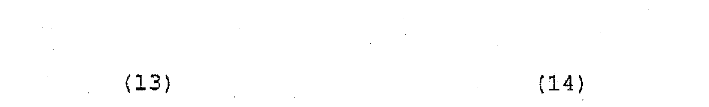 in the formula (14), n represents 2, 3, or 4, and the compound represented by the formula (14) comprises a dimer using L₃ as a linking group for n=2, a trimer using L₃ as a linking group for n=3, or a tetramer using L₃ as a linking group for n=4;
in the formulae (13) and (14), L₁ represents a single bond, an alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formula (13), L₂ represents a single bond, an alkylene group having 1 to 20 carbon atoms, a substituted or unsubstituted cycloalkylene group having a ring formed of 3 to 20 carbon atoms, a silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formula (14), when n represents 2, L₃ represents a single bond, an alkylene group having 1 to 20 carbon atoms, a substituted
or unsubstituted cycloalkylene group having a ring formed of 3 to 20 carbon atoms, a divalent silyl group or a substitute divalent silyl group having 2 to 20 carbon atoms, a substituted or unsubstituted, divalent aromatic hydrocarbon group having a ring formed of 6 to 24 carbon atoms, ar a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted orunsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted r unsubstituted, tetravalent aromatic heterocyclic group which has a ring formed of 3 to 24 atoms and which is linked with the benzene ring c through a carbon-carbon bond;
in the formulae (13) and (14), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
in the formula (13), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formulae (13) and (14), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent, a number of 0, 1, 2, or 3, and e represents a number of 0, 1, or 2, provided that, when both L₁ and L₂ represent single bonds, and both A₁ and A₂ represent hydrogen atom, the benzene ring b has one or two Y₂ '5, and a case where Y₂ represents a methyl group or an unsubstituted phenyl group is excluded; and
in the formulae (13) and (14), A₁, A₂, L₁, L₂, and L₃ are each free of any carbonyl group.

15. The polycyclic compound according to claim 14, wherein A₁ in the formulae (13) and (14) represents a silyl group or a substituted silyl group having 3 to 20 carbon atoms, or an aromatic heterocyclic group which has a ring formed of 3 to 24 atoms and which is linked with L₁ through a carbon-carbon bond.

16. The polycyclic compound according to claim 14, wherein. A₁ in the formulae (13) and (14) represents an aromatic heterocyclic group selected from pyridazine, pyrimidine, pyrazine, 1,3,5-triazine, carbazole, dibenzofuran, dibenzothiophene, phenoxazine, phenothiazine, and dihydroacridine, the aromatic heterocyclic group being linked with L₁ through a carbon - carbon bond.

17. A polycyclic compound, which is represented by the following formula (15); 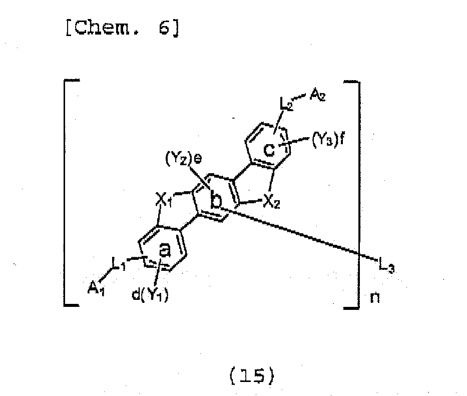 in the formula (15), X₁ and X₂ each independently represent oxygen (O), N-R₁, or CR₂R₃, provided that a case where both X₁ and X₂ represent CR₂R₃ is excluded, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms;
in the formula (15), n represents 2, 3, or 4, and the compound represented by the formula (15) comprises a dimer using L₃ as a linking group for n=2, a trimer using L₃ as a linking group for n=3, or a tetramer using L₃ as a linking group for n=4;
in the formula (15), L₁ represents a single bond, an alkylene group having 1 to 20 carbon atoms, a substituted or unsubstituted, cycloalkylene group having a ring formed of 3 to 20 carbon atoms, a divalent silyl group or a substituted divalent silyl group having 2 to 20 carbon atoms, a substituted on unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formula (15), L₂ represents a single blond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formula (15), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond;
in the formula (15), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
in the formula (15), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formula (15), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic groups which has a ring formed of 3 to 24 atoms and which is linked with the benzene ring a, b, or c through a carbon-carbon bond, d and f each represent a number of 0, 1, 2, or 3, and e represents a number of 0 or 1; and
in the formula (15), A₁, A₂, L₁, L₂, and L₃ are each free of any carbonyl group.

18. The polycyclic compound according to claim 17, wherein A₁ in the formula (15) represents a silyl group or a substituted silyl group having 3 to 20 carbon atoms, or an aromatic heterocyclic group which has a ring formed of 3 to 24 atoms and which is linked with L₁ through a carbon-carbon bond,

19. The polycyclic compound according to claim 17, wherein A₁ in the formula (15) represents an aromatic heterocyclic group selected from pyridazine, pyrimidine, pyrazine, 1,3,5-triazine, carbazole, dibenzofuran, dibenzothiophene, phenoxazine, phenothiazine, and dihydroacridine, the aromatic heterocyclic group being linked with L₁ through a carbon-carbon bond.

20. The polycyclic compound according to claim 17, wherein the polycyclic compound is represented by the following formula (16) : 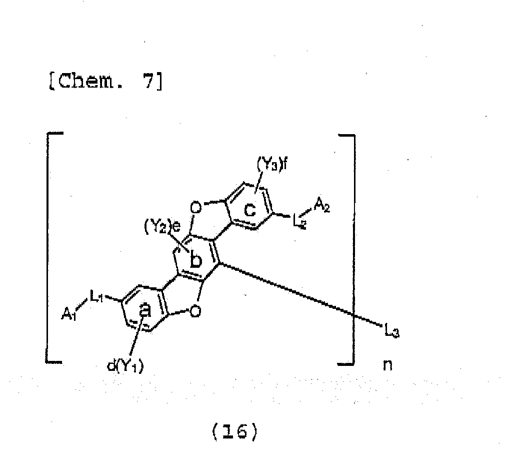 in the formulae (16), n represents 2, 3, or 4, and the compound represented by the formula (16) comprises a dimer using L₃ as a linking group for n=2, a trimer using L₃ as a linking group for n=3, or a tetramer using L₃ as a linking group for n=4;
in the formula (16), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formula (16), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formula (16), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond;
in the formula (16), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
in the formula (16), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formula (16), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 2 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0 or 1; and
in the formula (16), A₁, A₂, L₁, L₂, and L₃ are each free of any carbonyl group.

21. The polycyclic compound according to claim 20, wherein A₁ in the formula (16) represents a silyl group or a substituted silyl group having 3 to 20 carbon atoms, or an aromatic heterocyclic group which has a ring formed of 3 to 24 atoms and which is linked with L₁ through a carbon-carbon bond

22. The polycyclic compound according to claim 20, therein A₁ in the formula (16) represents an aromatic heterocyclic group selected from pyridazine, pyrimidine, pyrazine, 1,3,5-triazine, carbazole, dibenzofuran, dibenzothiophene, phenoxazine, phenothiazine, and dihydroacridine, the aromatic heterocyclic group being linked with L₁ through a carbon-carbon bond.

23. A polycyclic compound, which is represented by the following formula (17); 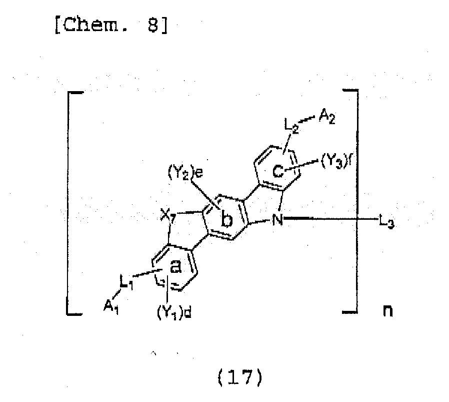 in the formula (17), X₇ represents oxygen (O) or CR₂R₃, and R2 and R₃ each independently represent an alkyl group having 1 to 20 carbon atoms, a substituted or unsubstituted cycloalkyl group having a ring formed of 3 to 20 carbon, atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or substituted or unsubstituted aromatic heterocyclic group having a ring formed of 3 to 24 atoms;
in the formula (17), represents 2, 3, or 4, and the compound represented by the formula (17) comprises a dimer using L₃ as a linking group for n=2, a trimer using L₃ as a linking group for n=3, or a tetramer using L₃ as a linking group for n=4;
in the formula (17), L₁ represents a single bond, an alkylene group having 1, to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formula (17), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond;
in the formula (17), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 2 0 carbonatoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring b through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring b through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring b through a carbon-carbon bond;
in the formula (17), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
in the formula (17), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formula (17), Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2; and
in the formula (17), A₁, A₂, L₁, L₂, and L₃ are each free of any carbonyl group.

24. The polycyclic compound according to claim 23, wherein A₁ in the formula (17) represents a silyl group or a substituted silyl group having 3 to 20 carbon atoms, or an aromatic heterocyclic group which has a ring formed of 3 to 24 atoms and which is linked with L₁ through a carbon-carbon bound.

25. The polycyclic compound according to claim 23, wherein A₁ in the formula (17) represents an aromatic heterocyclic group selected from pyridazine, pyrimidine, pyrazine, 1,3,5-triazine, carbazole, dibenzofuran, dibenzothiophene, phenoxazine, phenothiazine, and dihydroacridine, the aromatic heterocyclic group being linked with L₁ through a carbon-carbon bond.

26. A halogen compound, which is represented by the following formula (18) : 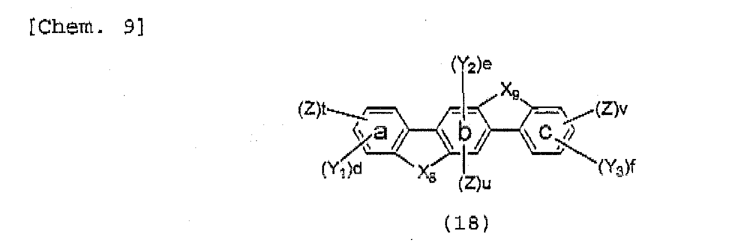 in the formula (18), X₈ and X₉ each independently represent oxygen (O) or N-R₁;
R₁ represents an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₁ and X₂ represent N-R₁, at least one R₁ represents a substituted or unsubstituted, monovalent fused aromatic heterocyclic group having a ring formed of 8 to 24 atoms;
Y₁, Y₂, and Y₃ each represent an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted a silyl group having 3 to 20 carbon atoms, a substituted or unsubstituted aromatic hydrocarbon group having a ring formed of to 24 carbon atoms, or a substituted or unsubstituted aromatic heterocyclic group which has a ring formed of 3 to 24 atoms and which is linked with the benzene ring a, b, or c through a carbon-carbon bond, d and f each represent a number of 0, 1, 2, or 3, and e represents a number of 0, 1, or 2;
Z represents a halogen atom that comprises a fluorine atom, a chlorine atom, a bromine atom, and an iodine atom; and
t, u, and v each represent 0 or 1, provided that t+u+v≥1.

27. The halogen compound according to claim 26, wherein the halogen compound is represented by the following formula (19): 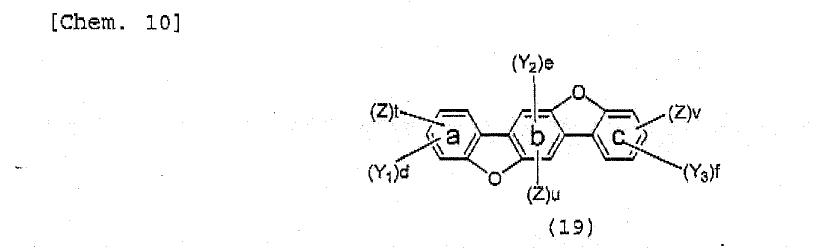 in the formula (19), Y₁, Y₂, and Y₃ each independently represent a hydrogen atom, an alkyl group having 1 to 20 carbon atoms, a substituted or unsubstituted cycloalkyl group having a ring formed of 3 to 20 carbon atoms, an alkoxy group having 1 to 20 carbon atoms, an aralkyl group having 7 two 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with a benzene ring a, b, or c through a carbon-carbon bond, and d and f each represent a number of 0, 1, 2, or 3, and e represents a number of 0, 1, or 2;
Z represents a halogen atom that comprises a fluorine atom, a chlorine atom, a bromine atom, or an iodine atom; and
t, u, and v each represent 0 or 1, provided that t+u+v≥1.

28. An organic electroluminescence device, comprising one or more organic thin film layers including a light emitting layer between a cathode and an anode, wherein at least one layer of the organic thin film layers contains the polycyclic compound according to any one of claims 1, 17, and 23.

29. The organic electroluminescence device according to claim 28, wherein the light emitting layer contains the polycyclic compound as a host material.

30. The organic electroluminescence device according to claim 28, wherein the light emitting layer further contains a phosphorescent material.

31. The organic electroluminescence device according to claim 28, wherein the light emitting layer contains a host material and a phosphorescent material, and the phosphorescent material comprises an orthometalated complex of an iridium (Ir), osmium (Os), or platinum (Pt) metal.

32. The organic electroluminescence device according to claim 28, further comprising an electron injecting layer between the light emitting layer and the cathode, wherein the electron injecting layer contains a nitrogen-containing ring derivative.

33. The organic electroluminescence device according to claim 28, further comprising an electron transporting layer between the light emitting layer and the cathode, wherein the electron transporting layer contains the polycyclic compound.

34. The organic electroluminescence device according to claim 33, wherein the light emitting layer comprises a material for an organic electroluminescence device as a host material, the material for an organic electroluminescence device comprising a compound having a n-conjugated heteroacene skeleton crosslinked with a carbon atom, a nitrogen atom, an oxygen atom, or a sulfur atom.

35. The organic electroluminescence device according to claim 33, wherein the light-emitting layer comprises a material for an organic electroluminescence device, which is represented by any one of the following formulae (20) to (23) as a host material: 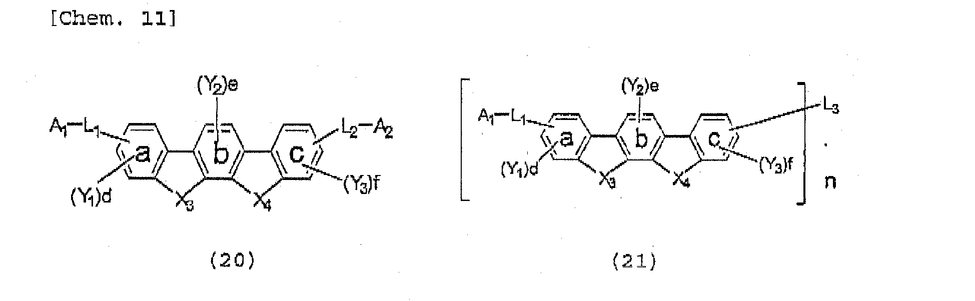 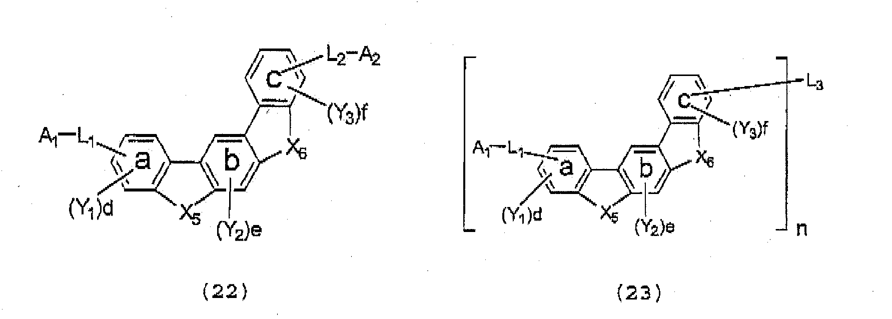 in the formulae (20) to (23) X₃, X₄, X₅, and X₆ each independently represent oxygen (O), sulfur (S), N-R₁, or CR₂R₃, and
R₁, R₂, and R₃ each independently represent an alkyl group having 1 to 20 carbon atoms, a substituted or unsubstituted cycloalkyl group having a ring formed of 3 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group having a ring formed of 3 to 24 atoms, provided that, when both X₃ and X₄ or both X₅ and X₆ represent N-R₁, at least one R₁ represents a substituted or unsubstituted, monovalent fused aromatic heterocyclic group having a ring formed of 8 to 24 atoms;
in the formulae (21) and (23), n represents 2, 3, or 4, and the compound represented by one of the formulae (21) and (23) comprise a dimer using L₃ as a linking group for n=2, a trimer using L₃ as a linking group for n=3, or a tetramer using L₃ as a linking group for n=4;
in the formulae (20) to (23), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring a through a carbon-carbon bond;
in the formulae (20) and (22), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with a benzene ring c through a carbon-carbon bond, provided that, when both X₃ and X₄ or both X₅ and X₆ represent CR₂R₃ and both L₁ and L₂ represent substituted or unsubstituted, divalent aromatic hydrocarbon groups each having a ring formed of 6 to 24 carbon atoms, a case where L₁ and L₂ are simultaneously linked at para positions with respect to a benzene ring b is excluded;
in the formulae (21) and (23), when n represents 2 L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms, a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond, provided that when both X₃ and X₄ or both X₅ and X₆ represent CR₂R₃ and both L₁ and L₃ represent substituted or unsubstituted, monovalent, divalent, or tetravalent aromatic hydrocarbon groups each having a ring formed of 6 to 24 carbon atoms, a case where L₁ and L₃ are simultaneously linked at para positions with respect to the benzene ring b is excluded;
in the formulae (20) to (23), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
in the formulae (20) and (22), A₂ represents a hydrogen atom, a substituted or unsubstituted cycloalkyl group having a ring formed of 3 to 20 carbon atoms, a silyl group or a substituted silyl, group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formulae (20) to (23), Y₁, Y₂, and Y₃ each represent an alkyl group having 1 to 20 carbon atoms, and the compound represented by any one of the formulae (20) to (23) comprise a substituted or unsubstituted cycloalkyl group having a ring formed of 3 to 20 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, a number of each of d and f is 0, 1, 2, or 3, and a number of e is 0 or 1, provided that, when X₃ and X₄ or X₅ and X₆ each represent oxygen (O), sulfur (S), or CR₂R₃, both L₁ and L₂ represent single bonds, and both A₁ and A₂ represent hydrogen atoms, the benzene ring b has one or two Y₂' s, and a case where Y₂ represents a methyl group or an unsubstituted phenyl group is excluded; and
in the formulae (20) to (23), A₁, A₂, L₁, L_{2,} and L₃ are each free of any carbonyl group.

36. The organic electroluminescence device according to claim 33, wherein the light-emitting layer comprises a material for an organic electroluminescence device, which is represented by any one of the following formulae (24) to (27) as a host material: 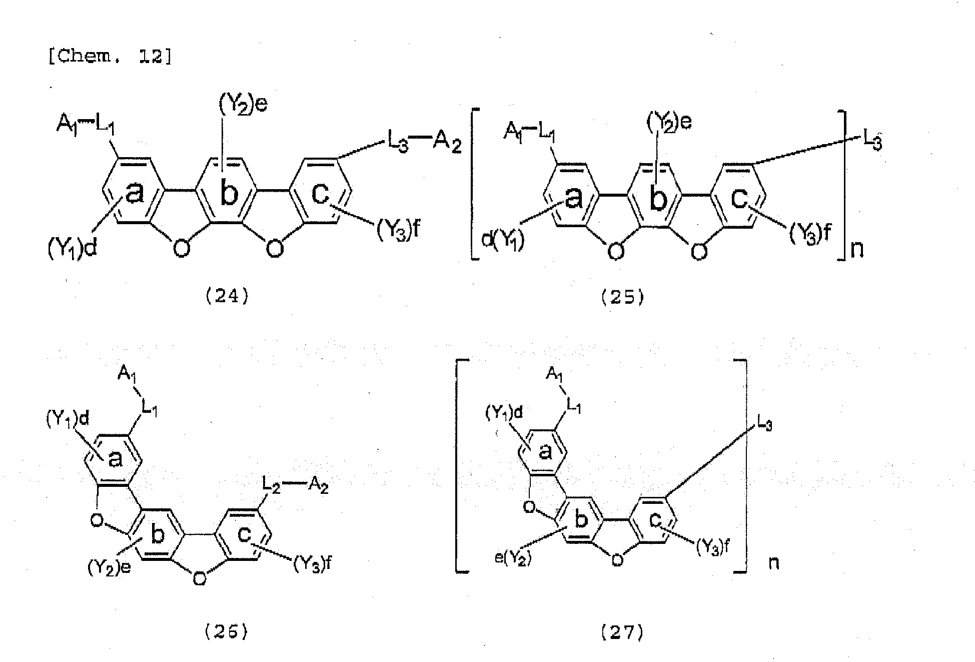 in the formulae (25) and (27), n represents 2, 3, or 4, and the compound represented by one of the formulae (25) and (27) comprises a dimer using L₃ as a linking group for n=2, a trimer using L₃ as a linking group for n=3 , or a tetramer using L₃ as a linking group for n=4;
in the formulae (24) to (27), L₁ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms , or a substituted or unsubstituted, divalent aromatic heterocyclic group which has a ring formed of 3 to 24 atoms and which is linked with a benzene ring a through a carbon-carbon bond;
in the formulae (24) and (26), L₂ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted orunsubstituted, divalent aromatic heterocyclic group which has a ring formed of 3 to 24 atoms and which is linked with a benzene ring c through a carbon-carbon bond;
in the formulae (25) and (27), when n represents 2, L₃ represents a single bond, an alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms and which is linked with the benzene ring c through a carbon-carbon bond, when n represents 3, L₃ represents a trivalent saturated hydrocarbon group having 1 to 20 carbon atoms , a substituted or unsubstituted, trivalent cyclic saturated hydrocarbon group having a ring formed of 3 to 20 carbon atoms, a trivalent silyl group or a substituted trivalent silyl group having 1 to 20 carbon atoms, a substituted or unsubstituted, trivalent aromatic hydrocarbon group having a ring formed of 6 to 24 carbon atoms, or a substituted or unsubstituted, trivalent aromatic heterocyclic group which has 3 to 24 atoms and which is linked with the benzene ring c through a carbon-carbon bond, or when n represents 4, L₃ represents a tetravalent saturated hydrocarbon group having 1 to 20 carbon atoms, a substituted or unsubstituted, tetravalent cyclic saturated hydrocarbon group having a ring formed of 3 to 20 carbon atoms, a silicon atom, a substituted or unsubstituted, tetravalent aromatic hydrocarbon group having a ring formed of 6 to 24 carbon atoms, or a substituted or unsubstituted, tetravalent aromatic heterocyclic group which has a ring formed of 3 to 24 atoms and which is linked with the benzene ring c through a carbon-carbon bond;
in the formulae (24) to (27), A₁ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₁ through a carbon-carbon bond, provided that, when L₁ represents an alkylene group having 1 to 20 carbon atoms, a case where A₁ represents a hydrogen atom is excluded;
in the formulae (24) and (26), A₂ represents a hydrogen atom, a substituted or unsubstituted cycloalkyl group having a ring formed of 3 to 20 carbon atoms, a silyl group or a substituted silyl group having 3 to 20 carbon atoms, a substituted or unsubstituted aromatic hydrocarbon group having a ring formed of 6 to 24 carbon atoms, or an aromatic heterocyclic group which has a ring formed of 3 to 24 atoms and which is linked with L₂ through a carbon-carbon bond, provided that, when L₂ represents an alkylene group having 1 to 20 carbon atoms, a case where A₂ represents a hydrogen atom is excluded;
in the formulae (24) to (27), Y₁, Y₂, and Y₃ each represent an alkyl group having 1 to 20 carbon atoms, a substituted or unsubstituted, cycloalkyl group having a ring formed of 3 to 30 carbon atoms, an alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms and which is linked with the benzene ring a, b, or c through a carbon-carbon bond, a number of each of d and f is 0, 1, 2, or 3, and a number of e is 0, 1, or 2, provided that, when both L₁ and L₂ represent single bonds, and both A₁ and A₂ represent hydrogen atoms, the benzene ring b has one or two Y₂' s, and a case where Y₂ represents a methyl group or an unsubstituted phenyl group is excluded; and
in the formulae (24) to (27), A₁, A₂, L₁, L₂, and L₃ are each free of any carbonyl group.

37. The organic electroluminescence device according to claim 28, further comprising a hole transporting layer between the light emitting layer and the anode, wherein the hole transporting layer contains the polycyclic compound.

38. The organic electroluminescence device according to claim 28, further comprising a reducing dopant at an interfacial region between the cathode and the organic thin film layers.
